(19)

Europäisches
Patentamt
European
Patent Office
Office européen
des brevets

(11) **EP 3 502 130 A1**

(12) **EUROPEAN PATENT APPLICATION**

(43) Date of publication:
**26.06.2019 Bulletin 2019/26**

(21) Application number: **17208924.5**

(22) Date of filing: **20.12.2017**

(51) Int Cl.:
*C07K 14/725* [(2006.01)]    *C07K 14/705* [(2006.01)]
*C07K 14/775* [(2006.01)]    *C07K 14/78* [(2006.01)]
*C07K 14/195* [(2006.01)]    *C07K 14/47* [(2006.01)]
*G01N 33/68* [(2006.01)]

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicants:
• **St. Anna Kinderkrebsforschung
  1090 Vienna (AT)**
• **Universität für Bodenkultur Wien
  1180 Wien (AT)**

(72) Inventors:
• **TRAXLMAYR, Michael
  3430 Tulln (AT)**
• **OBINGER, Christian
  1210 Vienna (AT)**
• **BREY, Charlotte
  1180 Vienna (AT)**
• **LEHNER, Manfred
  1090 Vienna (AT)**

(74) Representative: **Sonn & Partner Patentanwälte
Riemergasse 14
1010 Wien (AT)**

(54) **LIGAND REGULATED PROTEIN-PROTEIN INTERACTION SYSTEM**

(57)    Disclosed is a ligand regulated protein-protein interaction system based on a lipocalin-fold molecule comprising:
(a) a lipocalin-fold molecule
(b) a lipocalin-fold ligand with a low molecular weight of 1500 Da or below, and
(c) a lipocalin-fold binding interaction partner,
wherein the lipocalin-fold molecule can bind to the lipocalin-fold ligand; and
wherein the lipocalin-fold molecule bound to the lipocalin-fold ligand binds to the lipocalin-fold binding interaction partner with an affinity which is at least 10-fold higher than the affinity of the lipocalin-fold molecule not bound to the lipocalin-fold ligand,
and wherein the lipocalin-fold binding interaction partner is not a naturally occurring protein which has an affinity of <10 μM to any naturally occurring lipocalin-fold molecule in the presence of any lipocalin-fold ligand.

EP 3 502 130 A1

SEQUENCE LISTING

<110> ST. ANNA KINDERKREBSFORSCHUNG
      Universität für Bodenkultur Wien

<120> Ligand regulated protein-protein interaction system

<130> R 69941

<160> 42

<170> PatentIn version 3.5

<210> 1
<211> 42
<212> DNA
<213> Artificial Sequence

<220>
<223> epSSo primer

<400> 1
ggctctggtg gaggcggtag cggaggcgga gggtcggcta gc                    42

<210> 2
<211> 42
<212> DNA
<213> Artificial Sequence

<220>
<223> epSso primer

<400> 2
ctattacaag tcctcttcag aaataagctt ttgttcggat cc                    42

<210> 3
<211> 25
<212> DNA
<213> Artificial Sequence

<220>
<223> picZalpha primer

<400> 3
attgccagca ttgctgctaa agaag                                       25

<210> 4
<211> 21
<212> DNA
<213> Artificial Sequence

<220>
<223> picZalpha primer

<400> 4
gcaaatggca ttctgacatc c                                           21

<210> 5
<211> 457

```
<212>   PRT
<213>   Artificial Sequence

<220>
<223>   fusion protein

<400>   5

Met Lys Trp Val Trp Ala Leu Leu Leu Leu Ala Ala Leu Gly Ser Gly
1               5                   10                  15


Arg Ala Asp Ile Gln Met Thr Gln Thr Thr Ser Ser Leu Ser Ala Ser
            20                  25                  30


Leu Gly Asp Arg Val Thr Ile Ser Cys Arg Ala Ser Gln Asp Ile Ser
            35                  40                  45


Lys Tyr Leu Asn Trp Tyr Gln Gln Lys Pro Asp Gly Thr Val Lys Leu
        50                  55                  60


Leu Ile Tyr His Thr Ser Arg Leu His Ser Gly Val Pro Ser Arg Phe
65                  70                  75                  80


Ser Gly Ser Gly Ser Gly Thr Asp Tyr Ser Leu Thr Ile Ser Asn Leu
                85                  90                  95


Glu Gln Glu Asp Ile Ala Thr Tyr Phe Cys Gln Gln Gly Asn Thr Leu
            100                 105                 110


Pro Tyr Thr Phe Gly Gly Gly Thr Lys Leu Glu Ile Thr Gly Ser Thr
            115                 120                 125


Ser Gly Ser Gly Lys Pro Gly Ser Gly Glu Gly Ser Thr Lys Gly Glu
        130                 135                 140


Val Lys Leu Gln Glu Ser Gly Pro Gly Leu Val Ala Pro Ser Gln Ser
145                 150                 155                 160


Leu Ser Val Thr Cys Thr Val Ser Gly Val Ser Leu Pro Asp Tyr Gly
                165                 170                 175


Val Ser Trp Ile Arg Gln Pro Pro Arg Lys Gly Leu Glu Trp Leu Gly
            180                 185                 190


Val Ile Trp Gly Ser Glu Thr Thr Tyr Tyr Asn Ser Ala Leu Lys Ser
        195                 200                 205


Arg Leu Thr Ile Ile Lys Asp Asn Ser Lys Ser Gln Val Phe Leu Lys
    210                 215                 220
```

```
Met Asn Ser Leu Gln Thr Asp Asp Thr Ala Ile Tyr Tyr Cys Ala Lys
225             230             235             240

His Tyr Tyr Tyr Gly Gly Ser Tyr Ala Met Asp Tyr Trp Gly Gln Gly
            245             250             255

Thr Ser Val Thr Val Ser Ser Gly Gly Gly Gly Ser His His His His
        260             265             270

His His Glu Arg Asp Cys Arg Val Ser Ser Phe Arg Val Lys Glu Asn
        275             280             285

Phe Asp Lys Ala Arg Phe Ser Gly Thr Trp Tyr Ala Met Ala Lys Lys
        290             295             300

Asp Pro Glu Gly Leu Phe Leu Gln Asp Asn Ile Val Ala Glu Phe Ser
305             310             315             320

Val Asp Glu Thr Gly Gln Met Ser Ala Thr Ala Lys Gly Arg Val Arg
            325             330             335

Leu Leu Asn Asn Trp Asp Val Cys Ala Asp Met Val Gly Thr Phe Thr
            340             345             350

Asp Thr Glu Asp Pro Ala Lys Phe Lys Met Lys Tyr Trp Gly Val Ala
        355             360             365

Ser Phe Leu Gln Lys Gly Asn Asp Asp His Trp Ile Val Asp Thr Asp
    370             375             380

Tyr Asp Thr Tyr Ala Val Gln Tyr Ser Cys Arg Leu Leu Asn Leu Asp
385             390             395             400

Gly Thr Cys Ala Asp Ser Tyr Ser Phe Val Phe Ser Arg Asp Pro Asn
            405             410             415

Gly Leu Pro Pro Glu Ala Gln Lys Ile Val Arg Gln Arg Gln Glu Glu
            420             425             430

Leu Cys Leu Ala Arg Gln Tyr Arg Leu Ile Val His Asn Gly Tyr Cys
        435             440             445

Asp Gly Arg Ser Glu Arg Asn Leu Leu
    450             455
```

```
<210>   6
<211>   694
```

<212>    PRT
<213>    Artificial Sequence

<220>
<223>    fusion protein

<400>    6

Met Lys Trp Val Trp Ala Leu Leu Leu Leu Ala Ala Leu Gly Ser Gly
1               5                   10                  15

Arg Ala Asp Ile Gln Met Thr Gln Thr Thr Ser Ser Leu Ser Ala Ser
            20                  25                  30

Leu Gly Asp Arg Val Thr Ile Ser Cys Arg Ala Ser Gln Asp Ile Ser
            35                  40                  45

Lys Tyr Leu Asn Trp Tyr Gln Gln Lys Pro Asp Gly Thr Val Lys Leu
        50                  55                  60

Leu Ile Tyr His Thr Ser Arg Leu His Ser Gly Val Pro Ser Arg Phe
65                  70                  75                  80

Ser Gly Ser Gly Ser Gly Thr Asp Tyr Ser Leu Thr Ile Ser Asn Leu
                85                  90                  95

Glu Gln Glu Asp Ile Ala Thr Tyr Phe Cys Gln Gln Gly Asn Thr Leu
            100                 105                 110

Pro Tyr Thr Phe Gly Gly Gly Thr Lys Leu Glu Ile Thr Gly Ser Thr
        115                 120                 125

Ser Gly Ser Gly Lys Pro Gly Ser Gly Glu Gly Ser Thr Lys Gly Glu
        130                 135                 140

Val Lys Leu Gln Glu Ser Gly Pro Gly Leu Val Ala Pro Ser Gln Ser
145                 150                 155                 160

Leu Ser Val Thr Cys Thr Val Ser Gly Val Ser Leu Pro Asp Tyr Gly
                165                 170                 175

Val Ser Trp Ile Arg Gln Pro Pro Arg Lys Gly Leu Glu Trp Leu Gly
            180                 185                 190

Val Ile Trp Gly Ser Glu Thr Thr Tyr Tyr Asn Ser Ala Leu Lys Ser
            195                 200                 205

Arg Leu Thr Ile Ile Lys Asp Asn Ser Lys Ser Gln Val Phe Leu Lys
        210                 215                 220

```
Met Asn Ser Leu Gln Thr Asp Asp Thr Ala Ile Tyr Tyr Cys Ala Lys
225             230             235             240

His Tyr Tyr Tyr Gly Gly Ser Tyr Ala Met Asp Tyr Trp Gly Gln Gly
            245             250             255

Thr Ser Val Thr Val Ser Ser Gly Gly Gly Gly Ser Glu Pro Lys Ser
        260             265             270

Pro Asp Lys Thr His Thr Cys Pro Pro Cys Pro Ala Pro Glu Leu Leu
        275             280             285

Gly Gly Pro Ser Val Phe Leu Phe Pro Pro Lys Pro Lys Asp Thr Leu
        290             295             300

Met Ile Ser Arg Thr Pro Glu Val Thr Cys Val Val Val Asp Val Ser
305             310             315             320

His Glu Asp Pro Glu Val Lys Phe Asn Trp Tyr Val Asp Gly Val Glu
            325             330             335

Val His Asn Ala Lys Thr Lys Pro Arg Glu Glu Gln Tyr Asn Ser Thr
        340             345             350

Tyr Arg Val Val Ser Val Leu Thr Val Leu His Gln Asp Trp Leu Asn
        355             360             365

Gly Lys Glu Tyr Lys Cys Lys Val Ser Asn Lys Ala Leu Pro Ala Pro
        370             375             380

Ile Glu Lys Thr Ile Ser Lys Ala Lys Gly Gln Pro Arg Glu Pro Gln
385             390             395             400

Val Tyr Thr Leu Pro Pro Ser Arg Asp Glu Leu Thr Lys Asn Gln Val
            405             410             415

Ser Leu Thr Cys Leu Val Lys Gly Phe Tyr Pro Ser Asp Ile Ala Val
            420             425             430

Glu Trp Glu Ser Asn Gly Gln Pro Glu Asn Asn Tyr Lys Thr Thr Pro
            435             440             445

Pro Val Leu Asp Ser Asp Gly Ser Phe Phe Leu Tyr Ser Lys Leu Thr
        450             455             460

Val Asp Lys Ser Arg Trp Gln Gln Gly Asn Val Phe Ser Cys Ser Val
465             470             475             480
```

37

```
Met His Glu Ala Leu His Asn His Tyr Thr Gln Lys Ser Leu Ser Leu
                485             490                     495

Ser Pro Gly Lys Gly Gly Gly Gly Ser His His His His His His Glu
            500             505                 510

Arg Asp Cys Arg Val Ser Ser Phe Arg Val Lys Glu Asn Phe Asp Lys
            515             520                 525

Ala Arg Phe Ser Gly Thr Trp Tyr Ala Met Ala Lys Lys Asp Pro Glu
        530             535                 540

Gly Leu Phe Leu Gln Asp Asn Ile Val Ala Glu Phe Ser Val Asp Glu
545             550                 555                     560

Thr Gly Gln Met Ser Ala Thr Ala Lys Gly Arg Val Arg Leu Leu Asn
            565                 570                     575

Asn Trp Asp Val Cys Ala Asp Met Val Gly Thr Phe Thr Asp Thr Glu
            580                 585                 590

Asp Pro Ala Lys Phe Lys Met Lys Tyr Trp Gly Val Ala Ser Phe Leu
        595                 600                 605

Gln Lys Gly Asn Asp Asp His Trp Ile Val Asp Thr Asp Tyr Asp Thr
    610                 615                 620

Tyr Ala Val Gln Tyr Ser Cys Arg Leu Leu Asn Leu Asp Gly Thr Cys
625                 630                 635                     640

Ala Asp Ser Tyr Ser Phe Val Phe Ser Arg Asp Pro Asn Gly Leu Pro
            645                 650                     655

Pro Glu Ala Gln Lys Ile Val Arg Gln Arg Gln Glu Glu Leu Cys Leu
            660                 665                 670

Ala Arg Gln Tyr Arg Leu Ile Val His Asn Gly Tyr Cys Asp Gly Arg
            675                 680                 685

Ser Glu Arg Asn Leu Leu
            690
```

```
<210>   7
<211>   345
<212>   PRT
<213>   Artificial Sequence
```

<220>
<223>   fusion protein

<400>   7

Met Lys Trp Val Trp Ala Leu Leu Leu Ala Ala Leu Gly Ser Gly
1               5                   10                  15

Arg Ala Asp Ile Gln Met Thr Gln Thr Thr Ser Ser Leu Ser Ala Ser
            20                  25                  30

Leu Gly Asp Arg Val Thr Ile Ser Cys Arg Ala Ser Gln Asp Ile Ser
        35                  40                  45

Lys Tyr Leu Asn Trp Tyr Gln Gln Lys Pro Asp Gly Thr Val Lys Leu
    50                  55                  60

Leu Ile Tyr His Thr Ser Arg Leu His Ser Gly Val Pro Ser Arg Phe
65                  70                  75                  80

Ser Gly Ser Gly Ser Gly Thr Asp Tyr Ser Leu Thr Ile Ser Asn Leu
                85                  90                  95

Glu Gln Glu Asp Ile Ala Thr Tyr Phe Cys Gln Gln Gly Asn Thr Leu
            100                 105                 110

Pro Tyr Thr Phe Gly Gly Gly Thr Lys Leu Glu Ile Thr Gly Ser Thr
        115                 120                 125

Ser Gly Ser Gly Lys Pro Gly Ser Gly Glu Gly Ser Thr Lys Gly Glu
        130                 135                 140

Val Lys Leu Gln Glu Ser Gly Pro Gly Leu Val Ala Pro Ser Gln Ser
145                 150                 155                 160

Leu Ser Val Thr Cys Thr Val Ser Gly Val Ser Leu Pro Asp Tyr Gly
                165                 170                 175

Val Ser Trp Ile Arg Gln Pro Pro Arg Lys Gly Leu Glu Trp Leu Gly
        180                 185                 190

Val Ile Trp Gly Ser Glu Thr Thr Tyr Tyr Asn Ser Ala Leu Lys Ser
        195                 200                 205

Arg Leu Thr Ile Ile Lys Asp Asn Ser Lys Ser Gln Val Phe Leu Lys
    210                 215                 220

Met Asn Ser Leu Gln Thr Asp Asp Thr Ala Ile Tyr Tyr Cys Ala Lys
225                 230                 235                 240

```
His Tyr Tyr Tyr Gly Gly Ser Tyr Ala Met Asp Tyr Trp Gly Gln Gly
            245             250             255

Thr Ser Val Thr Val Ser Ser Gly Gly Gly Gly Ser His His His His
            260             265             270

His His Ala Thr Val Lys Leu Thr Tyr Gln Gly Glu Glu Lys Gln Val
            275             280             285

Asp Ile Ser Lys Ile Lys Arg Val Ala Arg Tyr Gly Gln Asn Ile Tyr
    290             295             300

Phe Ser Tyr Asp Glu Gly Gly Gly Ala Tyr Asp Tyr Gly Ala Val Ser
305             310             315             320

Glu Lys Asp Ala Pro Lys Glu Leu Leu Gln Met Leu Glu Lys Gln Glu
            325             330             335

Gln Lys Leu Ile Ser Glu Glu Asp Leu
            340             345


<210>  8
<211>  273
<212>  PRT
<213>  Artificial Sequence

<220>
<223>  fusion protein

<400>  8

Met Lys Trp Val Trp Ala Leu Leu Leu Leu Ala Ala Leu Gly Ser Gly
1               5               10              15

Arg Ala Ala Thr Val Lys Phe Thr Tyr Gln Gly Glu Glu Lys Gln Val
            20              25              30

Asp Ile Ser Lys Ile Met Tyr Val Ile Arg Gly Gly Gln Arg Ile Ala
            35              40              45

Phe Gly Tyr Asp Glu Gly Asp Gly Ala Trp Gly Asp Gly Ile Val Ser
    50              55              60

Glu Lys Asp Ala Pro Lys Glu Leu Leu Gln Met Leu Glu Lys Gln Gly
65              70              75              80

Gly Gly Gly Ser His His His His His His Glu Arg Asp Cys Arg Val
            85              90              95
```

```
Ser Ser Phe Arg Val Lys Glu Asn Phe Asp Lys Ala Arg Phe Ser Gly
        100                 105             110

Thr Trp Tyr Ala Met Ala Lys Lys Asp Pro Glu Gly Leu Phe Leu Gln
        115                 120             125

Asp Asn Ile Val Ala Glu Phe Ser Val Asp Glu Thr Gly Gln Met Ser
        130                 135             140

Ala Thr Ala Lys Gly Arg Val Arg Leu Leu Asn Asn Trp Asp Val Cys
145                 150             155                 160

Ala Asp Met Val Gly Thr Phe Thr Asp Thr Glu Asp Pro Ala Lys Phe
                165             170             175

Lys Met Lys Tyr Trp Gly Val Ala Ser Phe Leu Gln Lys Gly Asn Asp
            180             185             190

Asp His Trp Ile Val Asp Thr Asp Tyr Asp Thr Tyr Ala Val Gln Tyr
        195             200             205

Ser Cys Arg Leu Leu Asn Leu Asp Gly Thr Cys Ala Asp Ser Tyr Ser
    210             215             220

Phe Val Phe Ser Arg Asp Pro Asn Gly Leu Pro Pro Glu Ala Gln Lys
225             230             235             240

Ile Val Arg Gln Arg Gln Glu Glu Leu Cys Leu Ala Arg Gln Tyr Arg
            245             250             255

Leu Ile Val His Asn Gly Tyr Cys Asp Gly Arg Ser Glu Arg Asn Leu
        260             265             270

Leu
```

```
<210>   9
<211>   61
<212>   PRT
<213>   Artificial Sequence

<220>
<223>   fusion protein

<400>   9
```

```
Ala Thr Val Lys Phe Thr Tyr Gln Gly Glu Glu Lys Gln Val Asp Ile
1               5               10              15
```

```
Ser Lys Ile Lys Trp Val Ile Arg Trp Gly Gln His Ile Ala Phe Lys
            20                  25                  30


Tyr Asp Glu Gly Gly Gly Ala Ala Gly Tyr Gly Trp Val Ser Glu Lys
            35                  40                  45


Asp Ala Pro Lys Glu Leu Leu Gln Met Leu Glu Lys Gln
    50                  55                  60
```

<210> 10
<211> 183
<212> DNA
<213> Artificial Sequence

<220>
<223> fusion protein

<400> 10
gcaaccgtga aattcacata ccaaggcgaa gaagaacagg tggatattag caaaatcaag        60

aaagtggctc gttacggcca gaacatttac ttttcttatg atgaaggtgg tggtgcctgg       120

gattatggtg gcgtgagcga aaaagatgca ccgaaagaac tgctgcagat gctggaaaag       180

caa                                                                      183


<210> 11
<211> 61
<212> PRT
<213> Artificial Sequence

<220>
<223> fusion protein

<400> 11

```
Ala Thr Val Lys Phe Thr Tyr Gln Gly Glu Glu Glu Gln Val Asp Ile
1               5                   10                  15


Ser Lys Ile Lys Lys Val Ala Arg Tyr Gly Gln Asn Ile Tyr Phe Ser
            20                  25                  30


Tyr Asp Glu Gly Gly Gly Ala Trp Asp Tyr Gly Gly Val Ser Glu Lys
            35                  40                  45


Asp Ala Pro Lys Glu Leu Leu Gln Met Leu Glu Lys Gln
    50                  55                  60
```

<210> 12
<211> 183
<212> DNA
<213> Artificial Sequence

<220>
<223> fusion protein

42

<400> 12

gcaaccgtga aactcacata ccaaggcgaa gaaaaacagg tggatattag caaaatcaag    60

cgtgtggctc gttacggcca gggtatttac tttgactatg gtgaaggtgg tggtgcctgg    120

ggttacggta gcgtgagcga aaaagatgca ccgaaagaac tgctgcagat gctggaaaag    180

caa    183


<210> 13
<211> 61
<212> PRT
<213> Artificial Sequence

<220>
<223> fusion protein

<400> 13

Ala Thr Val Lys Leu Thr Tyr Gln Gly Glu Glu Lys Gln Val Asp Ile
1               5                   10                  15

Ser Lys Ile Lys Arg Val Ala Arg Tyr Gly Gln Gly Ile Tyr Phe Asp
                20                  25                  30

Tyr Gly Glu Gly Gly Gly Ala Trp Gly Tyr Gly Ser Val Ser Glu Lys
            35                  40                  45

Asp Ala Pro Lys Glu Leu Leu Gln Met Leu Glu Lys Gln
        50                  55                  60


<210> 14
<211> 183
<212> DNA
<213> Artificial Sequence

<220>
<223> fusion protein

<400> 14

gcaaccgtga aactcacata ccaaggcgaa gaaaaacagg tggatattag caaaatcaag    60

cgtgtggctc gttacggcca gaacatttac ttttcttatg atgaaggtgg tggtgcctat    120

gattatggtg cagtgagcga aaaagatgca ccgaaagaac tgctgcagat gctggaaaag    180

caa    183


<210> 15
<211> 61
<212> PRT
<213> Artificial Sequence

<220>
<223> fusion protein

<400> 15

Ala Thr Val Lys Leu Thr Tyr Gln Gly Glu Glu Lys Gln Val Asp Ile
1               5                   10                  15

Ser Lys Ile Lys Arg Val Ala Arg Tyr Gly Gln Asn Ile Tyr Phe Ser
            20                  25                  30

Tyr Asp Glu Gly Gly Gly Ala Tyr Asp Tyr Gly Ala Val Ser Glu Lys
        35                  40                  45

Asp Ala Pro Lys Glu Leu Leu Gln Met Leu Glu Lys Gln
    50                  55                  60


<210> 16
<211> 183
<212> DNA
<213> Artificial Sequence

<220>
<223> fusion protein

<400> 16
gcaaccgcga aattcacata ccaaggcgaa gaaaaacagg tggatattag caaaatcaag      60

cgcgtggctc gttacggcca gggtatttac ttttcttatg atgaaggtgg tggtgcctat     120

ggttatggta gcgtgagcga aaaagatgca ccgaaagaac tgctgcagat gctggaaaag     180

caa                                                                   183


<210> 17
<211> 61
<212> PRT
<213> Artificial Sequence

<220>
<223> fusion protein

<400> 17

Ala Thr Ala Lys Phe Thr Tyr Gln Gly Glu Glu Lys Gln Val Asp Ile
1               5                   10                  15

Ser Lys Ile Lys Arg Val Ala Arg Tyr Gly Gln Gly Ile Tyr Phe Ser
            20                  25                  30

Tyr Asp Glu Gly Gly Gly Ala Tyr Gly Tyr Gly Ser Val Ser Glu Lys
        35                  40                  45

Asp Ala Pro Lys Glu Leu Leu Gln Met Leu Glu Lys Gln
    50                  55                  60


<210> 18


44

```
<211>  183
<212>  DNA
<213>  Artificial Sequence

<220>
<223>  fusion protein

<400>  18
gcaaccgtga aattcacata ccaaggcgaa gaaaaacagg tggatattag caaaatcaag      60

cgtgtggctc gttacggcca gggtatttac tttgactatg gtgaaggtgg tggtgcctgg     120

ggttacggta gcgtgagcga agaagatgca ccgaaagaac tgctgcagat gctggaaaag     180

caa                                                                    183


<210>  19
<211>  61
<212>  PRT
<213>  Artificial Sequence

<220>
<223>  fusion protein

<400>  19

Ala Thr Val Lys Phe Thr Tyr Gln Gly Glu Glu Lys Gln Val Asp Ile
1               5                   10                  15


Ser Lys Ile Lys Arg Val Ala Arg Tyr Gly Gln Gly Ile Tyr Phe Asp
                20                  25                  30


Tyr Gly Glu Gly Gly Gly Ala Trp Gly Tyr Gly Ser Val Ser Glu Glu
            35                  40                  45


Asp Ala Pro Lys Glu Leu Leu Gln Met Leu Glu Lys Gln
        50                  55                  60


<210>  20
<211>  101
<212>  PRT
<213>  Artificial Sequence

<220>
<223>  fusion protein

<400>  20

Val Ser Asp Val Pro Arg Asp Leu Glu Val Val Ala Ala Thr Pro Thr
1               5                   10                  15


Ser Leu Leu Ile Ser Trp Asp Ala Pro Ala Val Thr Val Arg Tyr Tyr
                20                  25                  30


Arg Ile Thr Tyr Gly Glu Thr Gly Gly Asn Ser Pro Val Gln Glu Phe
            35                  40                  45
```

```
Thr Val Pro Gly Ser Lys Ser Thr Ala Thr Ile Ser Gly Leu Lys Pro
    50                  55                  60
```

```
Gly Val Asp Tyr Thr Ile Thr Val Tyr Ala Val Thr Gly Arg Gly Asp
65                  70                  75                  80
```

```
Ser Pro Ala Ser Ser Lys Pro Ile Ser Ile Asn Tyr Arg Thr Glu Ile
                85                  90                  95
```

```
Asp Lys Pro Ser Gln
            100
```

```
<210>  21
<211>  313
<212>  DNA
<213>  Artificial Sequence

<220>
<223>  fusion protein

<400>  21
gtttctgatg ttccgaggga cctggaagtt gttgctgcga cccccaccag cctactgatc        60

agctggtatt atcccaacgc tagtcatgcg ggttattaca gggtcactta cggagaaaca       120

ggaggaaata gccctgtcca ggagttcact gtgcctttct ctattcggta tactattgct       180

accatcagcg gccttaaacc tggagttgat cataccatca ctgtgtatgc tgtcactgac       240

tacgcctatt actaccgctc gtctgagcca atttccatta attaccgaac agaaattgac       300

aaaccatccc agg                                                         313
```

```
<210>  22
<211>  104
<212>  PRT
<213>  Artificial Sequence

<220>
<223>  fusion protein

<400>  22

Val Ser Asp Val Pro Arg Asp Leu Glu Val Val Ala Ala Thr Pro Thr
1               5                   10                  15
```

```
Ser Leu Leu Ile Ser Trp Tyr Tyr Pro Asn Ala Ser His Ala Gly Tyr
                20                  25                  30
```

```
Tyr Arg Val Thr Tyr Gly Glu Thr Gly Gly Asn Ser Pro Val Gln Glu
            35                  40                  45
```

```
Phe Thr Val Pro Phe Ser Ile Arg Tyr Thr Ile Ala Thr Ile Ser Gly
```

```
                50                      55                      60

        Leu Lys Pro Gly Val Asp His Thr Ile Thr Val Tyr Ala Val Thr Asp
        65                  70                  75                  80


        Tyr Ala Tyr Tyr Tyr Arg Ser Ser Glu Pro Ile Ser Ile Asn Tyr Arg
                        85                  90                  95


        Thr Glu Ile Asp Lys Pro Ser Gln
                        100
```

<210> 23
<211> 313
<212> DNA
<213> Artificial Sequence

<220>
<223> fusion protein

<400> 23

```
gtttctgatg ttccgaggga cctggaagtt gttgctgcga cccccaccag cctactgatc      60

agctggtatt atcccaacgc tagtcatgcg ggttattaca ggatcactta cggagaaaca     120

ggaggaaata gccctgtcca ggagttcact gtgcctttct ctattcggta tactattgct     180

accatcagcg gccttaaacc tggagttgat tataccatca ctgtgtatgc tgtcactgac     240

tacgcctatt actaccgctt gtctgagcca atttccatta attaccgaac agaaattgac     300

aaaccatccc agg                                                         313
```

<210> 24
<211> 104
<212> PRT
<213> Artificial Sequence

<220>
<223> fusion protein

<400> 24

```
Val Ser Asp Val Pro Arg Asp Leu Glu Val Val Ala Ala Thr Pro Thr
1               5                   10                  15


Ser Leu Leu Ile Ser Trp Tyr Tyr Pro Asn Ala Ser His Ala Gly Tyr
                20                  25                  30


Tyr Arg Ile Thr Tyr Gly Glu Thr Gly Gly Asn Ser Pro Val Gln Glu
            35                  40                  45


Phe Thr Val Pro Phe Ser Ile Arg Tyr Thr Ile Ala Thr Ile Ser Gly
        50                  55                  60
```

```
Leu Lys Pro Gly Val Asp Tyr Thr Ile Thr Val Tyr Ala Val Thr Asp
65              70              75              80


Tyr Ala Tyr Tyr Tyr Arg Leu Ser Glu Pro Ile Ser Ile Asn Tyr Arg
            85              90              95


Thr Glu Ile Asp Lys Pro Ser Gln
                100
```

```
<210>  25
<211>  307
<212>  DNA
<213>  Artificial Sequence

<220>
<223>  fusion protein

<400>  25
gtttctgatg ttccgaggga cctggaagtt gttgctgcga cccccaccag cctactgatc        60

agctggtatt atcccaacgc tagtcatgcg ggttattaca ggatcactta cggagaaaca       120

ggaggaaata gccctgtcca ggagttcact gtgcctttct ctattcggta tactattgct       180

accatcagcg gccttaaacc tggagttgat tataccatca ctgtgtatgc tgtcactgcc       240

agttgtcagt attgctctta tccaatttcc attaattacc gaacagaaat tgacaaacca       300

tcccagg                                                                 307
```

```
<210>  26
<211>  102
<212>  PRT
<213>  Artificial Sequence

<220>
<223>  fusion protein

<400>  26

Val Ser Asp Val Pro Arg Asp Leu Glu Val Val Ala Ala Thr Pro Thr
1               5               10              15


Ser Leu Leu Ile Ser Trp Tyr Tyr Pro Asn Ala Ser His Ala Gly Tyr
            20              25              30


Tyr Arg Ile Thr Tyr Gly Glu Thr Gly Gly Asn Ser Pro Val Gln Glu
            35              40              45


Phe Thr Val Pro Phe Ser Ile Arg Tyr Thr Ile Ala Thr Ile Ser Gly
        50              55              60


Leu Lys Pro Gly Val Asp Tyr Thr Ile Thr Val Tyr Ala Val Thr Ala
65              70              75              80
```

48

Ser Cys Gln Tyr Cys Ser Tyr Pro Ile Ser Ile Asn Tyr Arg Thr Glu
                85                  90                  95

Ile Asp Lys Pro Ser Gln
                100

<210>  27
<211>  320
<212>  PRT
<213>  Artificial Sequence

<220>
<223>  fusion protein

<400>  27

Met Pro Leu Leu Leu Leu Leu Pro Leu Leu Trp Ala Gly Ala Leu Ala
1                   5                   10                  15

Met Ala Thr Val Lys Phe Thr Tyr Gln Gly Glu Glu Glu Gln Val Asp
                20                  25                  30

Ile Ser Lys Ile Lys Lys Val Ala Arg Tyr Gly Gln Asn Ile Tyr Phe
                35                  40                  45

Ser Tyr Asp Glu Gly Gly Gly Ala Trp Asp Tyr Gly Gly Val Ser Glu
            50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
                100                 105                 110

Ala Ser Gln Pro Leu Ser Leu Arg Pro Glu Ala Cys Arg Pro Ala Ala
            115                 120                 125

Gly Gly Ala Val His Thr Arg Gly Leu Asp Phe Ala Cys Asp Ile Tyr
            130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
            195                 200                 205

Arg Val Lys Phe Ser Arg Ser Ala Asp Ala Pro Ala Tyr Lys Gln Gly
            210                 215                 220

Gln Asn Gln Leu Tyr Asn Glu Leu Asn Leu Gly Arg Arg Glu Glu Tyr
225                 230                 235                 240

Asp Val Leu Asp Lys Arg Arg Gly Arg Asp Pro Glu Met Gly Gly Lys
            245                 250                 255

Pro Arg Arg Lys Asn Pro Gln Glu Gly Leu Tyr Asn Glu Leu Gln Lys
            260                 265                 270

Asp Lys Met Ala Glu Ala Tyr Ser Glu Ile Gly Met Lys Gly Glu Arg
            275                 280                 285

Arg Arg Gly Lys Gly His Asp Gly Leu Tyr Gln Gly Leu Ser Thr Ala
            290                 295                 300

Thr Lys Asp Thr Tyr Asp Ala Leu His Met Gln Ala Leu Pro Pro Arg
305                 310                 315                 320

<210>   28
<211>   320
<212>   PRT
<213>   Artificial Sequence

<220>
<223>   fusion protein

<400>   28

Met Pro Leu Leu Leu Leu Leu Pro Leu Leu Trp Ala Gly Ala Leu Ala
1                   5                   10                  15

Met Ala Thr Val Lys Leu Thr Tyr Gln Gly Glu Glu Lys Gln Val Asp
            20                  25                  30

Ile Ser Lys Ile Lys Arg Val Ala Arg Tyr Gly Gln Gly Ile Tyr Phe
            35                  40                  45

Asp Tyr Gly Glu Gly Gly Gly Ala Trp Gly Tyr Gly Ser Val Ser Glu
            50                  55                  60

Lys Asp Ala Pro Lys Glu Leu Leu Gln Met Leu Glu Lys Gln Gly Gly

50

65                   70                   75                   80

Gly Gly Ser Gly Gly Gly Gly Ser Asn Trp Ser His Pro Gln Phe Glu
                  85               90               95

Lys Thr Thr Thr Pro Ala Pro Arg Pro Pro Thr Pro Ala Pro Thr Ile
            100              105           110

Ala Ser Gln Pro Leu Ser Leu Arg Pro Glu Ala Cys Arg Pro Ala Ala
            115              120           125

Gly Gly Ala Val His Thr Arg Gly Leu Asp Phe Ala Cys Asp Ile Tyr
            130              135           140

Ile Trp Ala Pro Leu Ala Gly Thr Cys Gly Val Leu Leu Leu Ser Leu
145                 150              155           160

Val Ile Thr Leu Tyr Cys Lys Arg Gly Arg Lys Lys Leu Leu Tyr Ile
            165              170           175

Phe Lys Gln Pro Phe Met Arg Pro Val Gln Thr Thr Gln Glu Glu Asp
            180              185           190

Gly Cys Ser Cys Arg Phe Pro Glu Glu Glu Glu Gly Gly Cys Glu Leu
            195              200           205

Arg Val Lys Phe Ser Arg Ser Ala Asp Ala Pro Ala Tyr Lys Gln Gly
            210              215           220

Gln Asn Gln Leu Tyr Asn Glu Leu Asn Leu Gly Arg Arg Glu Glu Tyr
225                 230              235          240

Asp Val Leu Asp Lys Arg Arg Gly Arg Asp Pro Glu Met Gly Gly Lys
            245              250          255

Pro Arg Arg Lys Asn Pro Gln Glu Gly Leu Tyr Asn Glu Leu Gln Lys
            260              265          270

Asp Lys Met Ala Glu Ala Tyr Ser Glu Ile Gly Met Lys Gly Glu Arg
            275              280          285

Arg Arg Gly Lys Gly His Asp Gly Leu Tyr Gln Gly Leu Ser Thr Ala
            290              295          300

Thr Lys Asp Thr Tyr Asp Ala Leu His Met Gln Ala Leu Pro Pro Arg
305                 310              315          320

```
<210>  29
<211>  320
<212>  PRT
<213>  Artificial Sequence

<220>
<223>  fusion protein

<400>  29

Met Pro Leu Leu Leu Leu Leu Pro Leu Leu Trp Ala Gly Ala Leu Ala
1               5                   10                  15

Met Ala Thr Val Lys Leu Thr Tyr Gln Gly Glu Glu Lys Gln Val Asp
            20                  25                  30

Ile Ser Lys Ile Lys Arg Val Ala Arg Tyr Gly Gln Asn Ile Tyr Phe
            35                  40                  45

Ser Tyr Asp Glu Gly Gly Gly Ala Tyr Asp Tyr Gly Ala Val Ser Glu
            50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Thr Thr Thr Pro Ala Pro Arg Pro Pro Thr Pro Ala Pro Thr Ile
            100                 105                 110

Ala Ser Gln Pro Leu Ser Leu Arg Pro Glu Ala Cys Arg Pro Ala Ala
            115                 120                 125

Gly Gly Ala Val His Thr Arg Gly Leu Asp Phe Ala Cys Asp Ile Tyr
            130                 135                 140

Ile Trp Ala Pro Leu Ala Gly Thr Cys Gly Val Leu Leu Leu Ser Leu
145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
            180                 185                 190

Gly Cys Ser Cys Arg Phe Pro Glu Glu Glu Glu Gly Gly Cys Glu Leu
            195                 200                 205

Arg Val Lys Phe Ser Arg Ser Ala Asp Ala Pro Ala Tyr Lys Gln Gly
```

```
            210                        215                        220

Gln Asn Gln Leu Tyr Asn Glu Leu Asn Leu Gly Arg Arg Glu Glu Tyr
225                     230                 235                 240

Asp Val Leu Asp Lys Arg Arg Gly Arg Asp Pro Glu Met Gly Gly Lys
            245                 250                 255

Pro Arg Arg Lys Asn Pro Gln Glu Gly Leu Tyr Asn Glu Leu Gln Lys
            260                 265                 270

Asp Lys Met Ala Glu Ala Tyr Ser Glu Ile Gly Met Lys Gly Glu Arg
            275                 280                 285

Arg Arg Gly Lys Gly His Asp Gly Leu Tyr Gln Gly Leu Ser Thr Ala
            290                 295                 300

Thr Lys Asp Thr Tyr Asp Ala Leu His Met Gln Ala Leu Pro Pro Arg
305                     310                 315                 320


<210>   30
<211>   330
<212>   PRT
<213>   Artificial Sequence

<220>
<223>   fusio protein

<400>   30

Met Pro Leu Leu Leu Leu Leu Pro Leu Leu Trp Ala Gly Ala Leu Ala
1               5                   10                  15

Met Ala Thr Val Lys Leu Thr Tyr Gln Gly Glu Glu Lys Gln Val Asp
            20                  25                  30

Ile Ser Lys Ile Lys Arg Val Ala Arg Tyr Gly Gln Asn Ile Tyr Phe
            35                  40                  45

Ser Tyr Asp Glu Gly Gly Gly Ala Tyr Asp Tyr Gly Ala Val Ser Glu
            50                  55                  60

Lys Asp Ala Pro Lys Glu Leu Leu Gln Met Leu Glu Lys Gln Glu Gln
65                  70                  75                  80

Lys Leu Ile Ser Glu Glu Asp Leu Gly Gly Gly Gly Ser Gly Gly Gly
                85                  90                  95

Gly Ser Asn Trp Ser His Pro Gln Phe Glu Lys Thr Thr Thr Pro Ala
            100                 105                 110
```

```
Pro Arg Pro Pro Thr Pro Ala Pro Thr Ile Ala Ser Gln Pro Leu Ser
    115                 120                 125

Leu Arg Pro Glu Ala Cys Arg Pro Ala Ala Gly Gly Ala Val His Thr
    130                 135                 140

Arg Gly Leu Asp Phe Ala Cys Asp Ile Tyr Ile Trp Ala Pro Leu Ala
145                 150                 155                 160

Gly Thr Cys Gly Val Leu Leu Leu Ser Leu Val Ile Thr Leu Tyr Cys
                165                 170                 175

Lys Arg Gly Arg Lys Lys Leu Leu Tyr Ile Phe Lys Gln Pro Phe Met
            180                 185                 190

Arg Pro Val Gln Thr Thr Gln Glu Glu Asp Gly Cys Ser Cys Arg Phe
            195                 200                 205

Pro Glu Glu Glu Glu Gly Gly Cys Glu Leu Arg Val Lys Phe Ser Arg
    210                 215                 220

Ser Ala Asp Ala Pro Ala Tyr Lys Gln Gly Gln Asn Gln Leu Tyr Asn
225                 230                 235                 240

Glu Leu Asn Leu Gly Arg Arg Glu Glu Tyr Asp Val Leu Asp Lys Arg
                245                 250                 255

Arg Gly Arg Asp Pro Glu Met Gly Gly Lys Pro Arg Arg Lys Asn Pro
            260                 265                 270

Gln Glu Gly Leu Tyr Asn Glu Leu Gln Lys Asp Lys Met Ala Glu Ala
            275                 280                 285

Tyr Ser Glu Ile Gly Met Lys Gly Glu Arg Arg Arg Gly Lys Gly His
    290                 295                 300

Asp Gly Leu Tyr Gln Gly Leu Ser Thr Ala Thr Lys Asp Thr Tyr Asp
305                 310                 315                 320

Ala Leu His Met Gln Ala Leu Pro Pro Arg
                325                 330
```

```
<210>   31
<211>   320
<212>   PRT
<213>   Artificial Sequence
```

<220>
<223>   fusion protein

<400>   31

Met Pro Leu Leu Leu Leu Leu Pro Leu Leu Trp Ala Gly Ala Leu Ala
1               5                   10                  15


Met Ala Thr Ala Lys Phe Thr Tyr Gln Gly Glu Glu Lys Gln Val Asp
            20                  25                  30


Ile Ser Lys Ile Lys Arg Val Ala Arg Tyr Gly Gln Gly Ile Tyr Phe
            35                  40                  45


Ser Tyr Asp Glu Gly Gly Gly Ala Tyr Gly Tyr Gly Ser Val Ser Glu
            50                  55                  60


Lys Asp Ala Pro Lys Glu Leu Leu Gln Met Leu Glu Lys Gln Gly Gly
65                  70                  75                  80


Gly Gly Ser Gly Gly Gly Gly Ser Asn Trp Ser His Pro Gln Phe Glu
                85                  90                  95


Lys Thr Thr Thr Pro Ala Pro Arg Pro Pro Thr Pro Ala Pro Thr Ile
            100                 105                 110


Ala Ser Gln Pro Leu Ser Leu Arg Pro Glu Ala Cys Arg Pro Ala Ala
            115                 120                 125


Gly Gly Ala Val His Thr Arg Gly Leu Asp Phe Ala Cys Asp Ile Tyr
            130                 135                 140


Ile Trp Ala Pro Leu Ala Gly Thr Cys Gly Val Leu Leu Leu Ser Leu
145                 150                 155                 160


Val Ile Thr Leu Tyr Cys Lys Arg Gly Arg Lys Lys Leu Leu Tyr Ile
            165                 170                 175


Phe Lys Gln Pro Phe Met Arg Pro Val Gln Thr Thr Gln Glu Glu Asp
            180                 185                 190


Gly Cys Ser Cys Arg Phe Pro Glu Glu Glu Glu Gly Gly Cys Glu Leu
            195                 200                 205


Arg Val Lys Phe Ser Arg Ser Ala Asp Ala Pro Ala Tyr Lys Gln Gly
            210                 215                 220


Gln Asn Gln Leu Tyr Asn Glu Leu Asn Leu Gly Arg Arg Glu Glu Tyr
225                 230                 235                 240

```
Asp Val Leu Asp Lys Arg Arg Gly Arg Asp Pro Glu Met Gly Gly Lys
                245             250             255

Pro Arg Arg Lys Asn Pro Gln Glu Gly Leu Tyr Asn Glu Leu Gln Lys
                260             265             270

Asp Lys Met Ala Glu Ala Tyr Ser Glu Ile Gly Met Lys Gly Glu Arg
        275             280             285

Arg Arg Gly Lys Gly His Asp Gly Leu Tyr Gln Gly Leu Ser Thr Ala
    290             295             300

Thr Lys Asp Thr Tyr Asp Ala Leu His Met Gln Ala Leu Pro Pro Arg
305             310             315             320
```

<210> 32
<211> 320
<212> PRT
<213> Artificial Sequence

<220>
<223> fusion protein

<400> 32

```
Met Pro Leu Leu Leu Leu Leu Pro Leu Leu Trp Ala Gly Ala Leu Ala
1               5               10              15

Met Ala Thr Val Lys Phe Thr Tyr Gln Gly Glu Glu Lys Gln Val Asp
                20              25              30

Ile Ser Lys Ile Lys Arg Val Ala Arg Tyr Gly Gln Gly Ile Tyr Phe
        35              40              45

Asp Tyr Gly Glu Gly Gly Gly Ala Trp Gly Tyr Gly Ser Val Ser Glu
        50              55              60

Glu Asp Ala Pro Lys Glu Leu Leu Gln Met Leu Glu Lys Gln Gly Gly
65              70              75              80

Gly Gly Ser Gly Gly Gly Gly Ser Asn Trp Ser His Pro Gln Phe Glu
                85              90              95

Lys Thr Thr Thr Pro Ala Pro Arg Pro Pro Thr Pro Ala Pro Thr Ile
                100             105             110

Ala Ser Gln Pro Leu Ser Leu Arg Pro Glu Ala Cys Arg Pro Ala Ala
                115             120             125
```

56

```
Gly Gly Ala Val His Thr Arg Gly Leu Asp Phe Ala Cys Asp Ile Tyr
    130                 135                 140

Ile Trp Ala Pro Leu Ala Gly Thr Cys Gly Val Leu Leu Leu Ser Leu
    145                 150                 155                 160

Val Ile Thr Leu Tyr Cys Lys Arg Gly Arg Lys Lys Leu Leu Tyr Ile
                165                 170                 175

Phe Lys Gln Pro Phe Met Arg Pro Val Gln Thr Thr Gln Glu Glu Asp
                180                 185                 190

Gly Cys Ser Cys Arg Phe Pro Glu Glu Glu Glu Gly Gly Cys Glu Leu
                195                 200                 205

Arg Val Lys Phe Ser Arg Ser Ala Asp Ala Pro Ala Tyr Lys Gln Gly
    210                 215                 220

Gln Asn Gln Leu Tyr Asn Glu Leu Asn Leu Gly Arg Arg Glu Glu Tyr
225                 230                 235                 240

Asp Val Leu Asp Lys Arg Arg Gly Arg Asp Pro Glu Met Gly Gly Lys
                245                 250                 255

Pro Arg Arg Lys Asn Pro Gln Glu Gly Leu Tyr Asn Glu Leu Gln Lys
                260                 265                 270

Asp Lys Met Ala Glu Ala Tyr Ser Glu Ile Gly Met Lys Gly Glu Arg
                275                 280                 285

Arg Arg Gly Lys Gly His Asp Gly Leu Tyr Gln Gly Leu Ser Thr Ala
    290                 295                 300

Thr Lys Asp Thr Tyr Asp Ala Leu His Met Gln Ala Leu Pro Pro Arg
305                 310                 315                 320
```

```
<210>  33
<211>  330
<212>  PRT
<213>  Artificial Sequence

<220>
<223>  fusion protein

<400>  33
```

```
Met Pro Leu Leu Leu Leu Leu Pro Leu Leu Trp Ala Gly Ala Leu Ala
1               5                   10                  15
```

Met Ala Thr Val Lys Phe Thr Tyr Gln Gly Glu Glu Lys Gln Val Asp
            20                  25                  30

Ile Ser Lys Ile Lys Arg Val Ala Arg Tyr Gly Gln Gly Ile Tyr Phe
            35                  40                  45

Asp Tyr Gly Glu Gly Gly Gly Ala Trp Gly Tyr Gly Ser Val Ser Glu
        50                  55                  60

Glu Asp Ala Pro Lys Glu Leu Leu Gln Met Leu Glu Lys Gln Glu Gln
65                  70                  75                  80

Lys Leu Ile Ser Glu Glu Asp Leu Gly Gly Gly Gly Ser Gly Gly Gly
                85                  90                  95

Gly Ser Asn Trp Ser His Pro Gln Phe Glu Lys Thr Thr Thr Pro Ala
            100                 105                 110

Pro Arg Pro Pro Thr Pro Ala Pro Thr Ile Ala Ser Gln Pro Leu Ser
        115                 120                 125

Leu Arg Pro Glu Ala Cys Arg Pro Ala Ala Gly Gly Ala Val His Thr
        130                 135                 140

Arg Gly Leu Asp Phe Ala Cys Asp Ile Tyr Ile Trp Ala Pro Leu Ala
145                 150                 155                 160

Gly Thr Cys Gly Val Leu Leu Leu Ser Leu Val Ile Thr Leu Tyr Cys
                165                 170                 175

Lys Arg Gly Arg Lys Lys Leu Leu Tyr Ile Phe Lys Gln Pro Phe Met
            180                 185                 190

Arg Pro Val Gln Thr Thr Gln Glu Glu Asp Gly Cys Ser Cys Arg Phe
        195                 200                 205

Pro Glu Glu Glu Glu Gly Gly Cys Glu Leu Arg Val Lys Phe Ser Arg
        210                 215                 220

Ser Ala Asp Ala Pro Ala Tyr Lys Gln Gly Gln Asn Gln Leu Tyr Asn
225                 230                 235                 240

Glu Leu Asn Leu Gly Arg Arg Glu Glu Tyr Asp Val Leu Asp Lys Arg
                245                 250                 255

Arg Gly Arg Asp Pro Glu Met Gly Gly Lys Pro Arg Arg Lys Asn Pro
            260                 265                 270

```
Gln Glu Gly Leu Tyr Asn Glu Leu Gln Lys Asp Lys Met Ala Glu Ala
        275                 280                 285
```

```
Tyr Ser Glu Ile Gly Met Lys Gly Glu Arg Arg Arg Gly Lys Gly His
        290                 295                 300
```

```
Asp Gly Leu Tyr Gln Gly Leu Ser Thr Ala Thr Lys Asp Thr Tyr Asp
305                 310                 315                 320
```

```
Ala Leu His Met Gln Ala Leu Pro Pro Arg
                325                 330
```

<210> 34
<211> 524
<212> PRT
<213> Artificial Sequence

<220>
<223> fusion protein

<400> 34

```
Met Pro Leu Leu Leu Leu Leu Pro Leu Leu Trp Ala Gly Ala Leu Ala
1                   5                   10                  15
```

```
Met Ala Thr Val Lys Leu Thr Tyr Gln Gly Glu Glu Lys Gln Val Asp
            20                  25                  30
```

```
Ile Ser Lys Ile Lys Arg Val Ala Arg Tyr Gly Gln Asn Ile Tyr Phe
        35                  40                  45
```

```
Ser Tyr Asp Glu Gly Gly Gly Ala Tyr Asp Tyr Gly Ala Val Ser Glu
        50                  55                  60
```

```
Lys Asp Ala Pro Lys Glu Leu Leu Gln Met Leu Glu Lys Gln Glu Gln
65                  70                  75                  80
```

```
Lys Leu Ile Ser Glu Glu Asp Leu Gly Gly Gly Gly Ser Gly Gly Gly
                85                  90                  95
```

```
Gly Ser Asn Trp Ser His Pro Gln Phe Glu Lys Glu Pro Lys Ser Pro
            100                 105                 110
```

```
Asp Lys Thr His Thr Cys Pro Pro Cys Pro Ala Pro Glu Leu Leu Gly
            115                 120                 125
```

```
Gly Pro Ser Val Phe Leu Phe Pro Pro Lys Pro Lys Asp Thr Leu Met
        130                 135                 140
```

```
Ile Ser Arg Thr Pro Glu Val Thr Cys Val Val Val Asp Val Ser His
145             150             155             160

Glu Asp Pro Glu Val Lys Phe Asn Trp Tyr Val Asp Gly Val Glu Val
            165             170             175

His Asn Ala Lys Thr Lys Pro Arg Glu Glu Gln Tyr Asn Ser Thr Tyr
            180             185             190

Arg Val Val Ser Val Leu Thr Val Leu His Gln Asp Trp Leu Asn Gly
            195             200             205

Lys Glu Tyr Lys Cys Lys Val Ser Asn Lys Ala Leu Pro Ala Pro Ile
    210             215             220

Glu Lys Thr Ile Ser Lys Ala Lys Gly Gln Pro Arg Glu Pro Gln Val
225             230             235             240

Tyr Thr Leu Pro Pro Ser Arg Asp Glu Leu Thr Lys Asn Gln Val Ser
            245             250             255

Leu Thr Cys Leu Val Lys Gly Phe Tyr Pro Ser Asp Ile Ala Val Glu
            260             265             270

Trp Glu Ser Asn Gly Gln Pro Glu Asn Asn Tyr Lys Thr Thr Pro Pro
            275             280             285

Val Leu Asp Ser Asp Gly Ser Phe Phe Leu Tyr Ser Lys Leu Thr Val
            290             295             300

Asp Lys Ser Arg Trp Gln Gln Gly Asn Val Phe Ser Cys Ser Val Met
305             310             315             320

His Glu Ala Leu His Asn His Tyr Thr Gln Lys Ser Leu Ser Leu Ser
            325             330             335

Pro Gly Lys Lys Asp Pro Lys Phe Trp Val Leu Val Val Val Gly Gly
            340             345             350

Val Leu Ala Cys Tyr Ser Leu Leu Val Thr Val Ala Phe Ile Ile Phe
            355             360             365

Trp Val Arg Ser Lys Arg Ser Arg Leu Leu His Ser Asp Tyr Met Asn
            370             375             380

Met Thr Pro Arg Arg Pro Gly Pro Thr Arg Lys His Tyr Gln Pro Tyr
385             390             395             400
```

```
Ala Pro Pro Arg Asp Phe Ala Ala Tyr Arg Ser Leu Arg Val Lys Phe
            405                 410                 415

Ser Arg Ser Ala Asp Ala Pro Ala Tyr Gln Gln Gly Gln Asn Gln Leu
            420                 425                 430

Tyr Asn Glu Leu Asn Leu Gly Arg Arg Glu Glu Tyr Asp Val Leu Asp
            435                 440                 445

Lys Arg Arg Gly Arg Asp Pro Glu Met Gly Gly Lys Pro Arg Arg Lys
    450                 455                 460

Asn Pro Gln Glu Gly Leu Tyr Asn Glu Leu Gln Lys Asp Lys Met Ala
465                 470                 475                 480

Glu Ala Tyr Ser Glu Ile Gly Met Lys Gly Glu Arg Arg Arg Gly Lys
            485                 490                 495

Gly His Asp Gly Leu Tyr Gln Gly Leu Ser Thr Ala Thr Lys Asp Thr
            500                 505                 510

Tyr Asp Ala Leu His Met Gln Ala Leu Pro Pro Arg
            515                 520


<210>  35
<211>  524
<212>  PRT
<213>  Artificial Sequence

<220>
<223>  fusion protein

<400>  35

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
            20                  25                  30

Ile Ser Lys Ile Lys Arg Val Ala Arg Tyr Gly Gln Gly Ile Tyr Phe
            35                  40                  45

Asp Tyr Gly Glu Gly Gly Gly Ala Trp Gly Tyr Gly Ser Val Ser Glu
    50                  55                  60

Glu Asp Ala Pro Lys Glu Leu Leu Gln Met Leu Glu Lys Gln Glu Gln
65                  70                  75                  80
```

Lys Leu Ile Ser Glu Glu Asp Leu Gly Gly Gly Gly Ser Gly Gly Gly
              85              90                    95

Gly Ser Asn Trp Ser His Pro Gln Phe Glu Lys Glu Pro Lys Ser Pro
              100             105                   110

Asp Lys Thr His Thr Cys Pro Pro Cys Pro Ala Pro Glu Leu Leu Gly
        115             120             125

Gly Pro Ser Val Phe Leu Phe Pro Pro Lys Pro Lys Asp Thr Leu Met
    130             135             140

Ile Ser Arg Thr Pro Glu Val Thr Cys Val Val Val Asp Val Ser His
145             150             155             160

Glu Asp Pro Glu Val Lys Phe Asn Trp Tyr Val Asp Gly Val Glu Val
            165             170             175

His Asn Ala Lys Thr Lys Pro Arg Glu Glu Gln Tyr Asn Ser Thr Tyr
        180             185             190

Arg Val Val Ser Val Leu Thr Val Leu His Gln Asp Trp Leu Asn Gly
        195             200             205

Lys Glu Tyr Lys Cys Lys Val Ser Asn Lys Ala Leu Pro Ala Pro Ile
    210             215             220

Glu Lys Thr Ile Ser Lys Ala Lys Gly Gln Pro Arg Glu Pro Gln Val
225             230             235             240

Tyr Thr Leu Pro Pro Ser Arg Asp Glu Leu Thr Lys Asn Gln Val Ser
            245             250             255

Leu Thr Cys Leu Val Lys Gly Phe Tyr Pro Ser Asp Ile Ala Val Glu
        260             265             270

Trp Glu Ser Asn Gly Gln Pro Glu Asn Asn Tyr Lys Thr Thr Pro Pro
        275             280             285

Val Leu Asp Ser Asp Gly Ser Phe Phe Leu Tyr Ser Lys Leu Thr Val
    290             295             300

Asp Lys Ser Arg Trp Gln Gln Gly Asn Val Phe Ser Cys Ser Val Met
305             310             315             320

His Glu Ala Leu His Asn His Tyr Thr Gln Lys Ser Leu Ser Leu Ser
            325             330             335

62

```
Pro Gly Lys Lys Asp Pro Lys Phe Trp Val Leu Val Val Val Gly Gly
        340             345             350

Val Leu Ala Cys Tyr Ser Leu Leu Val Thr Val Ala Phe Ile Ile Phe
        355             360             365

Trp Val Arg Ser Lys Arg Ser Arg Leu Leu His Ser Asp Tyr Met Asn
    370             375             380

Met Thr Pro Arg Arg Pro Gly Pro Thr Arg Lys His Tyr Gln Pro Tyr
385             390             395             400

Ala Pro Pro Arg Asp Phe Ala Ala Tyr Arg Ser Leu Arg Val Lys Phe
            405             410             415

Ser Arg Ser Ala Asp Ala Pro Ala Tyr Gln Gln Gly Gln Asn Gln Leu
        420             425             430

Tyr Asn Glu Leu Asn Leu Gly Arg Arg Glu Glu Tyr Asp Val Leu Asp
        435             440             445

Lys Arg Arg Gly Arg Asp Pro Glu Met Gly Gly Lys Pro Arg Arg Lys
    450             455             460

Asn Pro Gln Glu Gly Leu Tyr Asn Glu Leu Gln Lys Asp Lys Met Ala
465             470             475             480

Glu Ala Tyr Ser Glu Ile Gly Met Lys Gly Glu Arg Arg Arg Gly Lys
            485             490             495

Gly His Asp Gly Leu Tyr Gln Gly Leu Ser Thr Ala Thr Lys Asp Thr
            500             505             510

Tyr Asp Ala Leu His Met Gln Ala Leu Pro Pro Arg
        515             520
```

```
<210>   36
<211>   514
<212>   PRT
<213>   Artificial Sequence

<220>
<223>   fusion protein

<400>   36
```

```
Met Pro Leu Leu Leu Leu Leu Pro Leu Leu Trp Ala Gly Ala Leu Ala
1               5               10              15
```

Met Ala Thr Val Lys Leu Thr Tyr Gln Gly Glu Glu Lys Gln Val Asp
         20                  25                  30

Ile Ser Lys Ile Lys Arg Val Ala Arg Tyr Gly Gln Asn Ile Tyr Phe
         35                  40                  45

Ser Tyr Asp Glu Gly Gly Gly Ala Tyr Asp Tyr Gly Ala Val Ser Glu
         50                  55                  60

Lys Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                 85                  90                  95

Lys Glu Pro Lys Ser Pro Asp Lys Thr His Thr Cys Pro Pro Cys Pro
             100                 105                 110

Ala Pro Glu Leu Leu Gly Gly Pro Ser Val Phe Leu Phe Pro Pro Lys
         115                 120                 125

Pro Lys Asp Thr Leu Met Ile Ser Arg Thr Pro Glu Val Thr Cys Val
     130                 135                 140

Val Val Asp Val Ser His Glu Asp Pro Glu Val Lys Phe Asn Trp Tyr
145                 150                 155                 160

Val Asp Gly Val Glu Val His Asn Ala Lys Thr Lys Pro Arg Glu Glu
                 165                 170                 175

Gln Tyr Asn Ser Thr Tyr Arg Val Val Ser Val Leu Thr Val Leu His
             180                 185                 190

Gln Asp Trp Leu Asn Gly Lys Glu Tyr Lys Cys Lys Val Ser Asn Lys
         195                 200                 205

Ala Leu Pro Ala Pro Ile Glu Lys Thr Ile Ser Lys Ala Lys Gly Gln
     210                 215                 220

Pro Arg Glu Pro Gln Val Tyr Thr Leu Pro Pro Ser Arg Asp Glu Leu
225                 230                 235                 240

Thr Lys Asn Gln Val Ser Leu Thr Cys Leu Val Lys Gly Phe Tyr Pro
             245                 250                 255

Ser Asp Ile Ala Val Glu Trp Glu Ser Asn Gly Gln Pro Glu Asn Asn
         260                 265                 270

64

```
Tyr Lys Thr Thr Pro Pro Val Leu Asp Ser Asp Gly Ser Phe Phe Leu
    275             280             285

Tyr Ser Lys Leu Thr Val Asp Lys Ser Arg Trp Gln Gln Gly Asn Val
    290             295             300

Phe Ser Cys Ser Val Met His Glu Ala Leu His Asn His Tyr Thr Gln
305             310             315             320

Lys Ser Leu Ser Leu Ser Pro Gly Lys Lys Asp Pro Lys Phe Trp Val
            325             330             335

Leu Val Val Val Gly Gly Val Leu Ala Cys Tyr Ser Leu Leu Val Thr
            340             345             350

Val Ala Phe Ile Ile Phe Trp Val Arg Ser Lys Arg Ser Arg Leu Leu
    355             360             365

His Ser Asp Tyr Met Asn Met Thr Pro Arg Arg Pro Gly Pro Thr Arg
    370             375             380

Lys His Tyr Gln Pro Tyr Ala Pro Pro Arg Asp Phe Ala Ala Tyr Arg
385             390             395             400

Ser Leu Arg Val Lys Phe Ser Arg Ser Ala Asp Ala Pro Ala Tyr Gln
            405             410             415

Gln Gly Gln Asn Gln Leu Tyr Asn Glu Leu Asn Leu Gly Arg Arg Glu
            420             425             430

Glu Tyr Asp Val Leu Asp Lys Arg Arg Gly Arg Asp Pro Glu Met Gly
    435             440             445

Gly Lys Pro Arg Arg Lys Asn Pro Gln Glu Gly Leu Tyr Asn Glu Leu
    450             455             460

Gln Lys Asp Lys Met Ala Glu Ala Tyr Ser Glu Ile Gly Met Lys Gly
465             470             475             480

Glu Arg Arg Arg Gly Lys Gly His Asp Gly Leu Tyr Gln Gly Leu Ser
            485             490             495

Thr Ala Thr Lys Asp Thr Tyr Asp Ala Leu His Met Gln Ala Leu Pro
            500             505             510

Pro Arg
```

```
<210>   37
<211>   514
<212>   PRT
<213>   Artificial Sequence

<220>
<223>   fusion protein

<400>   37

Met Pro Leu Leu Leu Leu Leu Pro Leu Leu Trp Ala Gly Ala Leu Ala
1               5                   10                  15

Met Ala Thr Val Lys Phe Thr Tyr Gln Gly Glu Glu Lys Gln Val Asp
                20                  25                  30

Ile Ser Lys Ile Lys Arg Val Ala Arg Tyr Gly Gln Gly Ile Tyr Phe
                35                  40                  45

Asp Tyr Gly Glu Gly Gly Gly Ala Trp Gly Tyr Gly Ser Val Ser Glu
        50                  55                  60

Glu Asp Ala Pro Lys Glu Leu Leu Gln Met Leu Glu Lys Gln Gly Gly
65                  70                  75                  80

Gly Gly Ser Gly Gly Gly Gly Ser Asn Trp Ser His Pro Gln Phe Glu
                85                  90                  95

Lys Glu Pro Lys Ser Pro Asp Lys Thr His Thr Cys Pro Pro Cys Pro
                100                 105                 110

Ala Pro Glu Leu Leu Gly Gly Pro Ser Val Phe Leu Phe Pro Pro Lys
                115                 120                 125

Pro Lys Asp Thr Leu Met Ile Ser Arg Thr Pro Glu Val Thr Cys Val
        130                 135                 140

Val Val Asp Val Ser His Glu Asp Pro Glu Val Lys Phe Asn Trp Tyr
145                 150                 155                 160

Val Asp Gly Val Glu Val His Asn Ala Lys Thr Lys Pro Arg Glu Glu
                165                 170                 175

Gln Tyr Asn Ser Thr Tyr Arg Val Val Ser Val Leu Thr Val Leu His
                180                 185                 190

Gln Asp Trp Leu Asn Gly Lys Glu Tyr Lys Cys Lys Val Ser Asn Lys
                195                 200                 205
```

```
Ala Leu Pro Ala Pro Ile Glu Lys Thr Ile Ser Lys Ala Lys Gly Gln
    210             215             220

Pro Arg Glu Pro Gln Val Tyr Thr Leu Pro Pro Ser Arg Asp Glu Leu
225             230             235             240

Thr Lys Asn Gln Val Ser Leu Thr Cys Leu Val Lys Gly Phe Tyr Pro
                245             250             255

Ser Asp Ile Ala Val Glu Trp Glu Ser Asn Gly Gln Pro Glu Asn Asn
        260             265             270

Tyr Lys Thr Thr Pro Pro Val Leu Asp Ser Asp Gly Ser Phe Phe Leu
        275             280             285

Tyr Ser Lys Leu Thr Val Asp Lys Ser Arg Trp Gln Gln Gly Asn Val
    290             295             300

Phe Ser Cys Ser Val Met His Glu Ala Leu His Asn His Tyr Thr Gln
305             310             315             320

Lys Ser Leu Ser Leu Ser Pro Gly Lys Lys Asp Pro Lys Phe Trp Val
            325             330             335

Leu Val Val Val Gly Gly Val Leu Ala Cys Tyr Ser Leu Leu Val Thr
            340             345             350

Val Ala Phe Ile Ile Phe Trp Val Arg Ser Lys Arg Ser Arg Leu Leu
        355             360             365

His Ser Asp Tyr Met Asn Met Thr Pro Arg Arg Pro Gly Pro Thr Arg
    370             375             380

Lys His Tyr Gln Pro Tyr Ala Pro Pro Arg Asp Phe Ala Ala Tyr Arg
385             390             395             400

Ser Leu Arg Val Lys Phe Ser Arg Ser Ala Asp Ala Pro Ala Tyr Gln
            405             410             415

Gln Gly Gln Asn Gln Leu Tyr Asn Glu Leu Asn Leu Gly Arg Arg Glu
            420             425             430

Glu Tyr Asp Val Leu Asp Lys Arg Arg Gly Arg Asp Pro Glu Met Gly
    435             440             445

Gly Lys Pro Arg Arg Lys Asn Pro Gln Glu Gly Leu Tyr Asn Glu Leu
    450             455             460
```

67

```
Gln Lys Asp Lys Met Ala Glu Ala Tyr Ser Glu Ile Gly Met Lys Gly
465             470             475             480

Glu Arg Arg Arg Gly Lys Gly His Asp Gly Leu Tyr Gln Gly Leu Ser
                485             490             495

Thr Ala Thr Lys Asp Thr Tyr Asp Ala Leu His Met Gln Ala Leu Pro
                500             505             510

Pro Arg


<210>  38
<211>  544
<212>  PRT
<213>  Artificial Sequence

<220>
<223>  fusion protein

<400>  38

Met Pro Leu Leu Leu Leu Leu Pro Leu Leu Trp Ala Gly Ala Leu Ala
1               5               10              15

Met Val Ser Asp Val Pro Arg Asp Leu Glu Val Val Ala Ala Thr Pro
                20              25              30

Thr Ser Leu Leu Ile Ser Trp Tyr Tyr Pro Asn Ala Ser His Ala Gly
            35              40              45

Tyr Tyr Arg Ile Thr Tyr Gly Glu Thr Gly Gly Asn Ser Pro Val Gln
    50              55              60

Glu Phe Thr Val Pro Phe Ser Ile Arg Tyr Thr Ile Ala Thr Ile Ser
65              70              75              80

Gly Leu Lys Pro Gly Val Asp Tyr Thr Ile Thr Val Tyr Ala Val Thr
                85              90              95

Asp Tyr Ala Tyr Tyr Tyr Arg Leu Ser Glu Pro Ile Ser Ile Asn Tyr
                100             105             110

Arg Thr Gly Gly Gly Gly Ser Gly Gly Gly Gly Ser Asp Pro Ala Glu
            115             120             125

Pro Lys Ser Pro Asp Lys Thr His Thr Cys Pro Pro Cys Pro Ala Pro
    130             135             140
```

```
Glu Leu Leu Gly Gly Pro Ser Val Phe Leu Phe Pro Pro Lys Pro Lys
145             150             155             160

Asp Thr Leu Met Ile Ser Arg Thr Pro Glu Val Thr Cys Val Val Val
                165             170             175

Asp Val Ser His Glu Asp Pro Glu Val Lys Phe Asn Trp Tyr Val Asp
            180             185             190

Gly Val Glu Val His Asn Ala Lys Thr Lys Pro Arg Glu Glu Gln Tyr
            195             200             205

Asn Ser Thr Tyr Arg Val Val Ser Val Leu Thr Val Leu His Gln Asp
            210             215             220

Trp Leu Asn Gly Lys Glu Tyr Lys Cys Lys Val Ser Asn Lys Ala Leu
225             230             235             240

Pro Ala Pro Ile Glu Lys Thr Ile Ser Lys Ala Lys Gly Gln Pro Arg
                245             250             255

Glu Pro Gln Val Tyr Thr Leu Pro Pro Ser Arg Asp Glu Leu Thr Lys
            260             265             270

Asn Gln Val Ser Leu Thr Cys Leu Val Lys Gly Phe Tyr Pro Ser Asp
            275             280             285

Ile Ala Val Glu Trp Glu Ser Asn Gly Gln Pro Glu Asn Asn Tyr Lys
        290             295             300

Thr Thr Pro Pro Val Leu Asp Ser Asp Gly Ser Phe Phe Leu Tyr Ser
305             310             315             320

Lys Leu Thr Val Asp Lys Ser Arg Trp Gln Gln Gly Asn Val Phe Ser
                325             330             335

Cys Ser Val Met His Glu Ala Leu His Asn His Tyr Thr Gln Lys Ser
            340             345             350

Leu Ser Leu Ser Pro Gly Lys Lys Asp Pro Lys Phe Trp Val Leu Val
            355             360             365

Val Val Gly Gly Val Leu Ala Cys Tyr Ser Leu Leu Val Thr Val Ala
            370             375             380

Phe Ile Ile Phe Trp Val Arg Ser Lys Arg Ser Arg Leu Leu His Ser
385             390             395             400
```

Asp Tyr Met Asn Met Thr Pro Arg Arg Pro Gly Pro Thr Arg Lys His
                405                     410                     415

Tyr Gln Pro Tyr Ala Pro Pro Arg Asp Phe Ala Ala Tyr Arg Ser Leu
                420                     425                     430

Arg Val Lys Phe Ser Arg Ser Ala Asp Ala Pro Ala Tyr Gln Gln Gly
                435                     440                     445

Gln Asn Gln Leu Tyr Asn Glu Leu Asn Leu Gly Arg Arg Glu Glu Tyr
        450                     455                     460

Asp Val Leu Asp Lys Arg Arg Gly Arg Asp Pro Glu Met Gly Gly Lys
465                     470                     475                     480

Pro Arg Arg Lys Asn Pro Gln Glu Gly Leu Tyr Asn Glu Leu Gln Lys
                485                     490                     495

Asp Lys Met Ala Glu Ala Tyr Ser Glu Ile Gly Met Lys Gly Glu Arg
                500                     505                     510

Arg Arg Gly Lys Gly His Asp Gly Leu Tyr Gln Gly Leu Ser Thr Ala
                515                     520                     525

Thr Lys Asp Thr Tyr Asp Ala Leu His Met Gln Ala Leu Pro Pro Arg
                530                     535                     540

<210> 39
<211> 604
<212> PRT
<213> Artificial Sequence

<220>
<223> fusion protein

<400> 39

Met Glu Thr Asp Thr Leu Leu Leu Trp Val Leu Leu Leu Trp Val Pro
1               5               10                      15

Gly Ser Thr Gly Asp Gly His His His His His Gly Ser Leu Gln
                20                      25                      30

Asp Ser Glu Val Asn Gln Glu Ala Lys Pro Glu Val Lys Pro Glu Val
        35                      40                      45

Lys Pro Glu Thr His Ile Asn Leu Lys Val Ser Asp Gly Ser Ser Glu
        50                      55                      60

```
Ile Phe Phe Lys Ile Lys Lys Thr Thr Pro Leu Arg Arg Leu Met Glu
65              70              75                      80

Ala Phe Ala Lys Arg Gln Gly Lys Glu Met Asp Ser Leu Thr Phe Leu
            85              90                      95

Tyr Asp Gly Ile Glu Ile Gln Ala Asp Gln Thr Pro Glu Asp Leu Asp
        100             105             110

Met Glu Asp Asn Asp Ile Ile Glu Ala His Arg Glu Gln Ile Gly Gly
    115             120             125

Gly Gly Gly Ser Gly Gly Gly Gly Ser Val Ser Asp Val Pro Arg Asp
    130             135             140

Leu Glu Val Val Ala Ala Thr Pro Thr Ser Leu Leu Ile Ser Trp Tyr
145             150             155             160

Tyr Pro Asn Ala Ser His Ala Gly Tyr Tyr Arg Ile Thr Tyr Gly Glu
            165             170             175

Thr Gly Gly Asn Ser Pro Val Gln Glu Phe Thr Val Pro Phe Ser Ile
            180             185             190

Arg Tyr Thr Ile Ala Thr Ile Ser Gly Leu Lys Pro Gly Val Asp Tyr
        195             200             205

Thr Ile Thr Val Tyr Ala Val Thr Asp Tyr Ala Tyr Tyr Tyr Arg Leu
    210             215             220

Ser Glu Pro Ile Ser Ile Asn Tyr Arg Thr Gly Gly Gly Gly Ser Gly
225             230             235             240

Gly Gly Gly Ser Asn Trp Ser His Pro Gln Phe Glu Lys Thr Thr Thr
            245             250             255

Pro Ala Pro Arg Pro Pro Thr Pro Ala Pro Thr Ile Ala Ser Gln Pro
            260             265             270

Leu Ser Leu Arg Pro Glu Ala Ser Arg Pro Ala Ala Gly Gly Ala Val
        275             280             285

His Thr Arg Gly Leu Asp Phe Ala Ser Asp Ile Tyr Ile Trp Ala Pro
    290             295             300

Leu Ala Gly Thr Cys Gly Val Leu Leu Leu Ser Leu Val Ile Thr Leu
305             310             315             320
```

```
Tyr Cys Lys Arg Gly Arg Lys Lys Leu Leu Tyr Ile Phe Lys Gln Pro
            325             330                     335

Phe Met Arg Pro Val Gln Thr Thr Gln Glu Glu Asp Gly Cys Ser Cys
            340             345                     350

Arg Phe Pro Glu Glu Glu Glu Gly Gly Cys Glu Leu Ser Arg Gly Ser
            355             360                     365

Gly Ser Gly Ser Gly Ser Met Gly Val Gln Val Glu Thr Ile Ser Pro
    370             375                     380

Gly Asp Gly Arg Thr Phe Pro Lys Arg Gly Gln Thr Cys Val Val His
385                 390                     395                 400

Tyr Thr Gly Met Leu Glu Asp Gly Lys Lys Val Asp Ser Ser Arg Asp
                405                     410                     415

Arg Asn Lys Pro Phe Lys Phe Met Leu Gly Lys Gln Glu Val Ile Arg
            420                 425                     430

Gly Trp Glu Glu Gly Val Ala Gln Met Ser Val Gly Gln Arg Ala Lys
        435                 440                     445

Leu Thr Ile Ser Pro Asp Tyr Ala Tyr Gly Ala Thr Gly His Pro Gly
    450                 455                     460

Ile Ile Pro Pro His Ala Thr Leu Val Phe Asp Val Glu Leu Leu Lys
465                 470                     475                 480

Leu Glu Gly Ser Gly Ser Gly Ser Gly Ser Ser Leu Arg Val Lys Phe
                485                     490                     495

Ser Arg Ser Ala Asp Ala Pro Ala Tyr Lys Gln Gly Gln Asn Gln Leu
            500                     505                     510

Tyr Asn Glu Leu Asn Leu Gly Arg Arg Glu Glu Tyr Asp Val Leu Asp
        515                     520                     525

Lys Arg Arg Gly Arg Asp Pro Glu Met Gly Gly Lys Pro Arg Arg Lys
    530                     535                     540

Asn Pro Gln Glu Gly Leu Tyr Asn Glu Leu Gln Lys Asp Lys Met Ala
545                 550                     555                 560

Glu Ala Tyr Ser Glu Ile Gly Met Lys Gly Glu Arg Arg Arg Gly Lys
```

```
                    565                      570                          575


        Gly His Asp Gly Leu Tyr Gln Gly Leu Ser Thr Ala Thr Lys Asp Thr
                    580                  585                  590


        Tyr Asp Ala Leu His Met Gln Ala Leu Pro Pro Arg
                595                  600


        <210>  40
        <211>  636
        <212>  PRT
        <213>  Artificial Sequence

        <220>
        <223>  fusion protein

        <400>  40

        Met Pro Leu Leu Leu Leu Leu Pro Leu Leu Trp Ala Gly Ala Leu Ala
        1                   5                   10                  15


        Met Glu Arg Asp Cys Arg Val Ser Ser Phe Arg Val Lys Glu Asn Phe
                    20                  25                  30


        Asp Lys Ala Arg Phe Ser Gly Thr Trp Tyr Ala Met Ala Lys Lys Asp
                    35                  40                  45


        Pro Glu Gly Leu Phe Leu Gln Asp Asn Ile Val Ala Glu Phe Ser Val
                50                  55                  60


        Asp Glu Thr Gly Gln Met Ser Ala Thr Ala Lys Gly Arg Val Arg Leu
        65                  70                  75                  80


        Leu Asn Asn Trp Asp Val Cys Ala Asp Met Val Gly Thr Phe Thr Asp
                        85                  90                  95


        Thr Glu Asp Pro Ala Lys Phe Lys Met Lys Tyr Trp Gly Val Ala Ser
                    100                 105                 110


        Phe Leu Gln Lys Gly Asn Asp Asp His Trp Ile Val Asp Thr Asp Tyr
                    115                 120                 125


        Asp Thr Tyr Ala Val Gln Tyr Ser Cys Arg Leu Leu Asn Leu Asp Gly
                    130                 135                 140


        Thr Cys Ala Asp Ser Tyr Ser Phe Val Phe Ser Arg Asp Pro Asn Gly
        145                 150                 155                 160


        Leu Pro Pro Glu Ala Gln Lys Ile Val Arg Gln Arg Gln Glu Glu Leu
                        165                 170                 175
```

Cys Leu Ala Arg Gln Tyr Arg Leu Ile Val His Asn Gly Tyr Cys Asp
              180                 185                 190

Gly Arg Ser Glu Arg Asn Leu Leu Gly Gly Gly Gly Ser Gly Gly Gly
              195                 200                 205

Gly Ser Asn Trp Ser His Pro Gln Phe Glu Lys Glu Pro Lys Ser Pro
              210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                  245                 250                 255

Ile Ser Arg Thr Pro Glu Val Thr Cys Val Val Val Asp Val Ser His
              260                 265                 270

Glu Asp Pro Glu Val Lys Phe Asn Trp Tyr Val Asp Gly Val Glu Val
          275                 280                 285

His Asn Ala Lys Thr Lys Pro Arg Glu Glu Gln Tyr Asn Ser Thr Tyr
          290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                  325                 330                 335

Glu Lys Thr Ile Ser Lys Ala Lys Gly Gln Pro Arg Glu Pro Gln Val
              340                 345                 350

Tyr Thr Leu Pro Pro Ser Arg Asp Glu Leu Thr Lys Asn Gln Val Ser
          355                 360                 365

Leu Thr Cys Leu Val Lys Gly Phe Tyr Pro Ser Asp Ile Ala Val Glu
          370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                  405                 410                 415

Asp Lys Ser Arg Trp Gln Gln Gly Asn Val Phe Ser Cys Ser Val Met

```
                420                      425                      430


      His Glu Ala Leu His Asn His Tyr Thr Gln Lys Ser Leu Ser Leu Ser
              435                  440              445


      Pro Gly Lys Lys Asp Pro Lys Phe Trp Val Leu Val Val Val Gly Gly
              450                  455              460


      Val Leu Ala Cys Tyr Ser Leu Leu Val Thr Val Ala Phe Ile Ile Phe
      465                  470              475                  480


      Trp Val Arg Ser Lys Arg Ser Arg Leu Leu His Ser Asp Tyr Met Asn
                  485                  490              495


      Met Thr Pro Arg Arg Pro Gly Pro Thr Arg Lys His Tyr Gln Pro Tyr
              500                  505              510


      Ala Pro Pro Arg Asp Phe Ala Ala Tyr Arg Ser Leu Arg Val Lys Phe
              515                  520              525


      Ser Arg Ser Ala Asp Ala Pro Ala Tyr Gln Gln Gly Gln Asn Gln Leu
              530                  535              540


      Tyr Asn Glu Leu Asn Leu Gly Arg Arg Glu Glu Tyr Asp Val Leu Asp
      545                  550              555                  560


      Lys Arg Arg Gly Arg Asp Pro Glu Met Gly Gly Lys Pro Arg Arg Lys
                  565                  570              575


      Asn Pro Gln Glu Gly Leu Tyr Asn Glu Leu Gln Lys Asp Lys Met Ala
              580                  585              590


      Glu Ala Tyr Ser Glu Ile Gly Met Lys Gly Glu Arg Arg Arg Gly Lys
              595                  600              605


      Gly His Asp Gly Leu Tyr Gln Gly Leu Ser Thr Ala Thr Lys Asp Thr
              610                  615              620


      Tyr Asp Ala Leu His Met Gln Ala Leu Pro Pro Arg
      625                  630              635


      <210>   41
      <211>   50
      <212>   DNA
      <213>   Artificial Sequence

      <220>
      <223>   FN3 primer
```

```
<400>  41
cgacgattga aggtagatac ccatacgacg ttccagacta cgctctgcag          50


<210>  42
<211>  50
<212>  DNA
<213>  Artificial Sequence

<220>
<223>  FN3 primer

<400>  42
atctcgagct attacaagtc ctcttcagaa ataagctttt gttcggatcc          50
```

**Description**

The present invention discloses ligand regulated protein-protein interaction systems and their use in diagnosis and therapy, especially in tumour therapy.

Protein-protein interactions (PPIs) are the physical contacts of high specificity established between two or more protein molecules as a result of biochemical events steered by electrostatic forces including the hydrophobic effect. Many are physical contacts with molecular associations between chains that occur in a cell or in a living organism in a specific bio-molecular context. PPIs have also been used in the prior art for establishing screening systems or defined switches for pharmaceutical purposes, especially in human medicine.

A specific example of PPIs is dimerization, especially chemically induced dimerization (CID) by small molecules. There are several systems for CID of proteins by small molecules. In these systems PPIs, i.e. homo- or heterodimerization, can only occur in the presence of a small molecule, which thus acts as a chemical dimerizer. In most cases, the dimerizing proteins, or respective domains thereof, are expressed as parts of fusion constructs containing the proteins of interest. Some of the systems are functional not only inside cells but also in the oxidative environment outside from cells. Until now CID systems mainly have been used for, e.g., regulating enzyme function, signal transduction, gene transcription, genome editing (by e.g. CRISPR/Cas), protein stability, and for generating logic gates. CID systems are also increasingly considered for clinical applications, as e.g., for regulating the function of T cells modified with a chimeric antigen receptor. The most frequently used system is based on FRB/FBKP-domains for heterodimerization or a mutant FKBP-domain for homodimerization. Several rapamycin derivatives have been developed, but they have not yet solved the problem of simultaneously regulating two processes in the same cell. Such functionality has been introduced by the more recent development of CID systems, which are based on different proteins and also different small molecules. These systems work well *in vitro,* however, their use *in vivo* is limited. A much higher impact would have the development of CID systems that are suited for *in vivo* and even clinical application and for simultaneous control of multiple processes. The current systems are not suited for this purpose, since they are based on xenogeneic proteins and are thus expected to be highly immunogenic, and/or since the small molecules are not suited for *in vivo* application.

The FKBP-based system for homodimerization is the most advanced system and has been in clinical use for several years for inducing apoptosis in administered T cells. It is based on a human protein, depends on an inert molecule and is fully orthogonalized, i.e., the dimerizer does not bind to endogenous proteins and the mutated FKBP domain binds only the synthetic dimerizer. This process of insulating a protein ligand pair, called "orthogonalization", prevents unwanted interaction in both directions and is essential for the use of CID systems in cellular systems. In the case of heterodimerization even the most advanced human protein based system (FKBP/FRB-system) is still not fully orthogonal. Engineering of the protein binding pocket and complementarily of the small molecule was performed only for the interaction of the rapalog with FRB but not with FKBP, which is still based on the wild-type FBKP domain. Due to the binding mode of rapalogues to FRB and FKBP (see PDB 1NSG for the structure), redesigning the rapalogues for orthogonalization and for pharmacokinetic optimization poses a serious obstacle. Furthermore, the synthesis of rapalogues is difficult, potential contamination with rapamycin is a danger, and the molecules are not clinically approved.

Examples for CID systems have been disclosed in WO 2014/127261 A1 and WO 2017/032777 A1.

It is an object of the present invention to provide ligand regulated PPI systems (LRPPI) that can be regulated by small molecules for inducing dimerization of interaction partners and which are advantageous compared to existing systems. More specifically, novel LRPPI systems should be applicable *in vivo,* especially for the treatment of human patients, without the risk of adverse reactions or at least with reduced adverse reactions. It is a further object to provide means for tumour treatment, especially immunotherapy concepts for the treatment of tumours.

Therefore, the present invention provides a ligand regulated protein-protein interaction system based on a lipocalin-fold molecule comprising:

   (a) a lipocalin-fold molecule
   (b) a lipocalin-fold ligand with a low molecular weight of 1500 Da or below, and
   (c) a lipocalin-fold binding interaction partner,

wherein the lipocalin-fold molecule can bind to the lipocalin-fold ligand; and
wherein the lipocalin-fold molecule bound to the lipocalin-fold ligand binds to the lipocalin-fold binding interaction partner with an affinity which is at least 10-fold higher than the affinity of the lipocalin-fold molecule not bound to the lipocalin-fold ligand,
and wherein the lipocalin-fold binding interaction partner is not a naturally occurring protein which has an affinity of <10 $\mu$M to any naturally occurring lipocalin-fold molecule in the presence of any lipocalin-fold ligand.

The present invention also refers to a ligand regulated protein-protein interaction system based on a lipocalin-fold molecule comprising:

(a) a lipocalin-fold molecule
(b) a lipocalin-fold ligand with a low molecular weight of 1500 Da or below, and
(c) a lipocalin-fold binding interaction partner,

wherein the lipocalin-fold molecule has at least a first conformation when the lipocalin-fold ligand is not bound to the lipocalin-fold molecule and at least a second conformation when the lipocalin-fold ligand is bound to the lipocalin-fold molecule; and
wherein the lipocalin-fold molecule bound to the lipocalin-fold ligand in the second conformation binds to the lipocalin-fold binding interaction partner with an affinity which is at least 10-fold higher than the affinity of the lipocalin-fold molecule not bound to the lipocalin-fold ligand in the first conformation,
and wherein the lipocalin-fold binding interaction partner is not a naturally occurring protein which has an affinity of <10 $\mu$M to any naturally occurring lipocalin-fold molecule in the presence of any lipocalin-fold ligand.

The present invention is based on the high flexibility of the lipocalin-fold molecule system for binding to its binding partners ("binding interaction partner") with and without involvements of other molecules, especially small molecules which can be used as pharmaceutical agents. Although the system is based on naturally occurring counterparts, the systems of the present invention are artificial. This means that the lipocalin-fold molecule and/or the lipocalin-fold binding interaction partner may be derived from naturally occurring scaffolds by adapting the binding affinities and specificities of the three essential components of the present system (lipocalin-fold molecule, lipocalin-fold ligand and the binding interaction partner) to the binding specificities needed, especially those which are required in a targeted and specific pharmaceutical intervention on human patients.

The feature that the lipocalin-fold binding interaction partner is not a naturally occurring binding partner of the lipocalin-fold molecule is important for reducing or avoiding significant cross-reactivity (which is disadvantageous for the intended use of the present invention for pharmaceutical application). Such cross-reactivity could lead to side-effects which can eventually become severe (such side effects could also be hard to predict, if naturally occurring lipocalin-fold binding interaction partners were part of the "on-switch" system based on a lipocalin-fold molecule).

In the course of the present invention, it was found out that LRPPI systems based on lipocalin-fold molecules have surprisingly advantageous properties which make them excellently suitable for establishing LRPPI systems which have also the capability of working *in vivo* in human pharmaceutical therapy. This is not only based on the fact that lipocalin-fold molecule affinities for lipocalin-fold ligands are easily "tunable", specifically in the micro- and nanomolar range, but also due to the robust architecture of the central structure of the lipocalin-fold molecule (see further disclosure to the "lipocalin-fold", below) and the ability of some lipocalin-fold ligands to bind in the lipocalin-fold molecule. Due to these excellent properties, the LRPPI systems according to the present invention can be designed very specific, robust and sensitive to allow use in human medicine.

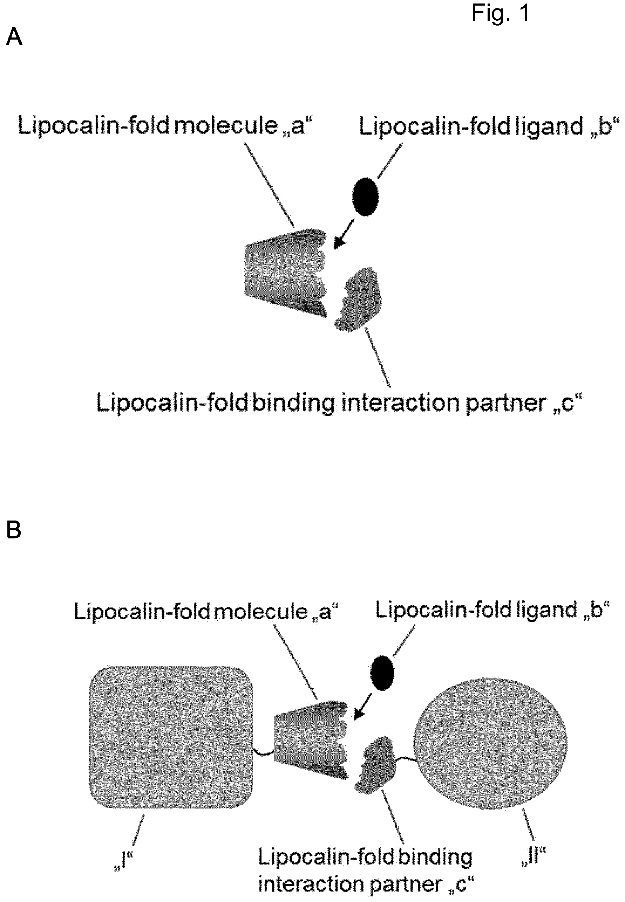

The present LRPPI system is therefore based on three essential components: the lipocalin-fold molecule ("a" in Fig. 1), the lipocalin-fold ligand ("b" in Fig. 1) and the lipocalin-fold binding interaction partner ("c" in Fig. 1).

The "lipocalin-fold molecule" according to the present invention can be any naturally occurring lipocalin-fold molecule or derived version thereof that is part of the "lipocalin-fold" superfamily of proteins according to the Structural Classification of Proteins (SCOP) database (version 1.75) (Murzin et al. J Mol Biol. 1995;247(4):536-540). It is this structural motif of the "lipocalin-fold" proteins that allows the generation of the flexible LRPPI systems according to the present invention. Including the lipocalin-fold molecules in the LRPPI system according to the present invention was thus a crucial step towards enabling flexible engineering of orthogonal LRPPI systems for clinical use. In the course of the present invention, this lipocalin-fold was identified as a uniquely shaped scaffold that can inherently bind a large variety of structurally different small molecules ("lipocalin-fold ligands" (or "ligands") according to the present invention) and can be engineered even for binding of small molecules that initially cannot be captured. A lipocalin-fold protein contains a small characteristic 8- or 10-stranded up-and-down $\beta$-barrel motif, in which the antiparallel $\beta$-strands are arranged in a +1 topology and which has evolved to wrap around mostly hydrophobic small molecule ligands (Lakshmi et al. PLoS One. 2015;10(8): e0135507; Zhang et al., PLoS One. 2012;7(5): e36772; Smathers et al., Hum Genomics. 2011;5(3):170-191; Grzyb et al., J Plant Physiol. 2006;163(9):895-915; Flower et al., Biochim Biophys Acta. 2000;1482(1-2):9-24; Schiefner et al., Acc Chem Res. 2015;48(4):976-985). In the SCOP database (version 1.75) the lipocalin-fold comprises only the lipocalin superfamily (Lakshmi et al. PLoS One. 2015;10(8): e0135507). Among the 9 families that are assigned to the lipocalin superfamily, retinol binding protein-like and fatty acid binding protein-like proteins comprise the lipocalins (LCNs) and intracellular lipid binding proteins (iLBPs), respectively, and are the most relevant families. LCNs are 8-stranded $\beta$-barrel proteins (molecular mass roughly 20 kDa) (Schiefner et al., Acc Chem Res. 2015;48(4):976-985) and iLBPs are 10-stranded $\beta$-barrel proteins (molecular mass roughly 15 kDa) that comprise FABPs (fatty acid binding proteins), CRBPs (cellular retinol binding proteins) and CRABPs (cellular retinoic acid binding proteins) (Smathers et al., Hum Genomics. 2011;5(3):170-191). It is assumed that lipocalin-fold proteins have evolved from a common ancestor by gene duplication and evolutionary divergence for adapting to capture a multitude of different small molecules. LCNs have already evolved in bacteria (>600 LCNs have been described among species; the human genome encodes at least 15 members), and iLBPs have likely evolved in animals after divergence from fungi and plants (the human genome encodes 10 FABPs and 6 retinoid binding proteins; Lakshmi et al. PLoS One. 2015;10(8): e0135507; Zhang et al., PLoS One. 2012;7(5): e36772; Smathers et al., Hum Genomics. 2011;5(3):170-191). All these proteins have maintained a striking structural homology despite very low sequence homology (for some FABPs around 20% and for many LCNs below 30%), which illustrates the extraordinary high tolerance of the β-barrel structure of the lipocalin superfamily to mutations in virtually all regions of the structure for adaption to binding of different ligands. In fact, it has not been possible to define any sequence motif that is common to all members of the lipocalin superfamily, i.e. especially the lipocalins and iLBPs (Flower et al., Biochim Biophys Acta. 2000;1482(1-2):9-24).

This extraordinary flexible β-barrel structure of the lipocalin-fold is used according to the present invention as a broad platform (based on the "lipocalin-fold molecule") for generating a novel family of LRPPI systems that can be regulated by ligands which insert into the hydrophobic pocket of the lipocalin-fold. Such regulating lipocalin-fold ligands can be chosen from a large pool of possible molecules with different characteristics. The ligand-bound lipocalin-fold molecule can be used as a target molecule that is recognized by another engineered protein, i.e. the lipocalin-fold binding interaction partner, in a ligand-dependent manner. That is, the lipocalin-fold binding interaction partner is engineered to specifically recognize the lipocalin-fold molecule with higher affinity if the ligand is inserted into the hydrophobic pocket. This other engineered protein can be, but is not limited to, an antibody, an antibody fragment or any other protein that can be engineered for antigen binding. Alternatively, the ligand-bound lipocalin-fold molecule, i.e. the lipocalin-fold thereof, is engineered for binding to another molecule, i.e. the lipocalin-fold binding interaction partner (e.g. a protein, carbohydrate, lipid, among others) in a ligand-dependent manner. That is, in the ligand-bound state the engineered lipocalin-fold molecule is able to bind to the other interaction partner with strongly enhanced affinity. As a consequence, the ligand can be used for regulating the interaction of the lipocalin-fold molecule with the lipocalin-fold binding interaction partner.

These regulating lipocalin-fold ligands can include clinically applicable molecules with beneficial pharmacokinetics. Moreover, it is possible to select lipocalin-fold ligands that are orthogonal to each other, thereby even enabling separate regulation of multiple processes in parallel. All this founds firstly on the capacity of this β-barrel structure to inherently accommodate a multitude of different ligands in its calyx-like binding pocket and secondly on its unique tolerance to mutations, which enables engineering of high affinity and specificity to a broad spectrum of non-natural ligands as previously disclosed (e.g. DE 19742706 A1, WO 99/016873 A1, EP 1 017 814 B1, WO 2012/065978 A1, WO 2016/113203 A1, Skerra, Biochim Biophys Acta. 2000;1482(1-2):337-350; Korndorfer et al., Proteins 2003;53(1):121-129; Korndorfer et al., J Mol Biol. 2003; 330(2): 385-396; Kim et al., J Am Chem Soc. 2009;131(10):3565-3576; Schlehuber et al., Biophys Chem. 2002;96(2-3):213-228). Just these two unique features of the β-barrel structure enable orthogonalization by engineering of the binding pocket for binding the ligands with high affinity and specificity, instead of redesigning the ligands, which significantly reduces the clinical entry barrier and allows for choosing molecules from a large pool of possible candidates. An additional degree of flexibility and specificity is introduced by lipocalin-fold binding interaction partners that are engineered for specifically recognizing the β-barrels in the ligand-bound state. For this purpose, the ligand-loaded β-barrels (i.e. the ligand-loaded lipocalin-fold molecules) may be used as antigens for binder screening from appropriate libraries. Alternatively, the lipocalin-fold can also be engineered as ligand-dependent binders. The latter is based on the fact that lipocalin-fold molecules can not only be engineered for small molecule binding but also for binding of large proteins. This has been meanwhile exemplified in the form of so-called "anticalins" or "muteins of lipocalin" with a range of protein antigens and has been applied for generating soluble blocking agents and novel tumour binding moieties in chimeric antigen receptors (e.g. DE 19742706 A1, WO 99/016873 A1, EP 1 017 814 B1, WO 2012/065978 A1, WO 2016/113203 A1, Richter et al., FEBS Lett. 2014; 588(2):213-218; Schonfeld et al., Proc Natl Acad Sci USA. 2009;106(20):8198-8203; Gebauer et al., J Mol Biol 2013;425(4):780-802; Barinka et al., Protein Eng Des Sel. 2016;29(3):105-115). Accordingly, there are already numerous examples of lipocalin-fold molecules available in the prior art which may be applied in the LRPPI system according to the present invention, such as the naturally occurring lipocalins and engineered lipocalins ("anticalins", "muteins of lipocalin", etc.). In contrast to the previous strategies provided on the basis of engineering LCN-based binder scaffolds, the present invention provides the engineering of lipocalin-fold based LRPPI systems for regulating PPIs by addition of small molecules.

Accordingly, any molecule comprising the lipocalin-fold as the central structural element may be used or adapted to be used in the LRPPI system according to the present invention. The LRPPI systems according to the present invention can easily be optimised and tuned with respect to affinity of the lipocalin-fold molecules to lipocalin-fold ligands and also with respect to differences in the affinity of lipocalin-fold molecules to lipocalin-fold binding interaction partners in absence and presence of lipocalin-fold ligands. In the present invention the "bound" or "unbound" state of the LRPPI system, i.e., a lipocalin-fold molecule "bound" or "unbound" to a lipocalin-fold ligand, is referred to a difference in affinity of the lipocalin-fold molecule to the lipocalin-fold binding interaction partner of at least ten-fold. However, preferred embodiments apply an even more significant affinity difference between the (at least) two states of the lipocalin-fold molecules in absence and presence of lipocalin-fold ligands. This is why the difference in affinity is preferably at least 20-fold, especially at least 50-fold. The present invention allows differences in affinity to be designed and tuned even further, e.g. at least 100-fold, at least 200-fold, at least 500-fold or at least 1000-fold. This increase in affinity difference may be specifically advantageous in the human therapy environment, especially as a safety measure to exclude unwanted side effects or toxicity.

Although the LRPPI systems according to the present invention are based on the advantageous properties of the naturally occurring lipocalin-fold molecules, the LRPPI systems according to the present invention are artificial systems which are designed to provide a suitable pharmaceutical system. This means that the LRPPI systems according to the present invention cannot have a naturally occurring counterpart (because this could not be used for the purpose intended by the present invention (since the natural systems have to fulfil their naturally intended purpose)). In this context, it is important to note here that in the course of the present invention the surprising observation was made that the LRPPI systems according to the present invention are highly flexible regarding the type (i.e. the structure (or fold)) of the lipocalin-fold binding interaction partner. The LRPPI systems described in Example 1 of the present invention demonstrate that different types of proteins with structurally very different binding sites (located on either rigid β-strands or loop regions, respectively) can be used as lipocalin-fold binding interaction partners. Moreover, the proteins used as lipocalin-fold binding interaction partners in Example 1 are not (and are not mutants of) any naturally occurring lipocalin-fold binding interaction partners. Instead, the lipocalin-fold binding interaction partners used in Example 1 are either mutated versions of the protein Sso7d (a DNA-binding protein from the archaeon *Sulfolobus solfataricus*) or mutated versions of the 10th type III domain of human fibronectin (FN3), demonstrating that structurally distinct proteins (or protein domains) derived from molecules with completely different original function can efficiently act as lipocalin-fold binding interaction partners according to the present invention. Accordingly, to create LRPPI systems which are more independent from naturally occurring PPI systems and to reduce the risk of disadvantageous cross-reactivity with endogenous lipocalin-fold binding interaction partners, the lipocalin-fold binding interaction partners in the LRPPI systems according to the present invention are not (and are preferably also not derived from) naturally occurring lipocalin-fold binding interaction partners. More precisely, the lipocalin-fold binding interaction partner (or any domain of it that mediates binding to the lipocalin-fold molecule) is not and is preferably also not derived from a naturally occurring protein which has an affinity of <10 μM to any naturally occurring lipocalin-fold molecule (in the presence of any lipocalin-fold ligand), wherein "being derived from" is defined as containing at least one segment of at least 50 consecutive amino acids with an amino acid sequence that is identical with the amino acid sequence of any segment of that naturally occurring protein which has an affinity of <10 μM to any naturally occurring lipocalin-fold molecule (in the presence of any lipocalin-fold ligand).

Accordingly, a preferred embodiment of the LRPPI system according to the present invention employs a lipocalin-fold binding interaction partner that does not contain a segment of at least 50 consecutive amino acids with an amino acid sequence that is identical with the amino acid sequence of any segment of a naturally occurring protein which has an affinity of <10 μM to any naturally occurring lipocalin-fold molecule, especially in the presence of a lipocalin-fold ligand. Preferably, the lipocalin-fold binding interaction partner does not contain a domain of a naturally occurring protein that mediates binding to a naturally occurring lipocalin-fold molecule. This further safeguards lack of cross-reactivity. Preferably, the affinity of the lipocalin-fold binding interaction partner to the lipocalin-fold molecule when bound to the lipocalin-fold ligand or in the second conformation, respectively, is below 10 μM, preferably below 2 μM, especially below 400 nM.

In order to further lowering the risk of immunogenicity in a human patient, the ligand regulated protein-protein interaction system according to the present invention preferably comprises a lipocalin-fold binding interaction partner that is not a homolog of a different species than human of a naturally occurring human lipocalin-fold binding interaction partner. Even more preferred, the lipocalin-fold molecule is not even a homolog of a different species than human of a naturally occurring human lipocalin-fold molecule.

Preferred ligand regulated protein-protein interaction systems according to the present invention apply molecules as lipocalin-fold ligands which are useable in a pharmaceutical environment, preferably molecules which are suitable and appropriate to be applied to humans. Accordingly, preferred embodiments of the present invention comprise a lipocalin-fold ligand which is a pharmaceutically active molecule, especially a pharmaceutically active molecule with a therapeutic activity in human patients. In this connection, molecules are preferred as lipocalin-fold ligands which can be effectively administered orally. This means that such molecules are suitable for oral administration and are taken up by the individual to whom the molecule is administered through intestinal absorption. Also molecules are preferred as lipocalin-fold ligands which can be effectively administered intravenously. This means that the molecule can be administered intravenously without significant side effects to a patient. Specifically preferred lipocalin-fold ligands are suitable for effective administration in both manners, intravenously and orally, to a human patient. Preferred lipocalin-fold ligands are therefore molecules which are (or have been) registered drugs for human use for which e.g. a valid marketing authorisation is present, e.g. in either the EU or the US, or both.

This artificial character of the LRPPI systems according to the present invention enables the proper regulation of the system (e.g. by the small molecule ligand) also *in vivo* (as needed to solve the object of the present invention). In a specifically preferred embodiment, both main system components of the ligand regulated protein-protein interaction system according to the present invention, namely, the lipocalin-fold molecule and the lipocalin-fold binding interaction partner and/or any domain of them that mediates binding are not part of a naturally occurring LRPPI system, i.e. a biological pathway, wherein the physiological function is performed by such a system. Accordingly, in such a preferred embodiment, both the lipocalin-fold molecule and the lipocalin-fold binding interaction partner are therefore not naturally occurring proteins, but mutated or artificially designed non-natural proteins (i.e. proteins with no native counterpart existing in nature).

Moreover, in order to design an LRPPI system that is independent from naturally occurring LRPPI or PPI systems, the LRPPI systems according to the present invention are artificial systems in which preferably the lipocalin-fold molecule and the lipocalin-fold binding interaction partner are not derived from naturally occurring molecules that bind to each other with an affinity of <10 μM. This means that (1) if the lipocalin-fold molecule used in a given LRPPI system according to the present invention is engineered, the naturally occurring lipocalin-fold molecule, that it is derived from, does not bind with an affinity of <10 μM to the lipocalin-fold binding interaction partner used in that LRPPI system; or (2) as the lipocalin-fold binding interaction partner used in a given LRPPI system according to the present invention is engineered, the naturally occurring molecule, that this lipocalin-fold binding interaction partner is derived from, does not bind with an affinity of <10 μM to the lipocalin-fold molecule used in that LRPPI system; or (3) if both the lipocalin-fold molecule and the lipocalin-fold binding interaction partner used in a given LRPPI system according to the present invention are engineered, the naturally occurring molecules, which the lipocalin-fold molecule and the lipocalin-fold binding interaction partner are derived from, respectively, do not bind to each other with an affinity of <10 μM.

Moreover, to avoid any disadvantageous cross-reactivity, the lipocalin-fold binding interaction partner used in a given LRPPI system according to the present invention preferably does not bind with an affinity of <1 μM (neither in the presence nor in the absence of a lipocalin-fold ligand) to any naturally occurring lipocalin-fold molecule except for (1) the naturally occurring lipocalin-fold molecule used in that LRPPI system (if a naturally occurring lipocalin-fold molecule is used in that LRPPI system) or the naturally occurring lipocalin-fold molecule that the β-barrel sequence of the engineered lipocalin-fold molecule used in that LRPPI system was derived from and except for (2) the naturally occurring lipocalin-fold molecules which are homologs (i.e. homologous lipocalin-fold molecules from other species) of the naturally occurring lipocalin-fold molecule used in that LRPPI system (if a naturally occurring lipocalin-fold molecule is used in that LRPPI system) or the naturally occurring lipocalin-fold molecules which are homologs of the naturally occurring lipocalin-fold molecule that the β-barrel sequence of the engineered lipocalin-fold molecule used in that LRPPI system was derived from.

A highly attractive field for clinical application of LRPPI systems based on lipocalin-fold molecules according to the present invention is for regulating the function of T cells modified with chimeric antigen receptors (CARs) instead of using the FKBP-based systems for homo- and heterodimerization for regulating the CAR T cell function. Importantly, the function of currently applied CARs cannot be regulated in a clinically applicable manner. Instead, inducing apoptosis in the CAR expressing effector cells is the only clinically applicable safety mechanism in the CAR field until now (Jones et al., Front Pharmacol. 2014;5:254). The most advanced strategies for regulating the function of the CAR molecule itself employ the FRB/FKBP-based system in different versions either for LRPPI (WO 2014/127261 A1, WO 2015/017214 A1, EP 3 087 101 A1, US 2017/0081411 A1) or for regulating protein stability (WO 2017/032777 A1). As mentioned above, however, the FRB/FKBP system is associated with severe problems in any potential clinical application (Sun et al., Cell Res. 2015;25(12):1281-1282). Thus, alternatively, the lipocalin-fold molecule based LRPPI systems according to the present invention are highly attractive for integration into CARs in order to control T cell activation upon CAR mediated target antigen recognition by administering small molecules. In contrast to the rather problematic FRB/FBKP-system the lipocalin-fold based LRPPI systems according to the present invention can pave the way for broad clinical application of switchable CARs.

The LRPPI system according to the present invention and shown in Figure 1 can be engineered using two strategies in principle: In strategy A, the lipocalin-fold binding interaction partner "c" (which may preferably be fused (+/- flexible linker) to a protein "II") is a binder, which binds to the barrel structure of the lipocalin-fold molecule "a" with higher affinity when lipocalin-fold ligand "b" is present. The lipocalin-fold binding interaction partner "c" can e.g. be generated either by immunization of animals with ligand ("b")-loaded lipocalin-fold molecule, or by state of the art protein engineering methods such as phage display, yeast display, bacterial display, mammalian cell display, ribosome display, mRNA display or covalent DNA display, among others (Sergeeva et al., Advanced Drug Delivery Reviews 2006;58:1622-1654). In strategy B, the lipocalin-fold molecule "a" itself can be engineered as a binder that after loading with a lipocalin-fold ligand "b" can bind with higher affinity to a chosen lipocalin-fold binding interaction partner "c" (which again can (according to a preferred embodiment) be fused to protein "II" or itself is a protein or non-protein antigen "II" (e.g. a tumor associated antigen)). The lipocalin-fold molecule "a" and the lipocalin-fold binding interaction partner "c" can be fused to the N- or C-termini or also internal sites of proteins "I" and "II" (see Fig. 1B). The lipocalin-fold molecule "a" may be engineered for increasing the affinity to a chosen small molecule lipocalin-fold ligand "b" and/or for decreasing the affinity towards endogenous natural lipocalin-fold ligands. Furthermore, the lipocalin-fold structure of lipocalin-fold molecule "a" may also be engineered for preventing or reducing interaction with natural interaction partners.

Preferably, the lipocalin-fold ligand induces a conformational change in the lipocalin-fold molecule "a", which facilitates the selection of a ligand-dependent binder (lipocalin-fold binding interaction partner) "c" (or also the selection of a ligand-dependent lipocalin-fold molecule "a" when in turn used as a binder). The lipocalin-fold ligand "b" can, but does not need to, interact directly with "c" or protein "II". However, in cases where binding of molecule "b" results only in rigidification of the structure of the lipocalin-fold molecule "a" with rather limited conformational selection, any direct contribution of molecule "b" for increasing the affinity of the interaction of "a" and "c" is beneficial.

Depending on the application, the LRPPI system can - according to a specific embodiment - be further employed in a strategic variant for regulating dimerization of two identical or different lipocalin-fold molecules (the first one being the lipocalin-fold molecule "a" according to the present invention, the second one being a "lipocalin-fold binding interaction partner" in the system according to the present invention (although being a "lipocalin-fold molecule", such second lipocalin-fold molecule would serve as the binding partner of the first lipocalin-fold molecule according to the present invention)). Accordingly, this specific embodiment uses lipocalin-fold ligands "b" with two identical or different head groups, respectively, to form a LRPPI with two lipocalin-fold molecules (different or the same (i.e. homodimerization or hetereodimerization) ), in analogy to existing LRPPI systems (Rutkowska et al., Angew Chem Int Ed Engl. 2012;51(33):8166-8176). One of the two lipocalin-fold molecules serves then (formally) as a "lipocalin-fold binding interaction partner" within the definitions in the system according to the present invention.

As already outlined above, the lipocalin-fold molecule according to the present invention may be any suitable molecule comprising the structural element of a lipocalin-fold. The lipocalin-fold of the lipocalin-fold molecule according to the present invention ("a") can be derived from the $\beta$-barrel of any known member of the LCN protein family or from the $\beta$-barrel of the iLBP protein family. This also includes LCN- and iLBP-variants, which deviate in the numbers of $\beta$-strands from the prototypic architecture as has been reported, e.g., for some LCNs (Papiz et al., Nature. 1986;324(6095):383-385; Spinelli et al., Eur J Biochem. 2002;269(10):2449-2456; Sevvana et al., J Mol Biol. 2010;404(3):363-371). As LCNs have evolved for extracellular transport of small molecules and iLBPs have evolved for intracellular transport of many of the same molecules, the two $\beta$-barrel variants offer different advantages for application in oxidizing versus reducing environments outside and inside of cells. In principle, the lipocalin-fold of both families are suited for the flexible LRPPI systems according to the present invention as they have very similar structure and all can accommodate a large variety of lipocalin-fold ligands in their binding pocket with high affinity. However, lipocalin-fold molecules with known lipocalin-fold ligand induced conformational adaption and variants enabling a direct contribution of the lipocalin-fold ligand "b" in the interaction with the lipocalin-fold binding interaction partner "c" are preferred. The organisms of origin of the lipocalin-fold molecule can be selected according to the target organisms, in which the proteins are supposed to be expressed at maximum levels and with correct posttranslational modification. In this context LCNs have the advantage that they have evolved already in bacteria. However, the intended primary advantage of the $\beta$-barrel based LRPPI systems is their clinical applicability, thus, lipocalin-fold molecules of human protein origin are preferred.

Currently, human LCNs comprise 15 well characterized proteins and a couple of yet elusive members (Schiefner et al., Acc Chem Res. 2015;48(4):976-985). For the well characterized LCNs a large diversity in the size and shape of the binding pockets has been described as well as an accordingly highly diverse spectrum of lipocalin-fold ligands such as, e.g., simple fatty acids, glyco- and phospholipids, all-trans retinol, cholesterol, vanillin, imatinib, staurosporine, or even large, complex molecules such as bacillibactin and heme. This adaptiveness has been found to particularly depend on the length and the sequence of the four loops at the entry site of the $\beta$-barrel (Schiefner et al., Acc Chem Res. 2015;48(4):976-985; Skerra, Biochim Biophys Acta. 2000;1482(1-2):337-350; Korndorfer et al., Proteins 2003;53(1):121-129; Korndorfer et al., J Mol Biol. 2003;330(2) :385-396; Kim et al., J Am Chem Soc. 2009;131(10):3565-3576; Schlehuber et al., Biophys Chem. 2002;96(2-3):213-228; Richter et al., FEBS Lett. 2014;588(2):213-218). Extensive conformational changes upon binding of different lipocalin-fold ligands so far have been reported for human and bovine retinol binding protein 4 (RBP4), human tear lipocalin (TLC) and human apolipoprotein M (Ap-oM) (Berni et al., FEBS Lett. 1992;308(1) :43-45; Zanotti et al., J Biol Chem. 1993;268(33):24873-24879; Zanotti et al., J Biol Chem. 1994;269(47) :29613-29620; Pattanayek et al., Protein Sci. 1999;8(10):2027-2032; Motani et al., J Biol Chem. 2009;284(12):7673-7680; Gasymov et al., Biochim Biophys Acta. 1998;1386(1):145-156; Breustedt et al., Acta Crystallogr D Biol Crystallogr. 2009;65(Pt 10):1118-1125; Gasymov et al., Biochemistry. 2012;51(14):2991-3002; Zhang et al., Sci Rep. 2016;6:30655; Christoffersen et al., Proc Natl Acad Sci USA. 2011;108(23):9613-9618) . Among them, human TLC is characterized by elevated conformational flexibility and an extraordinary flexible binding behavior compared to other LCNs (Schiefner et al., Acc Chem Res. 2015;48(4):976-985; Breustedt et al., Acta Crystallogr D Biol Crystallogr. 2009;65(Pt 10):1118-1125; Gasymov et al., Biochemistry. 2012;51(14):2991-3002). In the case of the particularly well characterized native human RBP4 there are two loop regions, which undergo conformational alteration upon lipocalin-fold ligand binding and are involved in interaction with natural interaction partners transthyretin (TTR) (via EF loop, residues 89-101) and the receptor STRA6 (via CD loop, residues 59-68; Redondo et al., FASEB J. 2008;22(4):1043-1054). Meanwhile, several natural and synthetic retinoid and non-retinoid lipocalin-fold ligands for human RBP4 have been described (i.e., Fenretinide, N-Ethylretinamide, all-trans retinoic acid, retinyl acetate, axerophthene, A1120 (PubChem CID 25138295)), which induce conformational changes resulting in dissociation from TTR (Berni et al., FEBS Lett. 1992;308(1):43-45; Zanotti et al., J Biol Chem.

1993;268(33):24873-24879; Zanotti et al., J Biol Chem. 1994;269(47) :29613-29620; Motani et al., J Biol Chem. 2009;284(12) :7673-7680; Coward et al., Anal Biochem. 2009;384(2) :312-320; Sharif et al., Anal Biochem. 2009;392(2):162-168;). Reported crystal structures for human RBP4 illustrate that different lipocalin-fold ligands can induce different conformations in distinct loop regions of the LCN (e.g., protein data bank (PDB) 1RBP, 3FMZ and 2WR6 for retinol, A1120 and linoleic acid, respectively). Similar effects have been reported for ApoM (PDB 2YG2 and 2WEW for sphingosine-1-phosphate versus myristic acid; Christoffersen et al., Proc Natl Acad Sci USA. 2011;108(23):9613-9618).

For selecting appropriate lipocalin-fold molecules according to the present invention, differences with regard to existence of glycosylation sites, free cysteines, disulfide bridges, oligomerization behavior, ligand spectrum etc., and the necessity for removing more or less characterized interaction sites for preventing interaction with natural protein partners or lipid membranes can be considered (Schiefner et al., Acc Chem Res. 2015;48(4):976-985). Among the human LCNs, RBP4, TLC and ApoM (UniProt IDs P02753, P31025, and 095445, respectively) are characterized by already known lipocalin-fold ligand induced conformational adaption and are thus preferred members of the lipocalin family for generating LRPPI systems according to the present invention. One additional preferred lipocalin molecule is the human neutrophil gelatinase-associated lipocalin (NGAL) (UniProt ID P80188), for which a very detailed structural knowledge with respect to interacting amino acid residues and binding pocket engineering has accumulated (Kim et al., J Am Chem Soc. 2009;131(10):3565-3576; Schonfeld et al., Proc Natl Acad Sci USA. 2009;106(20):8198-8203; Barinka et al., Protein Eng Des Sel. 2016;29(3):105-115; Gebauer et al., J Mol Biol 2013;425(4):780-802; Bao et al., RSC Adv. 2015;5(126) :104363-104374; Eggenstein et al., J Struct Biol. 2014;185(2):203-214). This is in particular due to the fact that its ligand binding pocket can easily be modified and was the basis of structurally resolved anticalins engineered for high affinity binding against different ligands.

Importantly, β-barrels of LCNs are not only an option for extracellular use but also for intracellular use of the LRPPI systems. This is based on the fact that, although all human LCNs have at least one disulfide bridge in their β-barrel structure, the β-barrel of, e.g., human TLC is functional and sufficiently stable also in the fully reduced state (Gasymov et al., Biochim Biophys Acta. 2011;1814(5):671-683). Other LCNs could be more affected under reducing conditions, however, these proteins can be stabilized by e.g. inserting stabilizing mutations. The latter has been exemplified for a zinc-binding mutant of human RBP4, which was stabilized by introducing the five mutations A43L, A55V, A57I, H104W and Q117I (Skerra; Biochim Biophys Acta. 2000;1482(1-2):337-35049; Schmidt, Untersuchungen zur Protein-faltung durch Protein-Design am Retinol-Bindungsprotein. Vol. ISBN 3-89675-314-2. Munchen: Herbert Utz Verlag; 1998).

The LCN-derived β-barrels preferably represent the full-length coding sequences; signal peptides can be replaced; both, the N- and the C-terminal ends of the full length or trimmed β-barrels (i.e. lipocalin molecules) are suited for fusion to protein partners (with or without linker sequences), protein domains, peptides or single amino acids since the termini of the β-barrels are not involved in ligand binding (Skerra; Biochim Biophys Acta. 2000;1482(1-2):337-35049).

Possible sequence modifications can comprise:

a) Preferably, the engineering of the binding pocket of the lipocalin-fold molecules for increasing the affinity to a chosen ligand "b" and/or lowering the affinity to other lipocalin-fold ligands by directed evolution including any sort of random mutagenesis and subsequent selection or screening processes, such as phage display, yeast display, bacterial display, mammalian cell display, ribosome display, mRNA display or covalent DNA display, among others (Sergeeva et al., Advanced Drug Delivery Reviews 2006;58:1622-1654). Alternatively, mutations can be based on in silico calculations and subsequently be introduced by site-directed mutagenesis (Whitehead et al., Methods Enzymol. 2013;523:1-19; Strauch et al., Proc Natl Acad Sci USA. 2014 Jan 14;111(2):675-80). Such engineering processes can require only limited mutagenesis at specific sites. If small molecules are chosen that initially do not bind the lipopocalin-fold, then more extensive mutagenesis in or nearby the center of the binding pocket of the β-barrel may be required for generating mutants with binding capability (as described in DE 19742706 A1 and exemplified for several small molecules in Korndorfer et al., Proteins 2003;53(1):121-129; Korndorfer et al., J Mol Biol. 2003; 330(2):385-396; Kim et al., J Am Chem Soc. 2009;131(10):3565-3576; Schlehuber et al., Biophys Chem. 2002;96(2-3):213-228). In the preferred case, this process is compatible with screening for lipocalin-fold ligand induced conformational changes. Such a screening process is feasible by employing proteins that can bind to these lipocalin-fold molecules in the absence of any ligand but dissociate upon lipocalin-fold ligand binding for selecting lipocalin-fold molecule mutants, which have maintained conformational switch behaviour. Such a protein can be the natural protein TTR in the case of RBP4 or other natural binding partners that bind to the respective lipocalin-fold molecule. Alternatively, binders, which have been separately engineered for binding to a chosen lipocalin-fold molecule in the absence of a ligand, may also be used for such a screening process. In a typical screening process, the lipocalin-fold molecule libraries are alternately screened for mutants that are capable for binding to these proteins in the absence of any ligand. Then the capability for lipocalin-fold ligand binding and conformational switching of the lipocalin-fold molecule is concomitantly selected by screening of the lipocalin-fold molecule libraries for non-binding to these proteins, or binding with lower affinity, in the presence of the lipocalin-fold ligand.

b) Additional mutagenesis (possibly, but not necessarily, after engineering of the binding pocket of the lipocalin-fold molecule for lipocalin-fold ligand binding) with a focus on the loop regions (as described in DE 19742706 A1), if the lipocalin-fold molecule is intended for use as a binder in the LRPPI system (instead of using it as an antigen); preferably, the lipocalin-fold molecule is engineered by directed evolution including any sort of random mutagenesis and subsequent selection or screening processes, such as phage display, yeast display, bacterial display, mammalian cell display, ribosome display, mRNA display or covalent DNA display, among others (Sergeeva et al., Advanced Drug Delivery Reviews 2006;58:1622-1654). Maintenance of the capability for small molecule binding of the lipocalin-fold molecules is warranted by alternating screening for antigen binding of the lipocalin-fold molecules in presence of the lipocalin-fold ligand and for non-binding (or binding with lower affinity) in the absence of the lipocalin-fold ligand.

c) Mutation/deletion/insertion of residues for preventing dimerization or interaction with other proteins or lipid membranes (e.g., free cysteines, unprocessed signal peptide in Ap-oM, loop CD residues 59-68 in RBP4, etc.; see e.g. Skerra, Biochim Biophys Acta. 2000;1482(1-2):337-350; Zhang et al., Sci Rep. 2016;6:30655; Redondo et al., FASEB J. 2008;22(4):1043-1054) .

d) Mutation/deletion/insertion of residues for preventing post translational protein modification.

e) Engineering the lipocalin-fold molecule for improved stability, e.g., under reducing conditions in the cytoplasm as has been demonstrated for antibody fragments (Worn et al., J Biol Chem. 2000;275(4):2795-2803). This can be achieved, for example, by rational design of stabilizing mutations or by directed evolution experiments which select for improved stability (Traxlmayr et al, Biochim Biophys Acta. 2012;1824(4):542-549). However, also any other method for stabilization of proteins is possible.

The human intracellular iLBP protein family comprises a group of 6 retinoid binding proteins termed CRBPs and CRABPs, and the group of 10 fatty acid binding proteins (FABPs). Although iLBPs are intracellular proteins, some of them, e.g., FABP4 may have a function also in the extracellular space (Hotamisligil et al., Nat Rev Endocrinol. 2015; 11(10):592-605) . All iLBPs have the same architecture with 10 anti-parallel β-strands connected by more or less elongated loops with the exception of an intervening helix-turn-helix motif between strands βA and βB (Lakshmi et al. PLoS One. 2015;10(8): e0135507; Zhang et al., PLoS One. 2012;7(5): e36772; Smathers et al., Hum Genomics. 2011;5(3):170-191). Compared to LCNs their barrel structure is more compact and their ligands are hardly exposed to the solvent due to shielding by the helix-turn-helix motif at the entrance. However, like LCNs iLBPs have adapted to binding of a diverse spectrum of partially shared ligands and they also have in common the high tolerance to mutagenesis. The latter includes even the complete deletion of the helix-turn-helix motif at the barrel entrance and substitution by a simple loop (Curto et al., Protein Sci. 2009;18(4):735-746; Ogbay et al., Protein Sci. 2004;13(5):1227-1237). Among the iLBPs, FABP1 and FABP6 are characterized by higher backbone flexibility and a larger ligand entrance, resulting in their unique capacity to accommodate bulky molecules and two of each. Binding of the so far tested ligands generally revealed only small conformational changes due to ligand induced rigidification (Yu et al., Sci Rep. 2016;6:34171; Sharma et al., J Biol Chem. 2011;286(36):31924-31928; Cai et al., Biophys J. 2012;102(11):2585-2594; Franzoni et al., J Lipid Res. 2010;51(6) :1332-1343; Vaezeslami et al., J Mol Biol. 2006;363(3):687-701; Gillilan et al., J Mol Biol. 2007;372(5):1246-1260; Menozzi et al., J Struct Biol. 2017;197(3):330-339; Long et al., Biophys J. 2010;98(12):3054-3061) . Obviously, these changes are sufficient to control interaction with other proteins and to mediate, e.g., nuclear transport and interaction with nuclear receptors, as well as, e.g., shuttling retinoids within the cell by mediating interaction with the receptor STRA6 in the cytoplasmic membrane in the case of CRBP-I (Gillilan et al., J Mol Biol. 2007;372(5) :1246-1260; Armstrong et al., J Biol Chem. 2014;289(21):14941-14954; Amber-Vitos et al., PLoS One. 2015;10(8):e0132138; Berry et al., Mol Cell Biol. 2012;32(15):3164-3175; Sessler et al., Mol Cell. 2005;18(3):343-353; Hofer et al., J Biol Chem. 2015;290(30) :18438-18453; Furuhashi et al., Nat Rev Drug Discov. 2008;7(6):489-503). Some iLBPs thereby interact via the formation/selection of a structural nuclear location signal (NLS) within the α2-helix at the entry of the calyx, which is elicited by some but not all of their small molecule ligands (Furuhashi et al., Nat Rev Drug Discov. 2008;7(6):489-503).

Like with LCNs, the human variants of iLBPs are preferred for use in a LRPPI system if applied in humans *in vivo*. Among them the retinoid binding proteins, in particular the very well characterized CRABP-II (UniProt ID P29373), are preferred due to their retinoid ligand specificity (Zhang et al., PLoS One. 2012;7(5): e36772; Franzoni et al., J Lipid Res. 2010;51(6):1332-1343; Vaezeslami et al., J Mol Biol. 2006;363(3):687-701; Menozzi et al., J Struct Biol. 2017;197(3):330-339). Furthermore, FABP2 (P12104), for which the helix-turn-helix substitution was exemplified, and FABP1 (P07148) due to their known low affinity to several clinically approved lipophilic drugs are also attractive iLBP members for engineering the binding pocket for recognition of clinically applicable ligands (Smathers et al., Hum Genomics. 2011;5(3):170-191; Curto et al., Protein Sci. 2009;18(4):735-746; Ogbay et al., Protein Sci. 2004;13(5):1227-1237; Velkov T, Chem Biol. 2007;14(4):453-465; Velkov T. PPAR Res. 2013;2013:938401; Beringhelli T, PLoS One. 2015;10(7):e0132096; Chuang S, J Med Chem. 2008;51(13) :3755-3764) .

For integration into the LRPPI system according to the present invention, fusion of the β-barrels of iLBPs to protein partners (with or without linker sequences), protein domains, peptides or single amino acids is possible via the N- and the C-terminal ends (of full length or trimmed iLBP proteins). The sequences are preferably modified for preventing unwanted interaction with their natural protein partners in the cytoplasm by modifying or deleting the respective interacting sequence elements (e.g., the helix-turn-helix motif containing the hidden structural NLS in the α2-helix and other interaction sites; Gillilan et al., J Mol Biol. 2007;372(5):1246-1260; Armstrong et al., J Biol Chem. 2014;289(21):14941-14954; Amber-Vitos et al., PLoS One. 2015;10(8):e0132138; Berry et al., Mol Cell Biol. 2012;32(15):3164-3175; Sessler et al., Mol Cell. 2005;18(3): 343-353). For increasing the affinity to selected lipocalin-fold ligands ("b") and/or for decreasing the affinity to their endogenous small molecule ligands, the iLBPs may be engineered similarly to LCNs. To facilitate the engineering of lipocalin-fold ligand dependent recognition, it is possible to substitute the helix-turn-helix motif by a loop sequence (as exemplified by Curto et al. and Ogbay et al. (Curto et al., Protein Sci. 2009;18(4):735-746; Ogbay et al., Protein Sci. 2004;13(5):1227-1237) .

The rationale of developing the LRPPI system according to the present invention (i.e. based on the "lipocalin-fold" of the lipocalin-fold molecule) is to maximize the freedom of choice in selecting the lipocalin-fold ligands ("b"). The crucial innovation for enabling this freedom is based on proteins of the superfamily of lipocalin proteins that contain the "lipocalin-fold" structure. Their key feature, i.e., the characteristic deep binding pocket with a calyx like shape and high structural flexibility is crucial for the flexibility and reliability of the present invention. As a consequence, these binding pockets can be engineered by relatively few mutations for binding to a broad range of small molecule ligands with diverse structural and biophysical characteristics. This has been exemplified with the proteins BBP and NGAL, which were engineered by substitution of 12 to 17 amino acid residues for high affinity binding to originally non-binding small molecules such as fluorescein, digoxigenin, digitoxigenin and a diaminepentaacetic acid (DTPA) based chelator (Korndorfer et al., Proteins 2003;53(1):121-129; Korndorfer et al., J Mol Biol. 2003;330(2):385-396; Kim et al., J Am Chem Soc. 2009;131(10):3565-3576). Thus, contrary to existing LRPPI systems, the novel system according to the present invention is defined by employing lipocalin-fold containing proteins (i.e., lipocalin-fold molecules) and neither by any definitive list of possible small molecules (i.e., lipocalin-fold ligands) nor by any specific structural and chemical properties of such molecules.

The lipocalin-fold ligand "b" according to the present invention is a "small molecule", e.g. "small" compared to polypeptides and proteins, such as the lipocalin-fold molecule. Accordingly, the lipocalin-fold ligand according to the present invention has a molecular weight of 1500 Da or less, preferably 1000 Da or less, especially 750 Da or less. It may be as small as, e.g. glycine (75 Da) or even below, provided that it allows a specific binding within the LRPPI system according to the present invention (i.e. binding to the lipocalin-fold molecule). Accordingly, preferred Mw ranges of the lipocalin-fold ligand of the present invention are 50 to 1500 Da, preferably 75 to 1500 Da, especially 150 to 750 Da. Preferably, the lipocalin-fold ligand can bind in the calyx of the lipocalin-fold molecule formed by the barrel and the loop regions of the lipocalin-fold structure. The lipocalin-fold ligand has a substantial and specific affinity to the lipocalin-fold molecule (in at least one conformation of the lipocalin-fold molecule) which may be 1 mM or lower, preferably 100 μM or lower, especially 10 μM or lower. Such affinity can be increased by directed evolution of the binding pocket of the lipocalin-fold molecules. Although up to 30 mutations or more may be applied to a given lipocalin-fold molecule (see, for example, Schonfeld et al., Proc Natl Acad Sci USA. 2009;106(20):8198-8203), it is usually not necessary to exchange more than 25 or more than 20 to significantly increase the affinity to a given lipocalin-fold ligand (see, for example, Kim et al., J Am Chem Soc. 2009;131(10):3565-3576; Korndorfer et al., Proteins 2003;53(1):121-129; Korndorfer et al., J Mol Biol. 2003; 330 (2) :385-396; Gebauer et al., J Mol Biol 2013;425(4):780-802). Methods for generating lipocalin-fold molecules with an improved affinity towards the lipocalin-fold ligand are well available in the art (see above). Usually, only a few mutations are necessary for a significant increase in affinity towards a given lipocalin-fold ligand, for example only one, two or less, three or less, four or less, five or less, six or less, seven or less, eight or less, nine or less, or ten or less. The capability of a lipocalin-fold ligand "b" to induce a conformational alteration in the lipocalin-fold molecule "a" and/or to positively affect the affinity of a lipocalin-fold binding interaction partner "c" by direct interaction can be predicted to some extent using pharmacophores from existing small molecule ligands with such function. Additionally, this functional capability can be screened for by employing binding proteins, which have a conformation specific binding affinity for the lipocalin-fold molecule (detailed in Example 2, below, and in Coward et al. Anal Biochem. 2009;384(2):312-320, Sharif et al., Anal Biochem. 2009;392(2):162-168 and Dobri et al., Invest Ophthalmol Vis Sci. 2013;54(1):85-95). In the case of RBP4, e.g., TTR can be used as a naturally occurring conformation specific binding protein. If there is no such protein available, as for example for TLC or ApoM, which have also been reported to undergo lipocalin-fold ligand dependent conformational switching (Gasymov et al., Biochim Biophys Acta. 1998;1386(1) :145-156; Breustedt et al., Acta Crystallogr D Biol Crystallogr. 2009;65(Pt 10) :1118-1125; Zhang et al., Sci Rep. 2016;6:30655; Christoffersen et al., Proc Natl Acad Sci USA. 2011;108(23):9613-9618), then it is possible to first generate a protein-based binder that interacts with the unloaded lipocalin-fold molecule, i.e. in the absence of lipocalin-fold ligands, by a process of alternating selection for binding to the lipocalin-fold molecule in the absence and non-binding (or binding with reduced affinity) in the presence of lipocalin-fold ligands known to shift conformational states of the lipocalin-fold molecule. This alternating selection strategy ensures that a binder is selected, which specifically recognizes the unloaded (i.e. not loaded with any lipocalin-fold ligand) state of the lipocalin-fold molecule. In Example 1 we exemplified this process of alternating screening in presence and absence of a lipocalin-fold ligand (A1120) in opposite direction for selecting a lipocalin-fold binding interaction partner, i.e., a protein that binds RBP4 (i.e. the lipocalin-fold molecule) with increased affinity in presence of the lipocalin-fold ligand A1120.

Typically, the process of selecting lipocalin-fold ligand molecules can start from:

1. any given molecule with or without initial binding affinity
2. any existing compound libraries for high throughput screening for binding affinity and/or function
3. any structural databases of compounds that can be used in virtual screening for binding and/or function

Ad 1: Initial binding affinity is not required in this approach, since the binding pocket of the lipocalin-fold molecule can be engineered for binding (see e.g. DE 19742706 A1). The feasibility of engineering of lipocalin-fold molecules for high affinity binding to arbitrary, initially non-binding, molecules has been exemplified by engineering of BBP and NGAL for binding of fluorescein, digoxigenin and a DTPA-based chelator (Korndorfer et al., Proteins 2003;53(1):121-129; Korndorfer et al., J Mol Biol. 2003;330(2):385-396; Kim et al., J Am Chem Soc. 2009;131(10):3565-3576). Hence, clinically attractive lipocalin-fold ligand "b" candidates can be selected regardless of initial binding affinity, just considering pharmacological properties as, e.g., tolerability, bioavailability, plasma levels, pharmacokinetics, tissue penetrance etc. Such molecules could be, for example, Indicaxanthin and Vulgaxanthin, which are pharmacologically well investigated and tolerated and are contained in sufficiently high amounts in a narrow segment of edible plants. There are, however, numerous examples of clinically approved drugs which have already been demonstrated to bind to certain lipocalin-fold molecules (e.g., human FABP1; Smathers et al., Hum Genomics. 2011;5(3):170-191; Velkov et al., Chem Biol. 2007;14(4):453-465; Velkov T., PPAR Res. 2013;2013:938401; Beringhelli et al., PLoS One. 2015;10(7):e0132096; Chuang at al., J Med Chem. 2008;51(13):3755-3764). Of course, such molecules are attractive starting points. The most attractive molecules are non-natural ligands, for which conformational effects have been proven and which have clinical application potential. For RBP4, for example, these ligands are A1120 and numerous derivatives as well as synthetic retinoids such as, e.g., fenretinide, N-Ethylretinamide, all-trans retinoic acid, retinyl acetate and axerophthene (Berni et al., FEBS Lett. 1992;308(1):43-45; Zanotti et al., J Biol Chem. 1993;268(33):24873-24879; Zanotti et al., J Biol Chem. 1994;269(47):29613-29620; Motani et al., J Biol Chem. 2009;284(12):7673-7680; Cioffi et al., J Med Chem. 2014;57(18):7731-7757; Cioffi et al., J Med Chem. 2015; 58(15):5863-5888).

Ad 2: High throughput screening of molecule libraries for molecules with binding affinity can be performed by various assays (reviewed in Auld et al., Receptor Binding Assays for HTS and Drug Discovery. In: Sittampalam et al., eds. Assay Guidance Manual. Bethesda MD: Eli Lilly & Company and the National Center for Advancing Translational Sciences; 2004). Screening of molecule libraries was, e.g., employed for identifying A1120 as a high affinity ligand for RBP4 (Motani et al., J Biol Chem. 2009;284(12):7673-7680). A consequence of this strategy is that the identified lipocalin-fold ligands display already some binding affinity and thus can be further assayed for mediating ligand-dependent binding or dissociation of a binder "c" without requirement of prior binding pocket engineering of the lipocalin-fold molecule. Of note, there are possibilities that enable high throughput screening for binding and function, like inducing conformational switching, in a single step. In the case of RBP4 this could be done, for example, by FRET based assays for detecting the conformation dependent interaction with TTR (Coward et al., Anal Biochem. 2009;384(2):312-320; Sharif et al., Anal Biochem. 2009;392(2):162-168; Dobri et al., Invest Ophthalmol Vis Sci. 2013;54(1):85-95)). If desired, the affinity of the respective lipocalin-fold ligand to the lipocalin-fold molecule can be further increased by engineering (i.e. mutagenesis) of the binding pocket of the lipocalin-fold molecule.

Ad 3: Virtual screening of compound databases is attractive when there are known small molecule ligands, which mediate conformational alterations in a lipocalin-fold molecule. Then a modeled pharmacophore of the small molecule can be used to screen for molecules, which might similarly interact with the lipocalin-fold molecule and thereby show similar function (see Qing et al., Journal of Receptor, Ligand and Channel Research 2014;7:81-92 for a review on virtual screening using pharmacophore modeling). In the optimal case, the crystal structure of the lipocalin-fold molecule charged with the modeled small molecule ligand is also known, since this helps to improve the accuracy of the prediction algorithms. There are several examples of known structures for lipocalin-fold molecules bound to a small molecule ligand (Berni et al., FEBS Lett. 1992;308(1):43-45; Zanotti et al., J Biol Chem. 1993;268(33):24873-24879; Zanotti et al., J Biol Chem. 1994;269(47):29613-29620; Pattanayek et al., Protein Sci. 1999;8(10):2027-2032; Motani et al., J Biol Chem. 2009;284(12):7673-7680; Gasymov et al., Biochim Biophys Acta. 1998;1386(1):145-156; Breustedt et al., Acta Crystallogr D Biol Crystallogr. 2009;65(Pt 10):1118-1125; Gasymov et al., Biochemistry. 2012;51(14):2991-3002; Zhang et al., Sci Rep.

2016;6:30655; Christoffersen et al., Proc Natl Acad Sci USA. 2011;108(23):9613-9618; Coward et al., Anal Biochem. 2009;384(2):312-320). This strategy is exemplified in the example section below with RBP4 charged with A1120, for which a pharmacophore model was generated in order to identify and prioritize molecules for functional testing and for engineering of the binding pocket of RBP4 (described in Example 2, below). Importantly, the pharmacophore approach does not deliver a final complete list of potentially conformation inducing molecules due to limitations of pharmacophore methods (reviewed in Qing et al., Journal of Receptor, Ligand and Channel Research 2014;7:81-92) and due to incomplete and not updated databases etc. Moreover, there are other molecules also known to alter the conformation of RBP4, e.g., fenretinide, for which a pharmacophore based approach would lead to identification of yet another set of molecules in the chemical space. In fact, *in silico* filtering of millions of molecules for function together with directed evolution of the highly flexible binding pocket of the lipocalin-fold structure in the lipocalin-fold molecules according to the present invention is a perfect combination and the crucial strength of the novel LRPPI system. Of note, virtual screening also enables identifying functional molecules, which do not have sufficient initial binding affinity, which is not a problem, since affinity can easily be generated by engineering of the lipocalin-fold molecule. In this case, lipocalin-fold molecule mutants are selected by use of a conformation specific binding protein that binds to the lipocalin-fold molecule mutatants in the absence but not in the presence of a known conformation inducing ligand. Thus, the dissociation of this conformation specific binding protein enables the identification of lipocalin-fold ligand candidates that bind to the lipocalin-fold molecule and that at the same time induce conformational alterations (detailed in Example 2, below).

Principally, the lipocalin-fold binding interaction partner "c" can be provided or engineered based on any available molecular binder scaffold including antibodies, antibody fragments [e.g. single-chain variable fragments (scFv), antigen binding fragments (Fabs), nanobodies, among others] and non-antibody based scaffolds such as affibodies, a further (or the same) lipocalin-fold molecule, preferably LCNs, especially anticalins; avimers, DARPins, fynomers, Kunitz domains, knottins, monobodies, binders based on Sso7d, reduced charge Sso7d (rcSso7d) or Sac7d, among many others (Simeon et al., Protein Cell. 2017; Gilbreth et al., Curr Opin Struct Biol. 2012;22(4):413-420; Koide et al., ACS Chem Biol. 2009;4(5):325-334; Traxlmayr et al., J Biol Chem. 2016;291(43):22496-22508). Meanwhile, many more non-antibody binding proteins have been reported (Pluckthun, Alternative Scaffolds: Expanding the options of antibodies. In: Little M, ed. New York: Cambridge University Press; 2009:244-271; Chapman et al., Cell Chem Biol. 2016;23(5):543-553; Binz et al., Nat Biotechnol. 2005;23(10):1257-1268; Vazquez-Lombardi et al., Drug Discov Today. 2015;20(10):1271-1283), and, in fact, synthetic library design and selection can be applied to any protein which then can potentially serve as lipocalin-fold binding interaction partner "c", too (Pluckthun, Alternative Scaffolds: Expanding the options of antibodies. In: Little M, ed. New York: Cambridge University Press; 2009:244-271). With the aim of clinical applicability of the LRPPI systems according to the present invention, lipocalin-fold binding interaction partners "c" are preferably derived from small human single protein domains (e.g., fibronectin type III domain (FN3) based Monobodies) with a minimized number of mutated amino acids for keeping immunogenicity as low as possible. It is of course also possible to attach such further molecules and scaffolds to the lipocalin-fold molecule (or to a lipocalin-fold binding interaction partner).

Accordingly, the lipocalin-fold molecule according to the present invention may be any protein that contains the structural motif of a lipocalin-fold to which (or in which) the lipocalin-fold ligand binds and which enables binding of the lipocalin-fold molecule to the lipocalin-fold binding interaction partner. Usually, the lipocalin-fold molecule according to the present invention is adapted to the specific needs for the lipocalin-fold molecule "a"/lipocalin-fold ligand "b"/lipocalin-fold binding interaction partner "c" triangle by providing a "starting" lipocalin-fold molecule which may already have a certain affinity to a given lipocalin-fold ligand and/or for which clinically attractive ligands with functional activity could be identified by virtual screening. This starting lipocalin-fold molecule is then engineered by one or more amino acid exchanges, insertions and/or deletions to optimize lipocalin-fold ligand binding, and in the case of strategy B of the lipocalin-fold based LRPPI system also for binding to a lipocalin-fold binding interaction partner "c". In strategy A the lipocalin-fold binding interaction partner "c" is generated by engineering of a protein (that is originally not able to bind to any naturally occurring lipocalin-fold molecule with an affinity of < 10 $\mu$M in the presence of any lipocalin-fold ligand) for binding to the (starting and/or engineered) lipocalin-fold molecule (bound to the lipocalin-fold ligand). Such sequence optimization of a protein for generating a lipocalin-fold ligand dependent lipocalin-fold binding interaction partner is shown in the example section of the present invention and well available for a person skilled in the art with the disclosure contained herein. In fact, it is established in the art of protein engineering that protein scaffolds (especially well-established scaffolds, such as antibodies and non-antibody-based scaffolds, especially lipocalins) can be adapted so that virtually any biological molecules (especially proteins) can be bound (see e.g. (for non-antibody scaffolds) Vasquez-Lombardi et al., Drug Discov. Today 20 (2015), 1271-1283; Pluckthun, Alternative Scaffolds: Expanding the Options of Antibodies. In: Recombinant Antibodies for Immunotherapy, Melvyn Little, Cambridge University Press, New York (2009), pp. 244-271) .

The (starting) lipocalin-fold molecule therefore preferably contains the structural lipocalin-fold of a naturally occurring protein of the lipocalin-fold superfamily (for clinical applications in human patients: preferably a naturally occurring human lipocalin-fold molecule) or a known variant thereof (e.g. an "anticalin" or "mutein of lipocalin" and the like; a vast number of such variants have already been disclosed in the prior art). The lipocalin-fold molecule to be used in the LRPPI system according to the present invention is then adapted to the specificities of the lipocalin-fold ligand/lipocalin-fold binding interaction partner by the introduction of modifications, as disclosed above (e.g. a) to e)), by engineering (i.e. amino acid changes, insertions and/or deletions) of the binding pocket, amino acid positions in the β-barrel structure, and/or amino acid positions in the regions adjoining the β-strands of the β-barrel structure, mutation/deletion/insertion of residues for preventing dimerization or interaction with other molecules or for preventing post-translational protein modification and/or engineering of the lipocalin-fold for improved stability.

As already stated above, the lipocalin-fold architecture is very robust and has high sequence flexibility, as demonstrated by the fact that it has not been possible to define any sequence motif that is common to all naturally occurring members of the lipocalin superfamily, including LCNs and iLBPs (Lakshmi et al. PLoS One. 2015;10(8): e0135507; Flower et al., Biochim Biophys Acta. 2000;1482(1-2):9-24). Apart from the generally high sequence variation in naturally occurring members of the lipocalin superfamily, the regions adjoining the structurally conserved β-strands not only show high variation in sequence, but also in structure (Flower et al., Biochim Biophys Acta. 2000;1482(1-2):9-24; Schiefner et al., Acc Chem Res. 2015;48(4):976-985). Given the extensive sequence variation among naturally occurring lipocalin superfamily members and/or structural variation in the regions adjoining the β-strands, it is not surprising that in the past lipocalin molecules have been successfully engineered by mutating more than 30 amino acid positions, corresponding to more than 15% of all amino acid positions in the respective protein, without any major structural changes in the structurally conserved β-strands of the characteristic β-barrel structure (Schonfeld et al., Proc Natl Acad Sci USA. 2009;106(20):8198-8203). Thus, it is well-established in the field that lipocalin-fold molecules can be extensively mutated without impairing the overall β-barrel fold (i.e. particularly the loop regions but also the β-strands of the β-barrel can be designed by exchanging, inserting and/or deleting a considerable number of amino acids). Therefore, in the present invention, which describes the usage of the lipocalin-fold structure in LRPPI systems, a lipocalin-fold molecule is defined as any naturally occurring molecule classified into the lipocalin superfamily in the SCOP database (version 1.75), or a mutant thereof. However, it is preferred to exchange only a limited number of amino acids. The LRPPI system according to the present invention therefore preferably comprises (1) a lipocalin-fold molecule being identical to a naturally occurring member of the lipocalin superfamily or (2) an already existing variant thereof (e.g. an already existing anticalin, lipocalin mutein, iLBP with complete deletion of the helix-turn-helix motif at the barrel entrance etc.) or (3) a derivative of (1) or (2) with at least 70%, preferably at least 80%, especially at least 90% sequence identity to its counterpart that it derives from.

According to a preferred embodiment, the lipocalin-fold molecule is a derivative of a naturally occurring or otherwise disclosed (by its amino acid sequence) lipocalin-fold molecule with at least 70%, preferably at least 80%, especially at least 90% sequence identity in the β-barrel structure, whereby this β-barrel structure is defined as the regions which correspond structurally to amino acid positions 21-30, 41-47, 52-58, 71-78, 85-88, 102-109, 114-120 and 132-138 in human RBP4 (according to the amino acid residue numbering scheme in the PDB entry 1RBP); or to the amino acid positions 14-23, 37-43, 48-54, 62-69, 76-79, 84-91, 96-102 and 111-117 in human tear lipocalin (TLC; as defined by Schiefner et al., Acc Chem Res. 2015;48(4):976-985); or to the amino acid positions 44-53, 69-75, 81-87, 96-103, 110-113, 119-126, 131-137 and 142-148 in human apolipoprotein M (Ap-oM; as defined by Schiefner et al., Acc Chem Res. 2015; 48 (4) :976-985); or to the amino acid positions 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6); or to the amino acid positions 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73). Preferred embodiments of the lipocalin-fold molecule according to the present invention also include lipocalin-fold molecules which comprise 1-30 amino acid exchanges or fragments of (1), (2) or (3) which (at least) contain the lipocalin-fold and are able to bind to the lipocalin-fold ligand and the lipocalin-fold binding interaction partner. Accordingly, the ligand regulated protein-protein interaction system preferably comprises as lipocalin-fold molecule a molecule identical with a naturally occurring iLBP (intracellular lipid binding protein), a naturally occurring lipocalin or an anticalin, and derivatives of any of these molecules with 1-30 amino acid exchanges and fragments thereof.

Alternatively, the LRPPI system according to the present invention may also comprise a (4) fragment of (1) or (2) or (3) with a length of at least 80, preferably at least 100, especially at least 120, amino acids in the case of LCNs [or with a length of of at least 80, preferably at least 85, especially at least 90, amino acids in the case of iLBPs (which are generally smaller proteins than LCNs)] covering at least the structurally conserved β-barrel structure of the lipocalin-fold. This structurally conserved β-barrel structure comprises or consists of amino acid positions which correspond structurally to the amino acid positions 21-30, 41-47, 52-58, 71-78, 85-88, 102-109, 114-120 and 132-138 in human RBP4 (according to the amino acid residue numbering scheme in the PDB entry 1RBP); or to the amino acid positions 14-23, 37-43, 48-54, 62-69, 76-79, 84-91, 96-102 and 111-117 in human tear lipocalin (TLC; as defined by Schiefner et al., Acc Chem Res. 2015;48(4):976-985); or to the amino acid positions 44-53, 69-75, 81-87, 96-103, 110-113, 119-126, 131-137 and 142-148 in human apolipoprotein M (ApoM; as defined by Schiefner et al., Acc Chem Res. 2015; 48(4): 976-985) ; or to the amino acid positions 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6); or to the amino acid positions 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73).

Further preferred lipocalin-fold molecules in the LRPPI system according to the present invention are derivatives of a naturally occurring lipocalin or iLBP with up to 15, up to 30, or up to 50 amino acid deletions and/or up to 15, up to 30, or up to 50 amino acid insertions outside of the structurally conserved β-barrel structure. Outside the structurally conserved β-barrel structure these molecules are very flexible to amino acid changes and therefore combinations of mutations, deletions and insertions are possible in these regions which correspond structurally to amino acid residues 1-20, 31-40, 48-51, 59-70, 79-84, 89-101, 110-113, 121-131 and 139-183 in human RBP4 (according to the amino acid residue numbering scheme in the PDB entry 1RBP), which define the regions adjoining the structurally conserved β-strands in human RBP4; or corresponding structurally to the regions of amino acid residues 1-13, 24-36, 44-47, 55-61, 70-75, 80-83, 92-95, 103-110 and 118-158 in human TLC (according to the amino acid resi-due numbering scheme in Schiefner et al., Acc Chem Res. 2015;48(4):976-985), which define the regions adjoining the structurally conserved β-strands in human TLC; or corresponding structurally to the regions of amino acid residues 1-43, 54-68, 76-80, 88-95, 104-109, 114-118, 127-130, 138-141 and 149-188 in human ApoM (according to the amino acid residue numbering scheme in Schiefner et al., Acc Chem Res. 2015;48(4):976-985), which define the regions adjoining the structurally conserved β-strands in human ApoM; or corresponding structurally to the regions of amino acid residues 1-4, 13-40, 46-49, 55-60, 66-70, 74-80, 88-92, 97-107, 113-118, 125-128 and 136-137 in human CRABPII (according to the amino acid residue numbering scheme in PDB entry 2FS6), which define the regions adjoining the structurally conserved β-strands in human CRABPII; or corresponding structurally to the regions of amino acid residues 1-4, 13-38, 44-47, 53-58, 64-68, 72-78, 86-90, 95-98, 104-108, 115-118 and 126-127 in human FABP1 (according to the amino acid residue numbering scheme in PDB entry 2F73), which define the regions adjoining the structurally conserved β-strands in human FABP1.

In a preferred embodiment, the LRPPI system according to the present invention comprises as lipocalin-fold molecule a derivative of a naturally occurring member of the lipocalin superfamily with at least one, two, three, four, five, six, seven, eight, nine, ten, eleven, twelve, 13, 14, 15, 16, 17, 18, 19, 20, 21, 22, 23, 24, 25, 26, 27, 28, 29, or 30 amino acid exchanges.

According to a further preferred embodiment, the lipocalin-fold molecule used in the LRPPI system according to the present invention is a lipocalin, i.e., a protein containing an eight-stranded up-and-down β-barrel arranged in a +1 topology, followed by an α-helix after the C-terminal end of the eighth β-strand.

In designing the lipocalin-fold molecule according to the present invention, one parameter which may be significantly improved in the derivatization/mutation process from a starting lipocalin-fold molecule to a molecule actually optimized for a given LRPPI system, is affinity to the lipocalin-fold ligand "b". Additionally or alternatively, in the case in which the lipocalin-fold molecule itself is engineered as a binder instead of the lipocalin-fold binding interaction partner, the parameter to improve is increasing the affinity difference between the two statuses of the lipocalin-fold molecule (bound or not bound to the lipocalin-fold ligand) for binding to the lipocalin-fold binding interaction partner "c". Selection of lipocalin-fold molecule/lipocalin-fold ligand pairs that are preferentially characterized by structural differences between the bound and unbound statuses (preferably resulting from conformational alterations) is the basis for maximum affinity differences of the two lipocalin-fold molecule statuses for a lipocalin-fold binding interaction partner "c". In the case, in which the lipocalin-fold molecule is used as an antigen (i.e. strategy A of the lipocalin-fold based LRPPI system), the lipocalin-fold binding interaction partner "c" can be subsequently engineered for maximum selectivity between the two statuses of the lipocalin-fold molecule, i.e., maximum affinity difference. The engineering of the lipocalin-fold binding interaction partner and/or the lipocalin-fold molecule leads to LRPPI systems wherein the affinity of the lipocalin-fold binding interaction partner to the lipocalin-fold molecule in the lipocalin-fold ligand-bound state is at least 10-fold higher, preferably at least 20-fold higher, especially at least 50-fold higher than the affinity of the lipocalin-fold binding interaction partner to the lipocalin-fold molecule in the unbound state (i.e. in the absence of the lipocalin-fold ligand). In the course of the present invention, the human lipocalin RBP4, which is known to undergo conformational alteration upon binding of some ligands, has been investigated in more detail to prove the concept of the present invention in principle. Similarly to RBP4, the human lipocalins TLC and ApoM are also known to undergo significant ligand induced conformational alterations and it is thus preferred to use one of these three lipocalins as lipocalin-fold molecule or as starting lipocalin-fold molecule for the generation of a LRPPI system according to the present invention.

A preferred embodiment of the LRPPI system according to the present invention therefore applies a lipocalin-fold molecule that has a sequence identity with the native versions of human RBP4, TLC or ApoM of at least 70%, preferably at least 80%, especially at least 90%.

According to a preferred embodiment, the lipocalin-fold molecule according to the present invention has a sequence identity with human RBP4 of at least 70%, preferably of at least 80%, especially of at least 90% in the structurally conserved β-barrel structure including the regions of amino acid residues 21-30, 41-47, 52-58, 71-78, 85-88, 102-109, 114-120 and 132-138 according to the amino acid residue numbering scheme in the PDB entry 1RBP, or a fragment thereof with at least 80, preferably at least 100, especially at least 120 amino acid residues and comprising the regions corresponding to amino acid residues 21-30, 41-47, 52-58, 71-78, 85-88, 102-109, 114-120 and 132-138 of human RBP4 (according to the amino acid residue numbering scheme in the PDB entry 1RBP); or (2) the lipocalin-fold molecule has a sequence identity with human tear lipocalin (TLC) of at least 70%, preferably of at least 80%, especially of at least 90% in the structurally conserved β-barrel structure including the regions of amino acid residues 14-23, 37-43, 48-54, 62-69, 76-79, 84-91, 96-102 and 111-117 in human TLC as defined by Schiefner et al., Acc Chem Res. 2015; 48(4):976-985, or a fragment thereof with at least 80, preferably at least 100, especially at least 120 amino acid residues and comprising the regions corresponding to amino acid residues 14-23, 37-43, 48-54, 62-69, 76-79, 84-91, 96-102 and 111-117 of human TLC; or (3) the lipocalin-fold molecule has a sequence identity with human apolipoprotein M (ApoM) of at least 70%, preferably of at least 80%, especially of at least 90% in the structurally conserved β-barrel structure including the regions of amino acid residues 44-53, 69-75, 81-87, 96-103, 110-113, 119-126, 131-137 and 142-148 in human ApoM as defined by Schiefner et al., Acc Chem Res. 2015;48(4):976-985, or a fragment thereof with at least 80, preferably at least 100, especially at least 120 amino acid residues and comprising the regions corresponding to amino acid residues 44-53, 69-75, 81-87, 96-103, 110-113, 119-126, 131-137 and 142-148 of human ApoM.

In a preferred embodiment, the lipocalin-fold molecule of the LRPPI system according to the present invention has a sequence identity with human RBP4 of at least 95%, or a sequence identity with human tear lipocalin (TLC) of at least 95%, or a sequence identity with human apolipoprotein M (ApoM) of at least 95%.

The LRPPI system according to the present invention consists of the central lipocalin-fold molecule "a"/lipocalin-fold ligand "b"/lipocalin-fold binding interaction partner "c" triangle (see Fig. 1A). However, this triangle can be further functionalized by linking further moieties to the lipocalin-fold molecule "a" and/or the lipocalin-fold binding interaction partner "c" (see e.g. Fig. 1B). The LRPPI triangle according to the present invention can therefore be expanded into various architectures, e.g. as CARs (as disclosed below). Again, it may be emphasised that the "triangles" according to the present invention differ from any naturally occurring (physiological) triangle, because the lipocalin-fold binding interaction partner (or any domain of it that mediates binding to the lipocalin-fold molecule) is not a naturally occurring lipocalin-fold binding interaction partner (i.e. a protein which has an affinity of <10 μM to any naturally occurring lipocalin-fold molecule in the presence of any lipocalin-fold ligand). Preferably, the lipocalin-fold binding interaction partner is also not derived from a naturally occurring lipocalin-fold binding interaction partner. "Being derived from" is defined as containing at least one segment of at least 50 consecutive amino acids with an amino acid sequence that is identical with the amino acid sequence of any segment of that naturally occurring protein which has an affinity of <10 μM to any naturally occurring lipocalin-fold molecule (in the presence of any lipocalin-fold ligand). Preferably both the lipocalin-fold molecule and the lipocalin-fold binding interaction partner are not naturally occurring molecules but artificially designed molecules (e.g. designed by recombinant technology and/or directed evolution). In a preferred embodiment, the lipocalin-fold binding interaction partner is or comprises the lipocalin-fold molecule and/or the lipocalin-fold binding interaction partner comprises an antigen, a cell surface receptor, an antibody, etc.

For the LRPPI system according to the present invention, it is also preferred that the affinity of the lipocalin-fold ligand to the lipocalin-fold molecule is in a range that allows binding of the lipocalin-fold ligand to the lipocalin-fold molecule at lipocalin-fold ligand concentrations that can be achieved in the physiological environment in the human body. Accordingly, it is preferred that the lipocalin-fold ligand has an affinity to the lipocalin-fold molecule of below 1 mM, preferably of below 100 μM, especially of below 10 μM.

The LRPPI system according to the present invention generally relies on a substantial difference in the affinities of the lipocalin-fold molecule "a" to the lipocalin-fold binding interaction partner "c" depending on whether the lipocalin-fold ligand "b" is bound or not. It is also preferred that this affinity window (i.e. the affinities of the lipocalin-fold binding interaction partner to the lipocalin-fold molecule bound or not bound to the lipocalin-fold ligand, respectively) is present in a reasonable affinity range which allows for regulation of this LRPPI system under physiological conditions. Therefore, it is preferred that the affinity of the lipocalin-fold binding interaction partner to the lipocalin-fold molecule in the ligand-bound state is below 10 μM, preferably below 2 μM, especially below 400 nM.

For the LRPPI system according to the present invention, the affinity difference of the lipocalin-fold molecule binding to the lipocalin-fold binding interaction partner between the states bound/unbound to the lipocalin-fold ligand should be as high as possible. This can be achieved by directed evolution of the interaction partner and screening for suitable affinity differences (for increased affinity differences). For being applicable in an *in vivo* environment, the lipocalin-fold molecule has to have a sufficiently high affinity to the lipocalin-fold ligand. This enables functioning of the LRPPI system according to the present invention also in a patient *in vivo.* Here, it has also to be considered that drug concentrations in plasma (e.g. for the lipocalin-fold ligand) is usually a few μM. Accordingly, the affinity of the lipocalin-fold ligand to the lipocalin-fold molecule has to be sufficiently high that such plasma concentrations can achieve appropriate binding of the ligand to the lipocalin-fold molecule. Moreover, the LRPPI system according to the present invention should be designed to minimize relevant binding of other substances to the lipocalin-fold molecules (which could elicit adverse reactions or hinder effectiveness of *in vivo* functioning of the system (e.g. by competing with binding to the lipocalin-fold molecule)).

The lipocalin-fold based LRPPI system enables the highest flexibility with respect to choosing the lipocalin-fold ligand used for controlling PPI. Whereas certain embodiments of the present invention work with substances already known to have a certain affinity to (certain) lipocalin-fold molecule(s), also the lipocalin-fold molecule may be designed to bind a small molecule of interest, thereby enabling the small molecule to become a lipocalin-fold ligand by such engineering of a lipocalin-fold molecule. Accordingly, any interesting small molecule, interesting for pharmaceutical purposes and thus preferred to be used as a lipocalin-fold ligand "b" according to the present invention (i.e. a substance enabling the binding of the lipocalin-fold molecule to the lipocalin-fold binding interaction partner) can be included in a LRPPI system according to the present invention. According to a preferred embodiment of the present invention, the lipocalin-fold ligand is fenretinide (PubChem CID: 5288209), N-Ethylretinamide (PubChem CID: 5288173), all-trans retinoic acid (PubChem CID: 444795), axerophthene (PubChem CID: 5287722), A1120 (PubChem CID 25138295), derivatives of A1120 (Cioffi et al., J Med Chem. 2014;57(18):7731-7757; Cioffi et al., J Med Chem. 2015;58(15):5863-5888)), 1,4-butanediol, sphingosine-1-phosphate, tetradecanoic acid, indicaxanthin, vulgaxanthin, Montelukast, Cyclandelate, Oxolamine, Mazaticol, Butoctamid, Tonabersat, Novazin, Diphenidol, Neobornyval, Alloclamide, Diacetolol, Clindamycin, Proxazole, Tropesin, Triclabendazole, Triclabendazole sulfoxide, Triclabendazole sulfone, Trametinib, etc.. Preferred substances are also provided in Table 1, below.

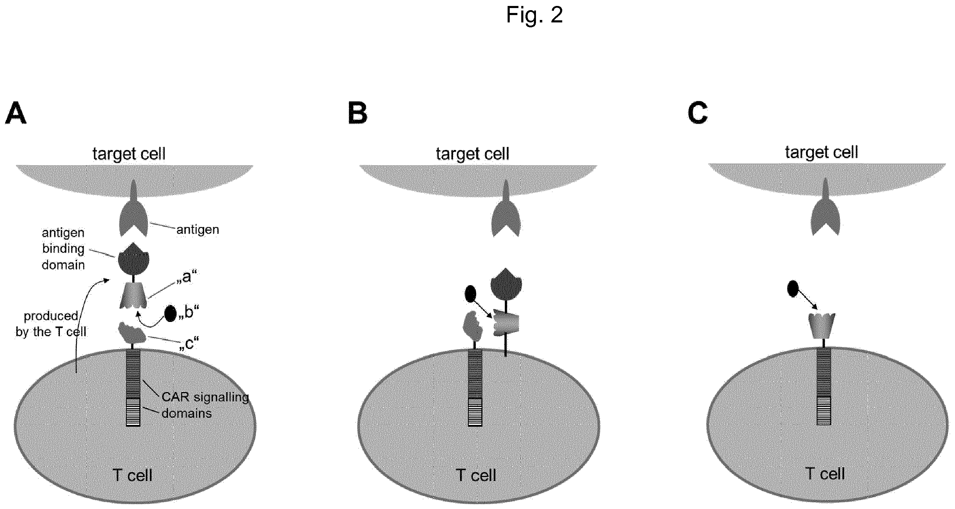

An unmet need in the field of cellular immunotherapy is the reversible control of CAR function by clinically applicable small molecules. Thus, in a preferred application the LRPPI system of the present invention is integrated into a CAR which is expressed in T cells or other effector cells such as, e.g., NK cells. The preferred version of the LRPPI system for controlling CAR function is where the lipocalin-fold ligand ("b")-loaded lipocalin-fold molecule "a" is used as antigen for a binder "c" (see schematic representation in Fig. 2A and B). In this version, the CAR construct that mediates binding to the target cell is separated from the CAR construct that mediates signal transduction, whereby the CAR construct that mediates binding to the target cell can be secreted by the effector cell or can be administered/added exogeneously as soluble protein (LRPPI-CAR strategy A) or can be membrane-anchored (LRPPI-CAR strategy B). Addition of lipocalin-fold ligand "b" induces interaction of the two constructs (containing the lipocalin-fold molecule "a" and the lipocalin-fold binding interaction partner "c"). The components "a" and "c" of the LRPPI system can be extracellularly (preferred) or intracellularly integrated (with or without linker sequences) into the two CAR constructs. Shown in Fig. 2A and 2B is the fusion of the lipocalin-fold molecule "a" to the CAR construct that mediates target cell binding and fusion of the lipocalin-fold binding interaction partner "c" to the signal transducing construct, which, however, could also be reverse. Binding to the target cell can be mediated by any protein capable of binding to a chosen antigen on the target cell surface. In an alternative version (LRPPI-CAR strategy C) the lipocalin-fold molecule itself can directly bind to the antigen, whereby the antigen then is part of the LRPPI system and acts as lipocalin-fold binding interaction partner "c". The lipocalin-fold molecule "a" in this case is engineered by *in vitro* directed evolution using protein engineering technologies and subsequent selection for lipocalin-fold ligand-dependent binding to the lipocalin-fold binding interaction partner "c", which in this case is an antigen of a target cell.

Examples of CAR architectures are well-known in the art (e.g. reviewed by Abate-Daga et al., Mol Ther Oncolytics. 2016;3:16014, and see e.g. WO 2014/127261 A1 and WO 2015017214 A1). In an embodiment, the signal transducing construct usually comprises one or two of the costimulatory signaling domains of the receptors CD27, CD28, CD134, CD137, ICOS, DAP12, activating NK cell receptors etc. (in any order from N- to C-terminus), with or without a domain for transmitting signal 1 (e.g. CD3zeta), or alternatively comprises the inhibitory cytoplasmic domains of the inhibitory receptors PD1, CTLA4, LAG3, TIM3, inhibitory NK cell receptors etc. Transmembrane domains and extracellular membrane anchors can be derived from these receptors or from other proteins such as, e.g., CD3zeta, CD8alpha, CD28 etc.

In LRPPI-CAR strategy B (Figure 2B), the signal transducing domain (s) do not need to be necessarily confined to one of the two constructs, but instead could be separated from each other by fusing them to the two different CAR constructs separately (not shown). Further diversity arises due to the fact that both CAR constructs, the antigen binding and/or the signal transducing chain, can contain spacer domains comprised of fragments of the signal transducing proteins or of, e.g., IgG-Fc domains. Moreover, the antigen binding domains can be based on single-chain Fv fragments, endogenous receptor- or ligand domains (e.g., NKG2D, IL13 etc.) or any other available binder scaffold (Simeon et al., Protein Cell. 2017; DOI 10.1007/s13238-017-0386-). If the lipocalin-fold molecule itself is the binding domain engineered for binding to a chosen target antigen (LRPPI-CAR strategy C, Figure 2C), the antigen on the target cell itself is part of the LRPPI system, i.e., representing the lipocalin-fold binding interaction partner "c". Principally, the binding domains of the CAR can be directed to any antigen, including non-protein antigens.

Accordingly, a preferred embodiment of the LRPPI system according to the present invention is a system, wherein the lipocalin-fold molecule is part of an ectodomain of a chimeric antigen receptor and wherein the lipocalin-fold binding interaction partner is a cell surface antigen.

The present invention is further described by the following examples and the figures, yet without being limited thereto.

Fig. 1 shows the schematics of the present invention as an LRPPI system based on a lipocalin-fold (A) and a preferred embodiment thereof (B). (A) lipocalin-fold molecule ("a") can accommodate in its calyx a small molecule lipocalin-fold ligand ("b") and can bind to a lipocalin-fold binding interaction partner "c" with higher affinity in the presence of "b"; (B) the lipocalin-fold molecule ("a") may be fused (+/- flexible linker) to terminal or internal sites of a protein "I" and also accommodates in its calyx a small molecule lipocalin-fold ligand ("b") and can bind to a lipocalin-fold binding interaction partner "c", which may be preferably a protein (that may be fused (+/flexible linker) to terminal or internal sites of a further protein), with higher affinity in the presence of "b".

Fig. 2 shows a schematic example of lipocalin-fold molecule-based LRPPI systems according to the present invention integrated into CARs. "a" can be part of a soluble protein (secreted or exogenously added) or of a transmembrane construct and "c" can be part of a signal transducing construct (shown in A and B). Of course, "a" and "c" can be integrated in the constructs vice versa (not shown). There are many more possible arrangements (not shown). For example, the signal transducing domains do not need to be necessarily confined to one of the two constructs, and "a" and "c" can alternatively be part of the cytoplasmic domains of the constructs. (C) shows an example in which "c" is an antigen.

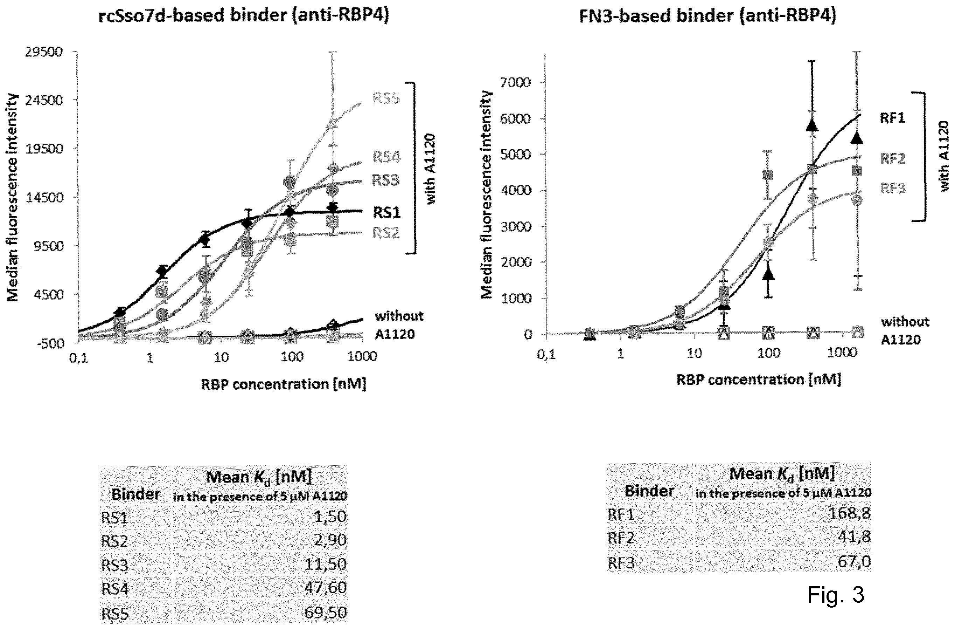

Fig. 3 shows A1120 dependent binding of increasing amounts of human RBP4 to differrent rcSso7d- and FN3-based lipocalin-fold binding interaction partners displayed on the surface of yeast. The assay was performed in the presence (5 μM) or absence of the lipocalin-fold ligand A1120. RBP4-binding was detected with an anti-penta-His antibody and subsequently analyzed by flow cytometry.

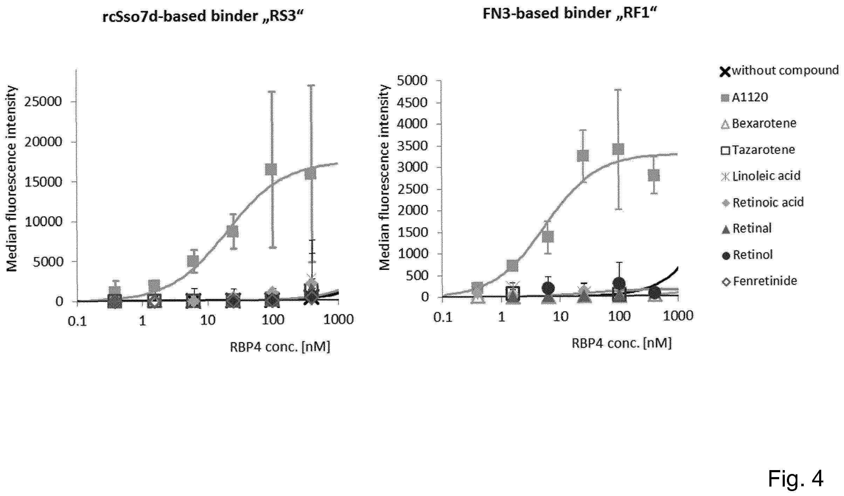

Fig. 4 shows binding of rcSso7d- and FN3-based lipocalin-fold binding interaction partners (displayed on the surface of yeast) to RBP4 loaded with different small molecules. All small molecules were present at a concentration of 5 μM. RBP4-binding was detected with an anti-penta-His antibody and subsequently analyzed by flow cytometry.

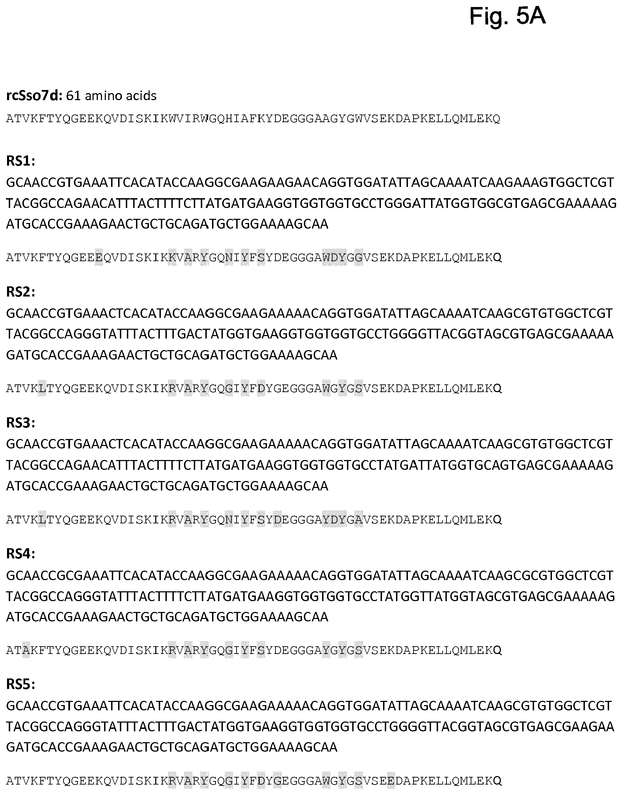

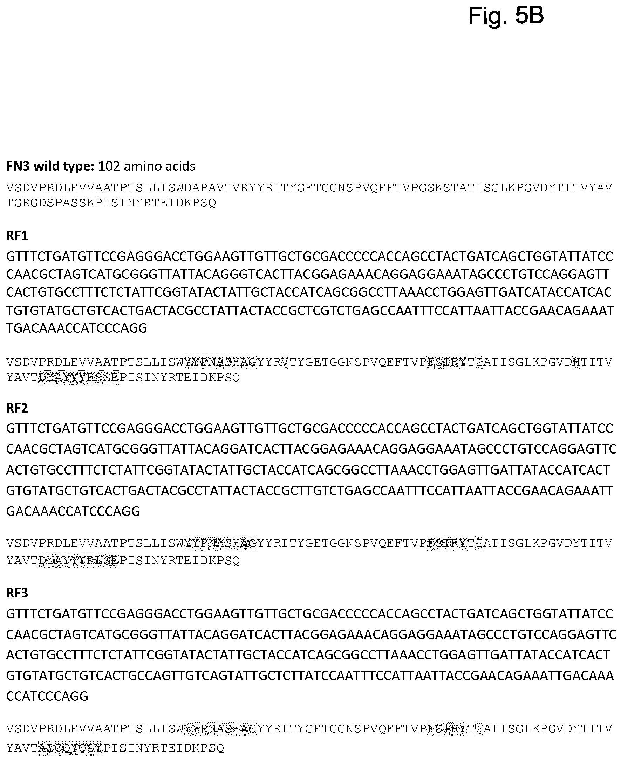

Figs. 5A and 5B show sequences of rcSso7d- and FN3-based lipocalin-fold binding interaction partners. The amino acids differing from the original rcSso7d or FN3 sequence are highlighted in blue.

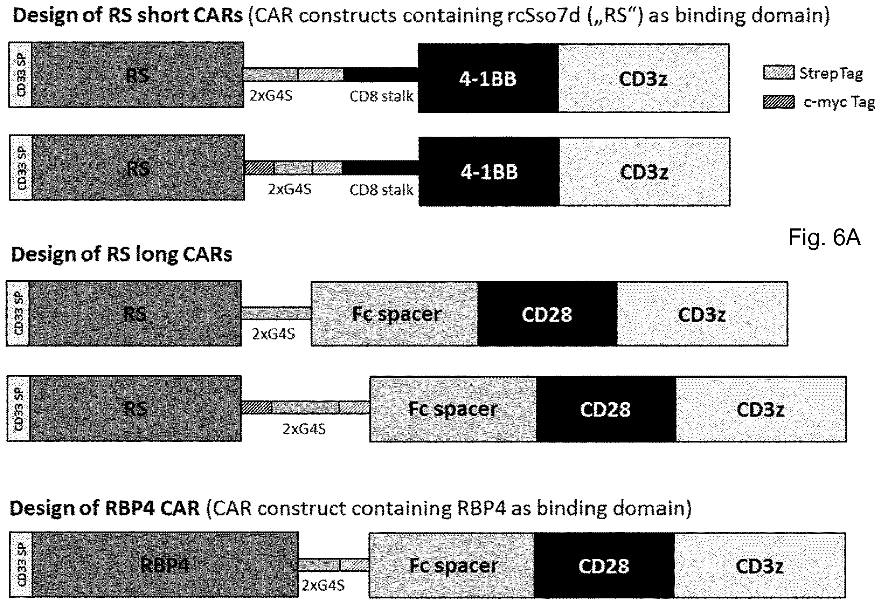

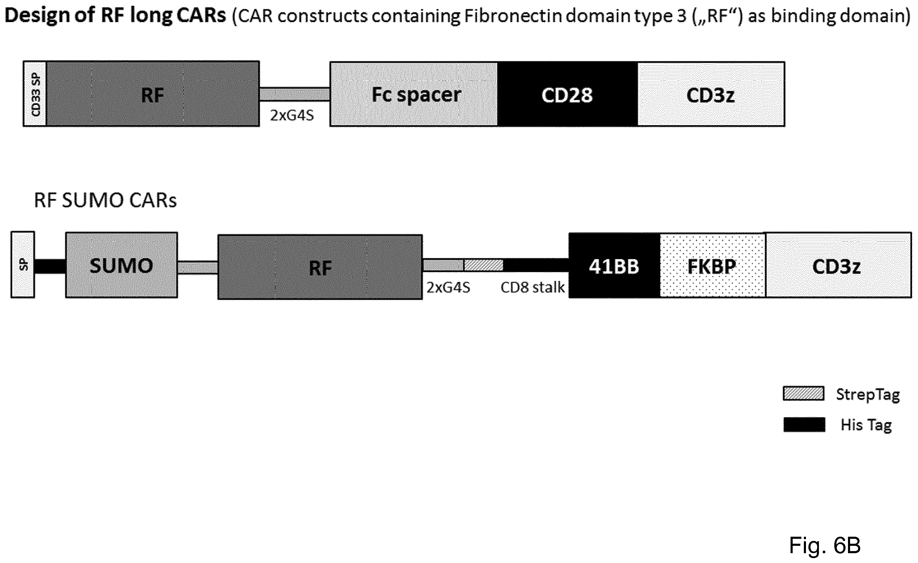

Figs. 6A and 6B show schematics of the signal transducing constructs, i.e. the part of the CAR, in which either the lipocalin-fold molecule or the lipocalin-fold binding interaction partner is fused to the signaling domains.

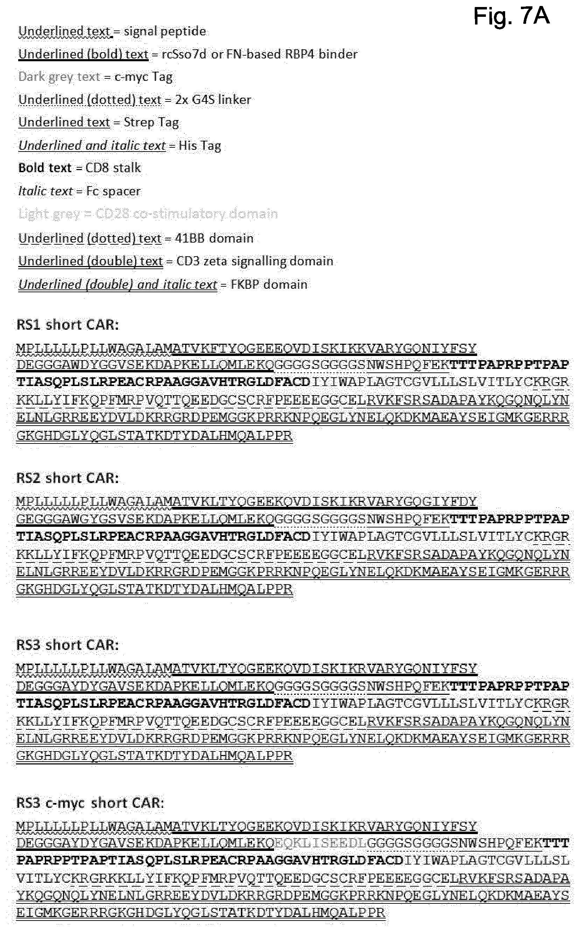

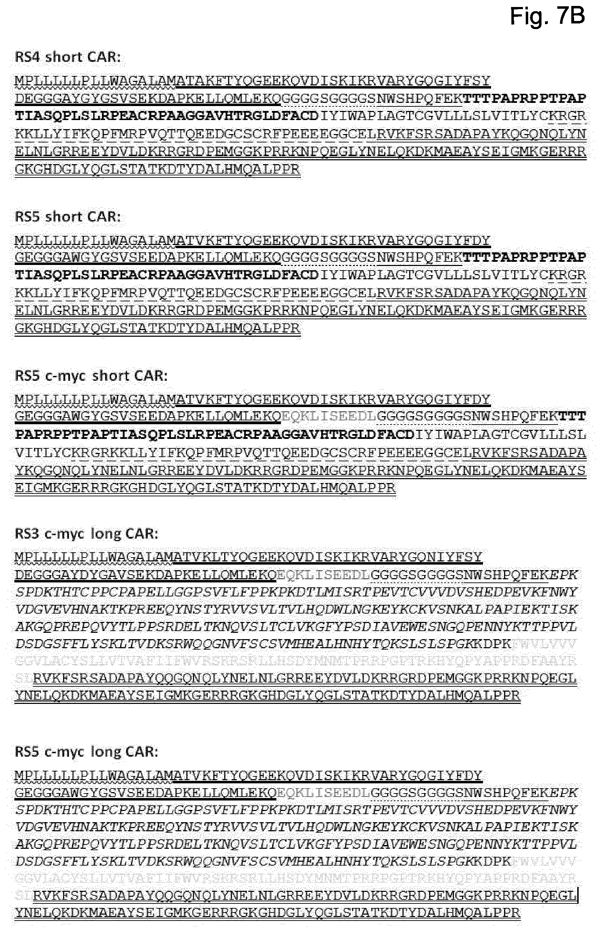

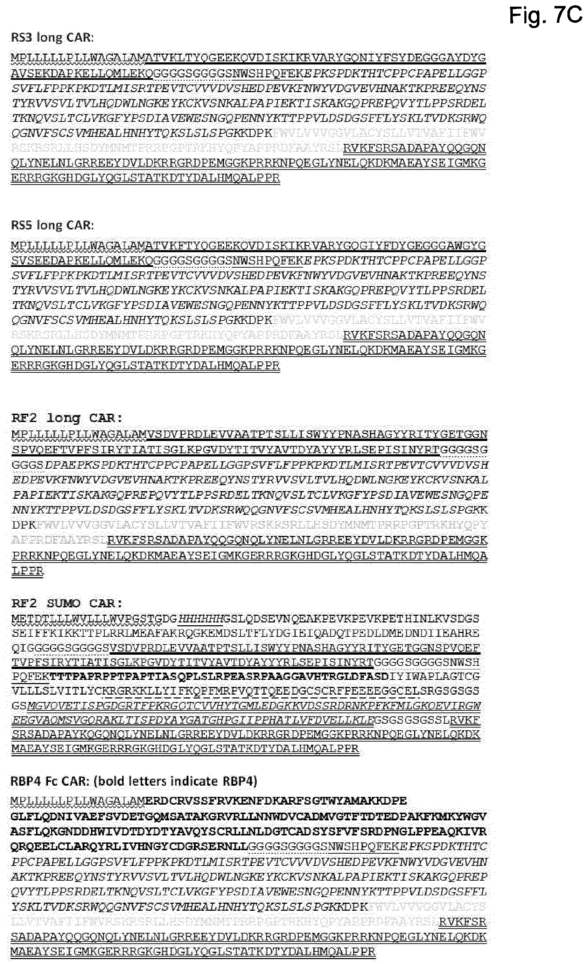

Figs. 7A, 7B and 7C show sequences of the signal transducing CAR constructs containing rcSso7d- and FN3-based RBP4 binding interaction partners.

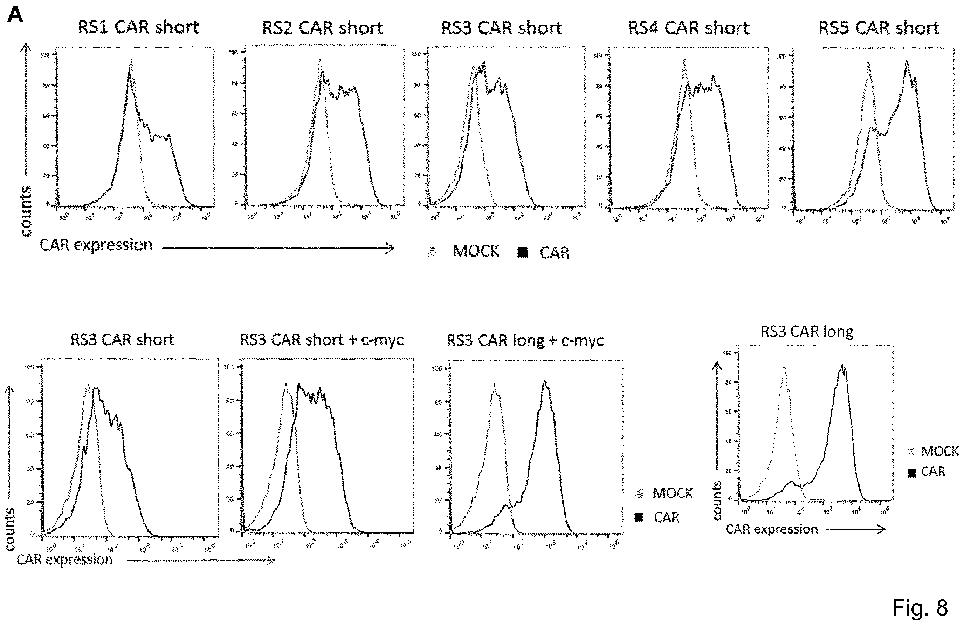

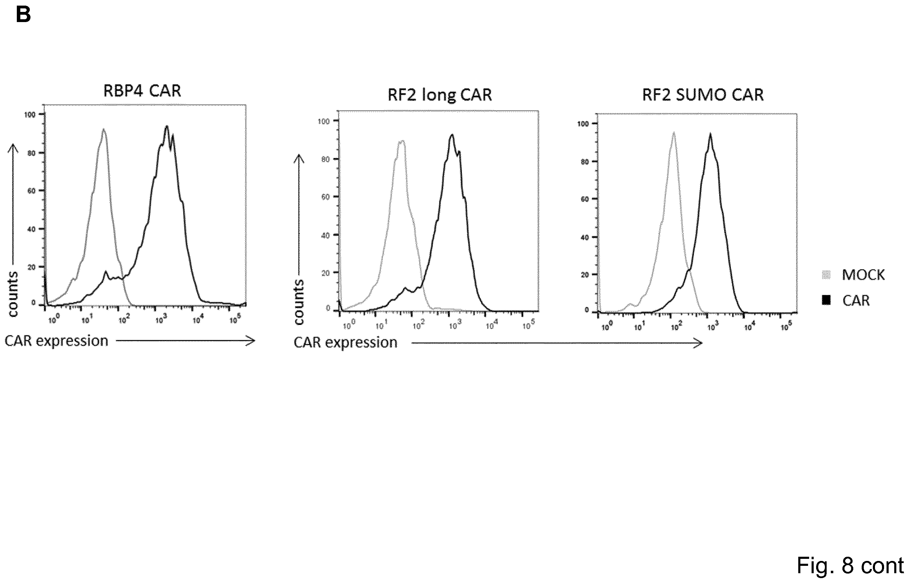

Figs. 8A and 8B show expression of the signal transducing CAR constructs in primary T cells: (A) Expression of constructs containing different rcSso7d-based RBP4 binding interaction partners; (B) Expression of constructs containing different FN3-based RBP4 binding interaction partners or alternatively RBP4 fused to the signaling domains.

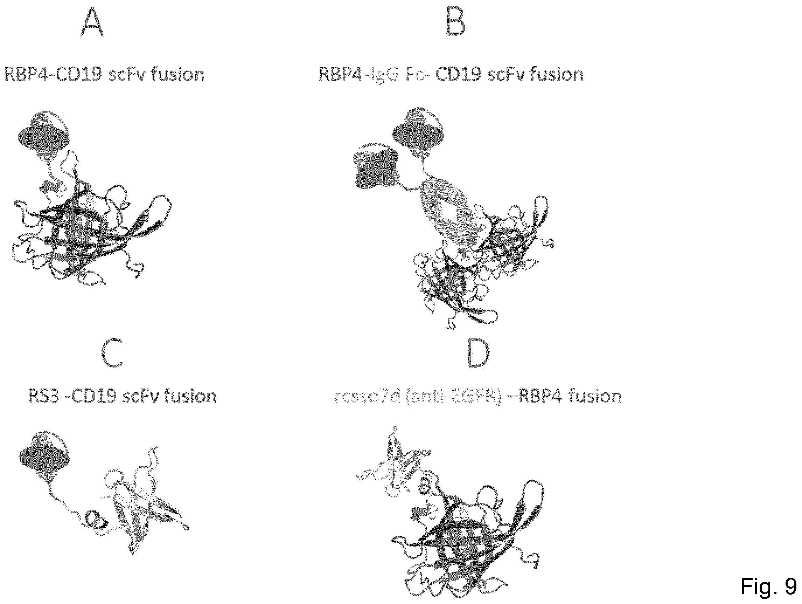

Fig. 9 shows schematics of different antigen binding CAR contructs, i.e., the part of the CAR, in which an antigen binding domain is fused to lipocalin-fold molecule or the lipocalin-fold binding interactin partner. In the shown example either a scFv directed against CD19 or an rcSso7d directed against EGFR was used as antigen binding domain.

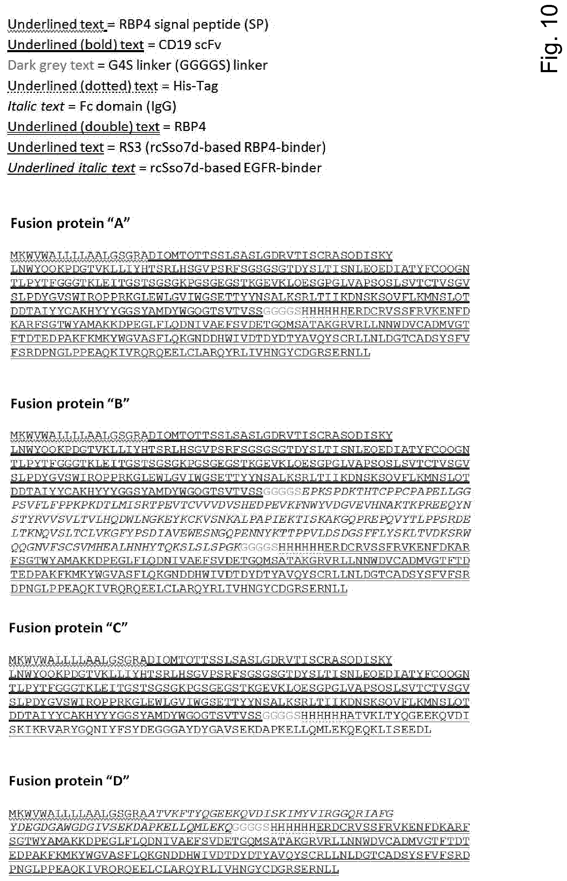

Fig. 10 shows sequences of the antigen binding CAR constructs.

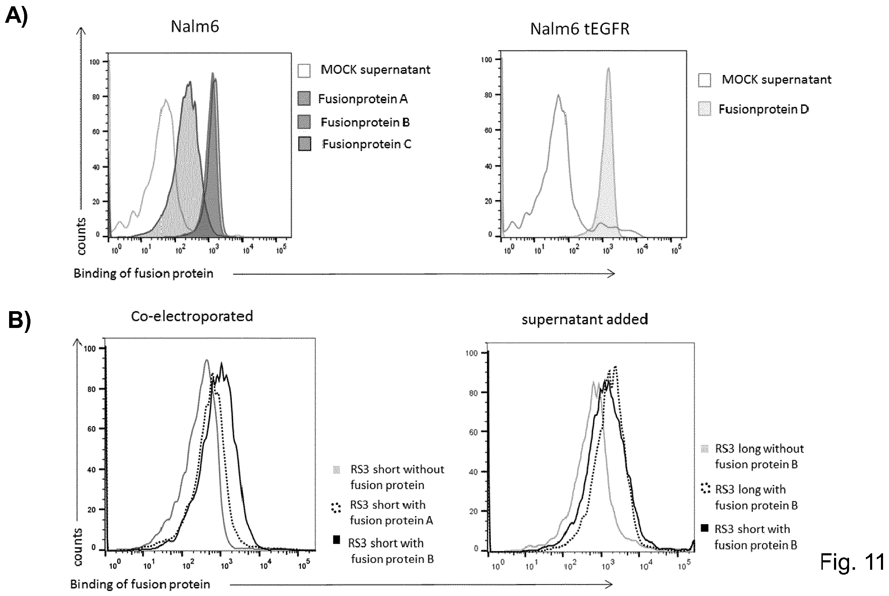

Fig. 11 shows the binding of antigen binding constructs (fusion proteins A-D) to antigen expressing target cells (A) and signaling CAR construct expressing primary human T cells (B): (A) CD19 positive Nalm-6 cells with and without stable expression of a truncated EGFR (tEGFR) were co-incubated with supernatants of Jurkat cells expressing fusion proteins A-D, as indicated; (B) Fusion proteins were either co-expressed in primary human T cells "co-electroporated") or added to primary T cells as supernatants obtained from Jurkat cells that expressed the fusion proteins ("supernatant added"). The primary T cells expressed a signaling transmembrane CAR construct that was either RS3 short CAR or RS3 long CAR, as indicated. Staining of fusion protein binding was performed in the presence of 5 μM A1120.

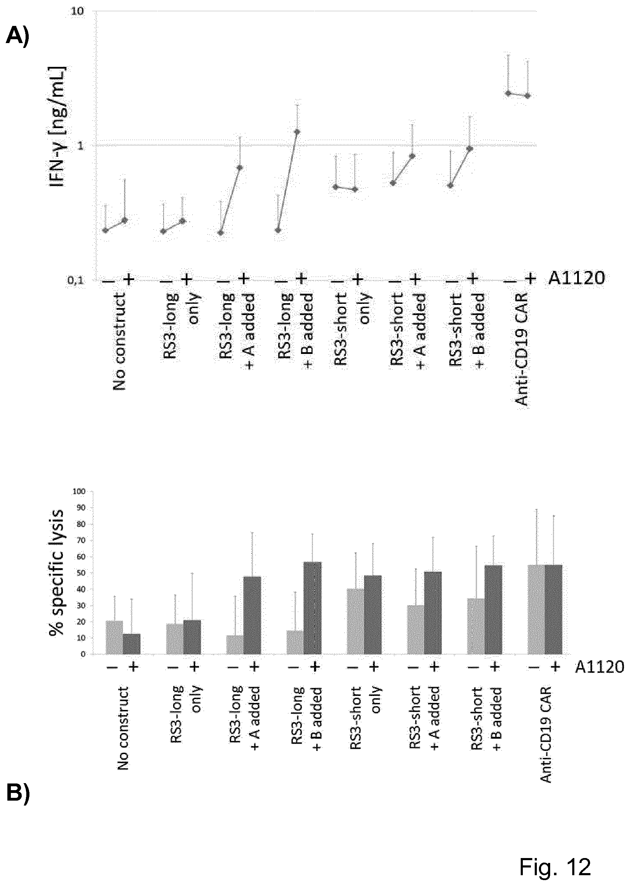

Fig. 12 shows lipocalin-fold ligand (in this case 5 μM A1120) dependent function of different CAR variants in primary T cells, whereby the soluble CAR constructs (fusion proteins A and B) were expressed in Jurkat cells and the supernatants therefrom were added to the primary T cells as indicated. Depicted in (A) and (B) is the capacity for triggering IFN-γ production and cytotoxicity, respectively.

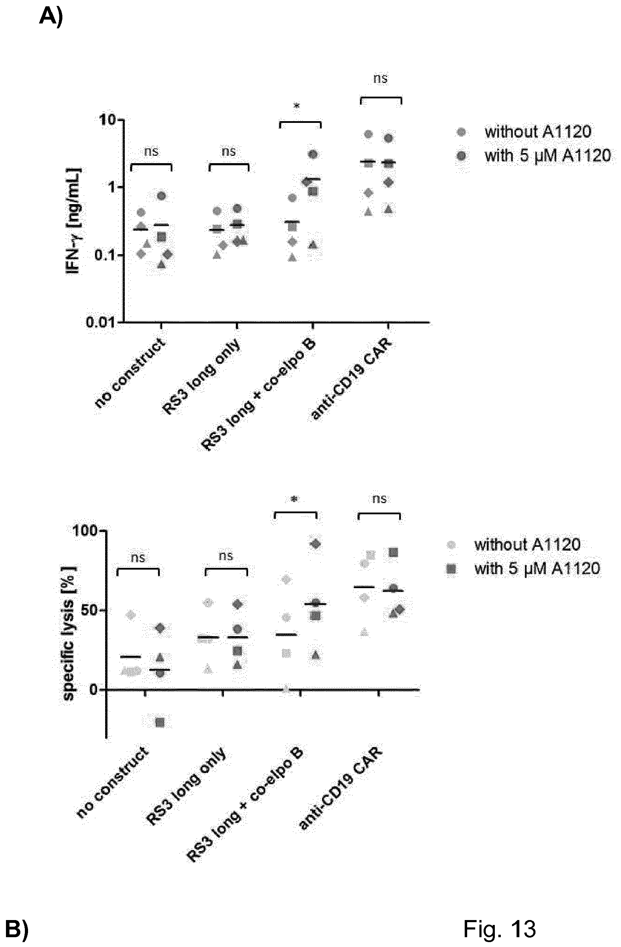

Fig. 13 shows lipocalin-fold ligand (in this case 5 μM A1120) dependent function of the soluble fusion protein B when co-expressed in primary T cells together with CAR construct "RS3 long" ("RS3 long + co-elpo B"). T cells without CAR ("no construct") and T cells expressing only the signal transducing construct but no antigen binding construct ("RS3 long only") served as negative controls. T cells expressing an anti-CD19 CAR served as positive control. Depicted in (A) and (B) is the capacity for triggering IFN-γ production and cytotoxicity, respectively. Statistical significance was calculated using the paired two-tailed and the ratio paired two-tailed Student's t test for specific lysis and IFN-γ levels, respectively (* = $p < 0.05$; ns = $p > 0.05$).

**Examples:**

**Example 1: RBP4-based LRPPI system**

For bovine and human RBP4 there are a series of ligands known to induce conformational changes in the loop regions, which result in dissociation of the natural protein partner TTR (see prior art cited above). In the present example, it is demonstrated by using human RBP4 and its synthetic non-retinoid ligand A1120 (PubChem CID 25138295) how such a ligand-induced conformational switch of a lipocalin-fold molecule can be used as an element in LRPPI. For this purpose, His-tagged full length RBP4 (UniProt ID P02753) was employed as antigen in an alternating screening process in presence (5 $\mu$M) and absence of A1120, respectively, in which ligand-dependent lipocalin-fold binding interaction partners were selected from libraries of two scaffolds with very different protein structure, i.e., FN3- and Sso7d-based scaffolds (Traxlmayr et al., J Biol Chem. 2016;291(43):22496-22508; Chen et al., Methods Enzymol. 2013;523:303-326). As described below, this process yielded lipocalin-fold binding interaction partners which bind RBP4 in an A1120-dependent manner. Figure 3 shows the affinity of these lipocalin-fold binding interaction partners in the presence (5 $\mu$M) and absence of the RBP4-ligand A1120. The diagrams in Fig. 3 show the flow cytometric analysis of the binding intensity at different concentrations of human RBP4 in the presence (5 $\mu$M) or absence of A1120 to selected rcSso7d- or FN3-based lipocalin-fold binding interaction partners, which were displayed on the surface of yeast. RBP4-binding was detected by using an anti-penta-His antibody (Qiagen), followed by flow cytometric detection. rcSso7d-based lipocalin-fold binding interaction partners are termed RS1 through RS5, respectively, whereas FN3-based lipocalin-fold binding interaction partners are termed RF1, RF2 and RF3. The calculated $K_d$ values are displayed below the diagrams (mean of three measurements). These data in Fig. 3 clearly show that it is possible to engineer LRPPI systems based on lipocalin-fold molecules, i.e. that the affinity of a lipocalin-fold molecule "a" (in this case RBP4) to a lipocalin-fold binding interaction partner "c" (in this case different mutants based on the protein scaffolds rcSso7d or FN3) is increased in the presence of a lipocalin-fold ligand "b" (in this case A1120). Moreover, the data in Fig. 3 also show that LRPPI systems based on lipocalin-fold molecules can be engineered by using structurally diverse lipocalin-fold binding interaction partners (in this example based on either rcSso7d or FN3, respectively) containing binding sites that are structurally very different (in FN3-based binders the binding sites are composed of loop regions, whereas in rcSso7d-based binders the binding sites are composed of rigid $\beta$-strands; (Traxlmayr et al., J Biol Chem. 2016;291(43):22496-22508; Chen et al., Methods Enzymol. 2013;523:303-326)). This means that LRPPI systems based on lipocalin-fold molecules are not crucially dependent on a particular structure (i.e. fold) of the lipocalin-fold binding interaction partner and therefore such LRPPI systems are highly flexible regarding the choice of the lipocalin-fold binding interaction partner.

The possibility to engineer lipocalin-fold binding interaction partners that can specifically recognize conformational changes in the lipocalin-fold molecule induced by one lipocalin-fold ligand but not or much less the conformations induced by other lipocalin-fold ligands is a key advantage in engineering clinically applicable LRPPI systems that are orthogonal to each other and can work in parallel. Figure 4 shows that the lipocalin-fold binding interaction partners selected for the A1120-induced conformation of RBP4 indeed have only very low or absent affinity to RBP4 loaded with other ligands known to induce conformational switching of RBP4. This was determined by flow cytometric analysis of the binding intensity (mean of three measurements) of different concentrations of human RBP4 in the presence (5 $\mu$M) or absence of the indicated ligands to selected rcSso7d- or FN3-based lipocalin-fold binding interaction partners, which were displayed on the surface of yeast. The sequences of the respective lipocalin-fold binding interaction partners are shown in Figure 5A and 5B.

**Expression and purification of human RBP4**

Human His-tagged RBP4 (residues 19-201) in pPICZ-alpha-A vector was kindly provided by John Findlay (Marie Curie lab for membrane proteins, Department of Biology, Maynooth University, Co Kildare, Ireland (Wysocka-Kapcinska et al., Protein Expr Purif. 2010;71(1):28-32)) and the sequence was verified using Sanger-sequencing. The protein was expressed in *P. pastoris* strain KM71H. Cells were cultivated in YPG (2% peptone, 1% yeast extract, 1% glycerol) supplemented with Zeocin (100 $\mu$g/mL) at 30°C and 180 rpm overnight. Cells were diluted on the following day and further cultivated until $OD_{600}$ of 2 was reached. Protein expression was induced by centrifugation of the cells and resuspension in YP-medium supplemented with 1% methanol and further incubation at 20°C and 180 rpm for 3 days. Every day fresh methanol was added to a final concentration of 1% to enhance protein expression and subsequent secretion. After 3 days, the supernatant was harvested by 2-step centrifugation (1500 g, 15 min, 4°C and 12200 g, 25 min, 4°C) to first remove the cells and further small particles, respectively. Subsequently, diafiltration was performed to exchange the medium with 50 mM phosphate buffer, pH 7.5. The following purification protocol was adapted from Wysocka-Kapcinska et al. (Protein Expr Purif. 2010;71(1):28-32).

Briefly, the diafiltrated supernatant was supplemented with 5 mM imidazole and applied to a HisTrap FF column (GE healthcare) connected to an ÄKTA FPLC purifier system to enable binding of the His-tagged RBP4 to the column.

After washing with 50 mM phosphate buffer (pH 7.5 containing 500 mM NaCl and 5 mM imidazole), elution was performed using a linear imidazole gradient (from 5% to 100% with 50 mM phosphate buffer, pH 7.5, containing 500 mM NaCl and 500 mM imidazole). Absorbance at 280 nm was detected to observe elution of the protein and the corresponding fractions were analysed by SDS-PAGE. Those fractions showing both absorbance and the presence of a protein band at the expected size (22 kDa corresponding to the His-tagged RBP4), were pooled and concentrated using Amicon Ultra-15 10K Centrifugal filters (Merck Millipore). In addition, buffer was exchanged to phosphate buffered saline (PBS, pH 7.4) to prepare the protein solution for size exclusion chromatography (SEC) with a Superdex 200 column (10 mm x 300 mm, GE Healthcare).

Before SEC, a fraction of RBP4 was labelled with biotin using the EZ-Link Sulfo-NHS-LC-LC-Biotin kit (Thermofisher Scientifc). 10 mM biotin solution was added in a molar excess of 5:1 to the protein solution and incubated at room temperature for 1 hour while stirring. Subsequent preparative SEC was performed to remove remaining unbound biotin molecules and possible aggregates of RBP4, which could only be observed to a limited amount.

**Screening for RBP4 binding interaction partners using yeast display**

Two different scaffolds were used for screening of RBP4 binding interaction partners ("binders"), one of them being reduced charge Sso7d (rcSso7d), a small (7 kDa) thermostable DNA-binding protein from *Sulfolobus solfataricus* in which excess positive charges have been minimized previously (Traxlmayr et al., J Biol Chem. 2016;291(43):22496-22508). The second binder scaffold is the tenth type III domain of human fibronectin (FN3), with a molecular weight of 10 kDa (Chen et al., Methods Enzymol. 2013;523:303-326). Yeast libraries based on rcSso7d (rcSso7d-11 and rcSso7d-18), containing $1.4 \times 10^9$ transformants each and the library G4 based on the FN3 domain (Hackel et al., JMB 2010;401:84-96), containing $2.5 \times 10^8$ transformants, were used. *S. cerevisiae* cells (strain EBY100) were grown in SD-CAA medium (20 g/L glucose, 6.7 g/L yeast nitrogen base, 5 g/L bacto casamino acids, 11.85 g/L sodium citrate dihydrate and 7.4 g/L citric acid monohydrate) at 30°C overnight while shaking. On the following day, cells were sub-cultivated to an $OD_{600}$ of 1 in SD-CAA and growth was monitored by measuring the $OD_{600}$. After reaching a maximum $OD_{600}$ of 4, yeast cells were centrifuged and resuspended in SG-CAA medium (containing the same composition as SD-CAA but 20 g/L galactose and 2 g/L glucose instead of 20 g/L glucose) for induction of surface expression of rcSso7d- or FN3-mutants, respectively. Cells were incubated at 20°C overnight with shaking and harvested by centrifugation. Two cycles of bead selection were performed with magnetic streptavidin-coated Dynabeads (Life technologies) loaded with biotinylated RBP4. To avoid the selection for non-specific binders or binders which are specific for streptavidin, negative selection (with bare beads) was performed between cycles of positive selection. In all bead selections, the RBP4 ligand A1120 was present at a concentration of 5 $\mu$M. After the second bead-selection cycle, error prone PCR (epPCR) was conducted in order to increase mutations in rcSso7d and FN3 genes which might contribute to binding. The plasmid DNA was subjected to 19 cycles of PCR using nucleotide analogs (2 $\mu$M of 8-oxo-2'-deoxyguanosine-5'-triphosphate, 8-oxo-dGTP, and 2$\mu$M 2'-deoxy-p-nucleoside-5'-triphosphate, dPTP) and the primers epSso_fwd (5'-GGCTCTGGTGGAGGCGGTAGCGGAGGCGGAGGGTCGGCTAGC-3') and epSso_rev (5'-CTATTACAAGTCCTCTTCAGAAATAAGCTTTTGTTCGGATCC-3') for the rcSso7d-based lipocalin-fold binding interaction partners; and the primers FN3_fwd (5'-CGACGATTGAAGGTAGATACCCATACGACGTTCCAGACTACGCTCTGCAG-3') and FN3_rev (5'-ATCTCGAGCTATTACAAGTCCTCTTCAGAAATAAGCTTTTGTTCGGAT CC-3') for the FN3-based lipocalin-fold binding interation partners. The resulting product was used as a template for amplification by a second PCR. Afterwards, EBY100 cells were transformed with linearized pCTCON2 vector (Chao et al., Nat Protoc. 2006;1(2):755-768; Angelini et al., Methods Mol Biol. 2015;1319:3-36) and insert (PCR product) using the square wave protocol (single pulse, 500 V, 15 ms) and Bio-Rad Gene Pulser Xcell (Bio-Rad). After a third round of bead-selection (comprising 3 negative and 1 positive selection), libraries were further enriched by fluorescence activated cell sorting (FACS). Staining of yeast cells was performed in PBS supplemented with 0.1% bovine serum albumin (BSA) in the presence of 5 $\mu$M A1120 (Sigma-Aldrich) using 300 nM biotinylated antigen (RBP4) and 5 $\mu$g/mL mouse anti-c-myc antibody 9E10 (Thermo Fisher Scientific) and incubation at 4°C for 1 hour while shaking. After a washing step, secondary staining was performed with 20 $\mu$g/mL streptavidin-Alexa Fluor 647 and 20 $\mu$g/mL anti-mIgG-AF488 (both from Thermo Fisher Scientific) for 20 minutes at 4°C while shaking. After a final washing step, cells were sorted using a FACS Aria Fusion (BD). In some sorting rounds, cells were stained with non-biotinylated RBP4 as primary reagent followed by secondary staining with 1 $\mu$g/mL anti-HA-Alexa Fluor 647 (clone 16B12, Bio-Legend) and 5 $\mu$g/mL Penta-His-Alexa Fluor 488 (Qiagen), to avoid enrichment of binders recognizing biotinylated epitopes. Between the second and third FACS selection, another epPCR was performed. One round of negative selection was performed in which no A1120 was present and mutants with strongly reduced binding signal were sorted. In total, 6 or 7 rounds of FACS were performed for selection of RBP4-binding interaction partners.

**Soluble expression of selected RBP4-binding interaction partners**

After the last selection round, 96 clones were sequenced. Based on the sequence, 16 RBP4-binders (9 rcSso7d- and 7 FN3-based lipocalin-fold binding interaction partners) were chosen and used for transformation of EBY100 cells. The affinity of single clones was determined by flow cytometry and titration of RBP4. Based on the affinity and the expression level, 8 binders (5 rcSso7d- and 3 FN3-based lipocalin-fold binding interaction partners) were further chosen for soluble expression. For this purpose, binders were subcloned into the pE-SUMO-vector (Life-Sensors) and expressed as fusion proteins with His6-tagged small ubiquitin-like modifier (SUMO) protein. After transformation of Rosetta (DE3) *E. coli* cells (Merck Millipore) with the sequence-verified plasmids, cultures were incubated in LB medium supplemented with kanamycin (50 $\mu$g/mL) and chloramphenicol (34 $\mu$g/mL) at 37°C overnight while shaking. Cells were diluted in terrific broth (12 g/L tryptone, 24 g/L yeast extract, 4% glycerol, 2,31 g/L $KH_2PO_4$ and 16,43 g/L $K_2HPO_4*3H_2O$) supplemented with kanamycin (50 $\mu$g/mL) and chloramphenicol (34 $\mu$g/mL) and further incubated at 37°C until an $OD_{600}$ of 2 was reached. Expression was induced by addition of 1 mM isopropyl $\beta$-D-1-thiogalactopyranoside (IPTG) and cells were further cultured overnight at 20°C. On the following day, cells were harvested by centrifugation (5000 g, 20 min, 4°C) and resuspended in sonication buffer (50 mM sodium phosphate, 300 mM NaCl, 3% glycerol, 1% Triton X-100, pH 8). After sonication (2 x 90 s, duty cycle 50%, amplitude set to 5) and centrifugation (20000 g, 30 min, 4°C), purification of the supernatants containing the HIS6-SUMO-fusion proteins was performed using the TALON metal affinity resin (Clontech). Before application to the column, imidazole was added to the supernatants to a final concentration of 10 mM to avoid unspecific binding. Afterwards, supernatants were applied to the column twice, followed by several washing steps with equilibration buffer (50 mM sodium phosphate, 300 mM NaCl, pH 8) containing increasing amounts of imidazole (5-15 mM). Elution was performed with equilibration buffer supplemented with 250 mM imidazole. The buffer was exchanged to PBS using Amicon Ultra-15 10K Centrifugal filters (Merck Millipore) and the concentration was determined by measuring the absorbance at 280 nm with a Nanodrop instrument. A portion of the fusion-proteins was directly frozen at -80°C while the rest was digested overnight at room temperature with SUMO-protease 1 (1 mg enzyme was used for 100 mg fusion-protein), resulting in cleavage of the hexahistidine tag and SUMO protein from the respective binder. The digested protein was again purified with TALON metal affinity resin (Clontech). His-tagged SUMO protein and His-tagged SUMO protease 1 bound to the resin while cleaved rcSso7d- or FN3-based lipocalin-fold binding interaction partners were found in the flow-through, which was further analyzed by SDS-PAGE. The absorbance at 280 nm of purified rcSso7d- and FN3-based lipocalin-fold binding interaction partners was determined by Nanodrop analysis and the concentration was calculated using the corresponding extinction coefficients. For biophysical measurements, the buffer was exchanged with PBS using Amicon Ultra-15 3K centrifugal filters (Merck Millipore) and the proteins were frozen at -80°C.

**Biophysical characterization of RBP4-binding interaction partners**

The stability of RBP4-binding interaction partners is evaluated by determining the melting temperature ($T_m$) by differential scanning calorimetry (DSC) using MicroCal VP-DSC capillary cell microcalorimeter (MicroCal). Briefly, 30 $\mu$M protein solution is exposed to an increasing temperature ranging from 20-110°C with a heating rate of 1°C/min. Buffer baseline is subtracted and the resulting data are normalized for protein concentration, followed by fitting with a non-two-state thermal unfolding model.

Surface plasmon resonance (SPR) is used to determine the affinity of the interaction between RBP4 and rcSso7d- or FN3-based lipocalin-fold binding interaction partners and is performed with BiacoreT200 (GE healthcare). The antigen (RBP4) is coated onto a chip and incubated with various concentrations of binder solution in PBS, followed by a dissociation phase in PBS only. All measurements are performed both in the presence and absence of A1120 or other ligands. Finally, $k_{on}$, $k_{off}$ and $K_d$ values are obtained by global fitting.

For analytical SEC analysis, a total amount of 25 $\mu$g rcSso7d or FN3 mutants in running buffer (PBS with 200 mM NaCl) is filtered through 0.1 $\mu$m Ultrafree-MC filter (Merck Millipore), applied to a Superdex 200 10/300 GL column (GE healthcare) connected to an HPLC prominence LC20 system (Shimadzu) and eluted with a flow rate of 0.75 mL/min at 25°C. In addition, multi-angle light scattering (MALS) is used for determining the molecular mass using WYATT Heleos Dawn8+ plus QELS, a refractive index detector RID-10A (Shimadzu) and a diode array detector SPD-M20A (Shimadzu).

For the thermodynamic characterization of the interaction between RBP4 and rcSso7d- or FN3-based lipocalin-fold binding interaction partners, isothermal titration calorimetry (ITC) is conducted using an automated MicroCal PEAQ-ITC instrument (Malvern Instruments). The samples are centrifuged (17,000 g, 10 min, 20°C) and filtered (0.1 $\mu$m Ultrafree-MC filter, Merck Millipore). RBP4 protein solution is applied to the sample cell and the respective binders are titrated with varying intervals. Different concentrations both for RBP4 and binders are used and in some experiments, binders are applied to the sample cell and RBP4 is titrated. The experiments are performed both in the presence and absence of the ligand A1120 or other ligands. Data analysis is performed with the MicroCal PEAQ-ITC analysis software.

**Example 2: Identification of clinically applicable lipocalin-fold ligands with capacity for inducing conformational switching in a lipocalin-fold**

By using human RBP4 and its native interaction partner TTR we exemplified a strategy for identifying novel lipocalin-fold ligands with capacity for inducing conformational switching of a lipocalin-fold. With the aim of identifying clinically applicable lipocalin-fold ligands we performed a virtual screening of several databases (World Drug Index, KEGG Medicus, KEGG Ligands, DrugBank, Human Metabolome Database, ChEBI, ChEMBL, MDDR) with different stringency by using a pharmacophore model deduced from the RBP4/A1120 complex (PDB 3FMZ). The result of this virtual screening is shown in Table 1, which contains a series of approved and experimental drugs and other molecules potentially attractive for clinical and non-clinical applications.

Table 1:

| Nr | database HMDB | | | | |
|---|---|---|---|---|---|
| 1 | Nafcillin | 28 | Gancaonin X | 59 | Imiprothrin |
| 2 | Gerberinol | 29 | Rubraflavone D | 60 | Triclabendazole |
| 3 | Montelukast | 30 | Cyclokievitone hydrate | 61 | Fasinex |
| 4 | Flurazepam | | | 62 | Brivanib alaninate |
| 5 rate | Quinidine barbitu- | 31 | Glyceollidin II | 63 | Transfluthrin |
| | | 32 | Cyclandelate | 64 | Enolicam sodium |
| | | 33 | Dulxanthone E | 65 | Enolicam sodium monohydrate |
| 6 | Glyceollin I | 34 | Morusin | | |
| 7 | Glyceollin II | 35 | (E)-2',4,4'-Trihydroxy-3-prenylchalcone | 66 | Dibucaine |
| 8 | (-)-Shinpterocarpin | | | 67 | Cinchocaine |
| 9 | Kanzonol W | 36 | Dulxanthone H | 68 | Nupercaine |
| 10 | 6alpha-Hydroxyphaseollin | 37 | Judeol | 69 | Trametinib |
| | | 38 | Artonin E | | |
| 11 | (la,5b,6a)-7-Protoilludene-1,5,6,14-tetrol 14-(2,4-dihydroxy-6-methylbenzoic acid) | 39 | Kanzonol T | | database WDI |
| | | 40 | Fragransol A | 70 | FLUCLOXACILLIN |
| | | 41 | Dulxanthone F | 71 | S-FARNESYLTHIOSALICYLIC ACID |
| | | 42 | Mulberrofuran T | | |
| 12 W | 4'-O-Methylkanzonol | 43 | Garcimangosone A | 72 | 2-FLUOROTROPAPRIDE |
| | | 44 | Artonin B | 73 | BUTAMPICILLIN |
| 13 | Cyclokievitone | 45 | Asteltoxin | 74 FATE | DICLOXACILLIN SUL- |
| 14 | Licofuranone | | | | |
| 15 | Armillatin | | database KEGG | 75 | FUROXACILLIN |
| 16 | 2-(4-Methyl-3-pentenyl)anthraquinone | 46 | Oxyfedrine | 76 | IBUCILLIN |
| | | 47 | Profluthrin | 77 | PRAZOCILLIN |
| | | 48 | Momfluorothrin | 78 | SALETAMIDE |
| 17 | Sorafenib beta-D-Glucuronide | 49 | Xyloylsulfamine | 79 ILIDE | TETRACHLORSALICYLAN- |
| 18 | Heterophyllin | 50 chloride | Cetotiamine hydro-hydrate | | |
| 19 | 2'-O-Methylphaseollinisoflavan | | | 80 | CARBENICILLIN |
| | | 51 chloride | Dicethiamine hydrohydrate | 81 | DICLOMETIDE |
| 20 | Tiapride | | | 82 | METHYL-SULFOMETURON |
| 21 | Gluten exorphin C | 52 | Dicetamin | 83 LIDE | TRICHLOROSALICYLANI- |
| 22 | Mulberrofuran M | 53 | Oxolamine | | |
| 23 | Dulxanthone G | 54 | Oksalamin | 84 | CLOMETOCILLIN |
| 24 | Sclareol | 55 | Crisnatol mesylate | 85 | CYSTODYTIN-F |

(continued)

| No. | Compound | No. | Compound | No. | Compound |
|---|---|---|---|---|---|
| 25 | Colupdox a | 56 | Ioflubenzamide I | 86 | DETANOSAL |
| 26 | Kanzonol F | 57 | Ceritinib | 87 | DIARBARONE |
| 27 | Mangostinone | 58 | Zykadia | 88 | DICLOFOP |
| 89 | EPIPHENETHICILLIN | 121 | PSORALIDIN | 157 | BECLOBRIC-ACID-GLUCURONIDE |
| 90 | FTALIL-MEDEYOL | 122 | RUBIGINONE-C1 | 158 | CLOXACILLIN |
| 91 | SALETAMIDE HYDRO-CHLORIDE | 123 | SECOPSEUDOPTEROSIN-E | 159 | HYPERGUINONE-B |
| 92 | TIAPRIDE HYDROCHLO-RIDE | 124 | ASADISULFIDE | 160 | OLIGOSPOROL-A |
| 93 | TRUNCULIN-A | 125 | BARANGCADOIC-ACID-A | 161 | PROPOXYCAINE HYDROCHLORIDE |
| 94 | DEOXYPENTALENYLGLU-CURON | 126 | BEPHEDON | 162 | RONIFIBRATE |
| 95 | LAFLUNIMUS | 127 | MELLEDONAL-B | 163 | SUDAN-BLUE-GN |
| 96 | MERAZOLAM | 128 | NERAMINOL | 164 | TRICHODERMAMIDE-B |
| 97 | DIBUSADOL | 129 | PHOMOPSOLIDE-A | 165 | BOTRYLLAMIDE-A |
| 98 | PHENETICILLIN POTAS-SIUM | 130 | ROBUSTIC-ACID | 166 | CARFECILLIN |
| 99 | PRANOSAL | 131 | ZOPFIELLAMIDE-A | 167 | CLETHODIM |
| 100 | DALEFORMIS | 132 | CYSTODYTIN-B | 168 | DUTADRUPINE |
| 101 | DIPHENICILLIN | 133 | DICLOXACILLIN | 169 | EPICOCHLIOQUINONE-B- |
| 102 | FENOTEROL HYDROCHLO-RIDE | 134 | FLUOROPROPRANOLOL | 14 | FLURAZEPAM |
| 103 | GIGANTIC-ACID | 135 | ILIOCICOLIN-B | 170 | HYDROXYFLUCLOXACIL-LIN |
| 104 | HALOLITORALIN-B | 136 | INDICANINE-B | 171 | RHINACANTHIN-C |
| 105 | ISOPROPICILLIN | 137 | JACAREUBIN | 172 | TEFLUBENZURON |
| 106 | PROGUANIL HYDROCHLO-RIDE | 138 | KOTTAMIDE-C | 173 | XENYSALATE |
| 107 | PYRANOKUNTHONE-B | 139 | MEXOLAMINE | 174 | ANTIMYCIN-A8A |
| 108 | TUBEROSIN | 140 | MYCAPEROXIDE-H | 175 | ARTOINDONESIANIN-U |
| 109 | ZOPFIELLAMIDE-B | 141 | OTOGIRIN | 176 | BRONCHOCAINE |
| 110 | CLOXACILLIN SODIUM | 142 | OXOLAMINE HYDROCHLO-RIDE | 177 | ENOLICAM |
| 111 | LETIMIDE HYDROCHLO-RIDE | 143 | OXOPROPALINE-A | 178 | IPAZILIDE |
| 112 | BAIGENE-B | 144 | PURVALANOL-A | 179 | MORDANT-BROWN-1 |
| 113 | BIDWILLON-B | 145 | RUBIGINONE-C2 | 180 | PROPRANOLOL PHENO BARBITAL |
| | | 146 | TERACRYLSHIKONIN | 181 | PUGHIININ-A |
| | | 147 | CLODINAFOP-PROPARGYL-ESTER | 182 | AMPHIBINE-H |
| | | 148 | MAZATICOL | 183 | |
| | | 149 | SETHOXYDIM | | |
| | | 150 | SULFAGUANOLE | | |

(continued)

| No. | Name |
| --- | --- |
| 114 | CARBENICILLIN DISO-DIUM |
| 115 | HYDROXY PROCAINE |
| 116 | OCHRATOXIN-A |
| 117 | THEROX |
| 118 | MACARANGAFLAVANONE-B |
| 119 | MENOXYMYCIN-B |
| 120 | PENICILLIN-S |
| 151 | BALAPERIDONE |
| 152 | FLUCLOXACILLIN SODI-UM |
| 153 | GEODIAMOLIDE-TA |
| 154 | LUCANTHONE-SULFOXIDE |
| 155 | MELLEOLIDE-D |
| 156 | NADOXOLOL HYDROCHLO-RIDE |
| 184 | ARMILLARIC-ACID |
| 185 | CARINDACILLIN |
| 186 | CHLOROBIOCATE |
| 187 | CHLORPROGUANIL |
| 188 | CYLINDROL-B |
| 189 | ECLIPTALBINE |
| 190 | GARCIGERRIN-A |
| 191 | O- |
| 192 | DEMETHYLCHLOROTHRICIN |
| 193 | SANGGENON-C |
| 194 | TETRAPTEROL-G |
| 195 | CHLORSULFURON |
| 196 | DEXAMETHASONE-DIETHYLAMINOACETATE |
| 197 | DIETHYXIME |
| 198 | PYRIDOVERICIN |
| 199 | SUBENDAZOLE |
| 200 | THIOCAINE |
| 201 | TRAPEZIFOLIXANTHONE |
| 202 | TRICLAZAN |
| 203 | 3',4'-DICHLOROBENZAMIL |
| 204 | CHAETOVIRIDIN-C |
| 205 | CYCLOGREGATIN |
| 206 | FLURAZEPAM MONOHYDROCHLORIDE |
| 207 | FUSIDILACTONE-B |
| 208 | GRISEOCHELIN-METHYL-ESTER |
| 209 | ISOBUTYL-SHIKONIN |
| 210 | MICINICATE |
| 211 | AJUDAZOL-B |
| 212 | CYSTODYTIN-E |
| 225 | IDARUBICIN HYDRO-CHLORIDE |
| 226 | MUTISICOUMARANONE-A |
| 227 | 3-O-METHYLCALOPOCARPIN |
| 228 | ALLOCLAMIDE HYDRO-CHLORIDE |
| 229 | ANOPTERINE |
| 230 | DIOXATION |
| 231 | EUGLOBAL-IIC |
| 232 | ILICICOLIN-C |
| 233 | MYCINOLIDE-II |
| 234 | SUILLIN |
| 235 | TOCOTRIENOL-GAMMA |
| 236 | INDOMYCINONE-BETA |
| 237 | ISOTHIORBAMINE |
| 238 | MEBEVERINE |
| 239 | MUTISICOUMARANONE-D |
| 240 | NYMPHAEOL-C |
| 241 | PRUSOCAIN |
| 242 | ZIMET-20-84 |
| 243 | 2,4-D-BUTOXYPROPYL |
| 244 | ABYSSINONE-IV |
| 245 | BAIGENE-A |
| 246 | CHRYSOCHLAMIC-ACID |
| 260 | RATJADONE |
| 261 | SALVERINE HYDROCHLO-RIDE |
| 262 | 4-MENAHYDROQUINONE |
| 263 | BOTRYLLAMIDE-C |
| 264 | ELSAMICIN |
| 265 | FENFLUTHRIN |
| 266 | MUTISIPHENONE-A |
| 267 | SANGGENON-A |
| 268 | SCHWEINFURTHIN-A |
| 269 | VANYLIDILOL |
| 270 | 4-O-METHYLMELLEOLIDE |
| 271 | ACETYLVISMIONE-F |
| 272 | ALFENTANIL-HYDROCHLORIDE |
| 273 | ASPERTETRONIN-A |
| 274 | BETA-HYDROXYISOVALERYLSHIKONIN |
| 275 | CARBISOCAINE |
| 276 | CHLORPROGUANIL HY-DROCHLORIDE |
| 277 | CRYPTOPHYCIN-52 |
| 278 | DIDEMETHYL-TOCOTRIENOL |
| 279 | FLUOSOL-DA |

(continued)

| | | | | | |
|---|---|---|---|---|---|
| 212 | DEMETHYLPRAECANSONE-A | 247 | EPOLONE-B | 280 | PYRIDOXYPHEN |
| | | 248 | ETHOXYCAINE | 281 | TIAZESIM HYDROCHLO-RIDE |
| 213 | DESTRUXIN-A | 249 | FLUORENAMIL | | |
| 214 | DIFENIDOL EMBONATE | 250 | GUAIACOL-MEFENAMATE | 282 | BUTOCTAMIDE |
| 215 | DISCOKIOLIDE-B | 251 | MAXIMA-ISOFLAVONE-C | 283 | DEOXYSHIKONIN |
| 216 | IRUMANOLIDE-2 | 252 | SARCODICTYIN-B | 284 | GAMBIERIC-ACID-A |
| 217 | LACTOQUINOMYCIN | 253 | TRICLABENDAZOLE | 285 | LICOFURANONE |
| 218 | NEOBORNYVAL | 254 | ACHYOFURAN | 286 | PREDNYLIDENE-DIETHYLAMINOACETATE |
| 219 | SAROTHRALEN-D | 255 | ASTERRIQUINONE | | |
| 220 | ABYSSINONE-V | 256 | DIETHYLGLYCOLATE- | 287 | PSEUDOPTEROSIN-G |
| 221 | AXITIROME | | TOLYHYDRAZIDE | 288 | TRIKENDIOL |
| 222 | CHLORFLUAZURON | 257 | MALLOTOPHILIPPENS-D | 289 | ZOAPATANOL |
| 223 | CHONDRILLIDIENE-18,20 | 258 | NAFTOXATE | 290 | ERGOKININ-C |
| 224 | EUGLOBAL-G2 | 259 | PACHYDICTYOL-A-EPOXIDE | 291 | PENTAMOXANE HYDRO-CHLORIDE |
| 292 | SCANDENIN | 327 | DIMETPRAMIDE | 357 | NAPYRADIOMYCIN-C1 |
| 293 | ACTINOPYRONE-C | 328 | FENOTEROL HYDROBROMIDE | 358 | SAROASPIDIN-B |
| 294 | AMITON (als Bsp. für Gift!) | | | 359 | SARRACINE |
| | | 329 | PHENKAPTON | 360 | ERECTONE-B |
| 295 | CRATOXYARBORENONE-C | 330 | CRATOXYARBORENONE-B | 361 | HOMOHARZIANIC-ACID |
| 296 | CYMOPOL | 331 | ETHOMOXANE HYDRO- | 362 | ASTERRIQUINONE-B1 |
| 297 | DOXYCYCLINE-HYCLATE | | CHLORIDE | 363 | DETAMID |
| 298 | FLAVIDULOL-C | 332 | ISOCENTRATHERIN | 364 | RUBRAXANTHONE |
| 299 | FRAN-12 | 333 | KALIMANTACIN-A | 365 | DOLASTATIN-19 |
| 300 | MYRIAPORONE-3 | 334 | HISPAGLABRIDIN-A | 366 | EDETOL |
| 301 | ORINOCINOLIDE | 335 | LANKACYCLINOL | 367 | PHASEOLLIDIN |
| 302 | TONABERSAT | 336 | MACLURAXANTHONE | 368 | GEDOCARNIL |
| 303 | VISMIONE-B | 337 | PIVAMPICILLIN HYDROCHLORIDE | 369 | MANUMYCIN-B |
| 304 | AMIKHELLINE | | | 370 | SANTALOL-BETA- |
| 305 | BUTAMOXANE HYDROCHLORIDE | 338 | PLURAFLAVIN-A | | SALICYLATE |
| | | 339 | SIGMOIDIN-I | 371 | COWANIN |
| 306 | CHLOROBIOCIN | 340 | FENALAMIDE | 372 | INDIBULIN |
| 307 | CYCLOCOMMUNIN | 341 | HYDROXYACLACINOMY-CIN-M,2 | 373 | COWANOL |
| 308 | FENAZAFLOR | | | 374 | KARATAVICIN |
| 309 | VISMIONE-D | 342 | SOPHORADIN | 375 | PICLOXYDINE |

(continued)

| | | | | | |
|---|---|---|---|---|---|
| 310 | 3-TRICHLORMETAPHOS | 343 | ASCOCHLORIN | 376 | PROXAZOLE |
| 311 | AMINOPROPYLONE PHENYLBUTAZONE | 344 | BETA-HYDROXYSANSHOOL | 377 | STROBILURIN-E |
| 312 | DIOCLENOL | 345 | BISTRAMIDE-K | 378 | ERECTQUIONE-B |
| 313 | GRIFOLIN | 346 | CHLORTETRACYCLINE BORATE | 379 | SURICAINIDE |
| 314 | HYPERJOVINOL-A | 347 | DEHYDROASCOCHLORIN-8+, 9+ | 380 | DIETHYLAMINOMETHYL-RUTIN |
| 315 | TAMOLARIZINE | 348 | DIMETHIALIUM CHLORIDE | 381 | TROPESIN |
| 316 | TOMENTOL | 349 | MACARANGIN | 382 | PICLOXYDINE HYDRO CHLORIDE |
| 317 | TRANS-DELTA-TOCOTRIENOLOIC-ACID | 350 | MARCELLOMYCIN | 383 | HEX-1 |
| 318 | DIACETOLOL HYDROCHLORIDE | 351 | BENZOYLGOMISIN-H | 384 | DECLOVANILLOBIOCIN |
| 319 | DUTOMYCIN | 352 | CLINDAMYCIN HYDROCHLORIDE | | database MDDR |
| 320 | LIGUROBUSTOSIDE-O | 353 | HYDROXYASCOCHLORIN-8+ | 385 | RUBIGINONE C2 |
| 321 | RAISPAILOL-B | 354 | NEOCARZILIN-A | 386 | FLOBUFEN |
| 322 | ERECTQUIONE-A | 355 | CHAETOVIRIDIN-B | 387 | TETRONOTHIODIN |
| 323 | SAROTHRALEN-A | 356 | CHLORTETRACYCLINE BISULFATE | 388 | LAFLUNIMUS |
| 324 | SITAMAQUINE | | | 389 | MYCAPEROXIDE A |
| 325 | TRICHOPOLYN-II | | database CHEMBL | 390 | FLURAZEPAM HYDRO CHLORIDE |
| 326 | 3-HYDROXYTOLUFAZEPAM | 396 | CARBENICILLIN | | |
| 391 | RUBIGINONE C1 | 397 | DICLOFOP | 399 | FLOBUFEN |
| 392 | CRISNATOL MESYLATE | 398 | EPIPHENITICILLIN | 400 | GIGANTIC ACID |
| 393 | DOLASTATIN D | | | 401 | PHENETICILLIN POTAS-SIUM |
| 394 | EPOLACTAENE | | | 402 | DIPHENICILLIN |
| 395 | LEXIPAFANT | | | | |

The capacity of some of these ligands for inducing conformational switching of RBP4 is detected by exploiting the conformation dependent binding of TTR, which is inhibited by ligands with capacity for inducing conformational switching of RBP4.

**Flow cytometric detection of ligand induced conformational switching of RBP4**

RBP4-encoding DNA is isolated out of *P.pastoris* using the zymoprep yeast plasmid miniprep kit II (Zymo Research) followed by amplification by PCR using the primers picZalpha_fwd (5'-ATTGCCAGCATTGCTGCTAAAGAAG-3') and picZalpha_rev (5'-GCAAATGGCATTCTGACATCC-3'). The DNA is purified using the illustra GFX PCR DNA and Gel Band Purification Kit (GE healthcare) and sequenced. For cloning into the vector pCTCon2, the RBP4-encoding DNA is amplified with PCR using primers encoding NheI/BamHI restriction sites homologous to the vector. After digestion with NheI and BamHI (both New England Biolabs), the RBP4 encoding sequence is ligated into the linearized vector and electroporated in *E.coli* for verification of the sequence. The different domains of the resulting fusion protein have the order Aga2p - HA-tag - (Gly$_4$Ser)$_3$ Linker - RBP4 - c-myc Tag. *S.cerevisiae* strain EBY100 is then transformed with the Quick and easy transformation kit (Takara).

Expression of RBP4 on yeast is induced by culturing the cells in SG-CAA medium (2 g/L glucose, 20 g/L galactose 6.7 g/L yeast nitrogen base, 5 g/L bacto casamino acids, 11.85 g/L sodium citrate dihydrate and 7.4 g/L citric acid monohydrate) overnight at 20°C and 180 rpm. For detection of RBP4 expression on the surface of yeast cells, cells are stained with either mouse anti-c-myc antibody 9E10 (Thermo Fisher Scientific) followed by secondary staining with anti-mIgG-AF488 (Thermo Fisher Scientific) or with anti-HA-Alexa Fluor 647 (BioLegend).

Flow cytometric detection of ligand induced conformational switching of RBP4 is based on employing the conformation dependent binding behaviour of TTR. Hereto, recombinant TTR (labelled with Alexa Fluor 488 NHS ester kit, Thermo Fisher Scientific) is added to RBP4-expressing yeast in absence or presence of different concentrations of various small molecule ligands in the staining buffer (PBS supplemented with 0.1% BSA).

**Example 3: Integration of an RBP4-based LRPPI system into a CAR**

The third example demonstrates the applicability of a LCN-fold based LRPPI for regulation of CAR function by using the RBP4-based LRPPI system described in example 1. The schematics of the CAR constructs shown in Figures 6A, 6B and 9 illustrate some tested possibilities of integrating such a lipocalin-fold based LRPPI into a CAR. Figures 7A, 7B, 7C and 10 show the sequences of the tested constructs and Figures 8A, 8B and 8C illustrates the expression of signaling CAR constructs in primary human T cells. Primary human T cells were electroporated with 5 μg mRNA for each construct and CAR expression was detected 20 h after electroporation via Strep II Tag or in the case of "RS3 CAR long without c-myc Tag" and "RF2 long CAR" via the Fc domain by using a biotinylated anti-human-IgGl-antibody as primary antibody and a PE-conjugated streptavidin as secondary staining reagent. Figures 11A and 11B show the binding of CAR constructs containing the antigen binding domain (fusion proteins A-D, detected via the integrated His Tag) to target cells and to primary human T cells expressing different transmembrane CAR signaling constructs in the presence of 5 μM A1120 (20 h after electroporation of 5 μg mRNA). The fusion proteins were either co-expressed in the primary T cells or added to the primary T cells as supernatants obtained form Jurkat cells that expressed the fusion proteins. The ligand-dependent function of the resulting CARs, as determined by induction of cytotoxicity and cytokine producinon of primary human T cells in absence and presence of 5 μM A1120, is shown in Figures 12 and 13.

**Design of CARs**

Selected rcSso7d- and FN3-based RBP4-binding interaction partners were incorporated into a second-generation CAR signaling backbone, comprising a CD8 stalk, a 4-1BB costimulatory domain and a CD3zeta signaling domain ("8a-BBz"), or an IgG-Fc spacer fused to a CD28 costimulatory domain and a CD3zeta signaling domain ("Fc-28z"), respectively. RBP4-binding interaction partners based on rcSso7d (RS1-RS5) and Fibronectin (RF1-RF3) were fused to the CAR backbones via a Strep-Tag and one or two repeats of G4S (4x glycine and 1x serine) amino acid residues as linker. The composition of the constructs is given in the schematics of Figure 6A and 6B and the sequences are shown in Figure 7A, 7B and 7C. The secreted antigen binding CAR constructs contained either a FMC63-based scFv directed against human CD19 or rcSso7d directed against human EGFR fused to either IgG1-Fc-RBP4, or RBP4 or alternatively a lipocalin-fold binding interaction partner rcSso7d RS3. The schematics and sequences are shown in Fig. 9 and 10, respectively. CAR constructs were constructed by Gibson Assembly (NEB), using 0.02-0.5 pmol of 2-3 PCR-amplified DNA fragments and 0.2-1 pmol of 4-6 PCR-amplified DNA fragments for assembly, respectively. The resulting constructs were amplified by PCR and subsequently used for *in vitro* transcription.

***In vitro* transcription and electroporation of mRNA**

*In vitro* transcription was performed with 50-200 ng of purified PCR product using the mMessage mMachine T7 Ultra Kit (Ambion) according to the manufacturer's instructions. The resulting mRNAs were column purified with an adapted protocol using the RNeasy Kit (Qiagen). According to this protocol, RLT buffer from the kit and 1% beta-mercaptoethanol were added followed by addition of absolute ethanol. The mixture was loaded onto an RNeasy column and purification was performed following the manufacturer's protocol. Purified mRNAs were frozen at -80°C until electroporation. For CAR expression, Jurkat cells and primary T cells were electroporated with 5 or 10 µg mRNA using the Gene Pulser (Biorad) (square wave protocol, 500 V, 5 ms, 4 mm cuvettes).

**Detection of the expression of signaling CAR constructs**

Expression of rcSso7d- and FN3-based CARs was detected via the Strep-II Tag using an anti-Strep-II Tag antibody (clone 5A9F9, GenScript) as primary antibody and a PE-conjugated secondary antibody (eBioscience). CARs containing an IgG spacer were detected by using a biotinylated anti-human-IgGl-antibody as primary antibody and a PE-conjugated streptavidin as secondary staining reagent. Finally, cells were analyzed by flow cytometry.

**Detection of binding of the antigen binding CAR constructs fusion proteins A-D**

Binding of fusion proteins A-D, which were either co-expressed with the signaling CAR constructs or produced by Jurkat cells, was detected by flow cytometry. Accordingly, $25 \times 10^3$ cells were either first incubated with fusion protein-containing supernatant for 45 min at 4°C in the presence of 5 µM A1120 (Sigma-Aldrich) and thereafter washed in the presence of 5 µM A1120 before the second step staining with anti-penta-His antibody (Qiagen), or were directly stained with the anti penta-His antibody for 25 min at 4°C. Before flow cytometric analysis, two washing steps were performed again in the presence of A1120.

**Cytotoxicity assay**

Analysis of the cytotoxic potential of CAR T cells wass performed by a luciferase-based cytotoxicity assay. Therefore, luciferase-expressing tumor target cells were co-cultured with CAR T cells at different effector:target cell ratios (1:1, 2:1, 5:1, 10:1, 25:1, 100:1) in round-bottom 96 well plates for 4 h or 24 h at 37°C. Remaining living cells were then quantified by addition of luciferin (150 µg/mL final concentration; Perkin Elmer) and luciferase activity was measured by using the ENSPIRE Multimode plate reader. The percentage of specific lysis was determined with the following formula:

```
% killing = 100 - ((RLU from well with effector and target cell
co-culture) / (RLU from well with target cells only) x 100)).
```

**Cytokine release by CAR T cells and CAR-expressing Jurkat cells**

Secretion of cytokines into supernatants was assessed by co-culturing the target cells with effector cells at effector:target (E:T) ratios of 1:1 or 2:1 in flat-bottom 96 well plates for 4 or 24 h at 37°C. The supernatants were centrifuged (1600 rpm, 7 min) to remove remaining cells and were frozen at - 20°C. For analysis of secreted cytokines, IFN-y ELISA was performed using the Human IFN gamma ELISA Ready-SET-Go!® (eBioscience) according to the manufacturer's instructions. Secreted IL-8 was quantified by IL-8 Ready-SET-Go!® ELISA (Thermo Fisher Scientific). Measurements were conducted using the ENSPIRE Multimode plate reader.

Accordingly, the present invention discloses the following preferred embodiments:

1. A ligand regulated protein-protein interaction system based on a lipocalin-fold molecule comprising:

(a) a lipocalin-fold molecule
(b) a lipocalin-fold ligand with a low molecular weight of 1500 Da or below, and
(c) a lipocalin-fold binding interaction partner,

wherein the lipocalin-fold molecule can bind to the lipocalin-fold ligand; and
wherein the lipocalin-fold molecule bound to the lipocalin-fold ligand binds to the lipocalin-fold binding interaction partner with an affinity which is at least 10-fold higher than the affinity of the lipocalin-fold molecule not bound to the lipocalin-fold ligand,

and wherein the lipocalin-fold binding interaction partner is not a naturally occurring protein which has an affinity of <10 $\mu$M to any naturally occurring lipocalin-fold molecule in the presence of any lipocalin-fold ligand.

2. A ligand regulated protein-protein interaction system based on a lipocalin-fold molecule comprising:

(a) a lipocalin-fold molecule
(b) a lipocalin-fold ligand with a low molecular weight of 1500 Da or below, and
(c) a lipocalin-fold binding interaction partner,

wherein the lipocalin-fold molecule has at least a first conformation when the lipocalin-fold ligand is not bound to the lipocalin-fold molecule and at least a second conformation when the lipocalin-fold ligand is bound to the lipocalin-fold molecule; and
wherein the lipocalin-fold molecule bound to the lipocalin-fold ligand in the second conformation binds to the lipocalin-fold binding interaction partner with an affinity which is at least 10-fold higher than the affinity of the lipocalin-fold molecule not bound to the lipocalin-fold ligand in the first conformation,
and wherein the lipocalin-fold binding interaction partner is not a naturally occurring protein which has an affinity of <10 $\mu$M to any naturally occurring lipocalin-fold molecule in the presence of any lipocalin-fold ligand.

3. A ligand regulated protein-protein interaction system according to embodiment 1 or 2, wherein the lipocalin-fold binding interaction partner does not contain any segment of at least 50 consecutive amino acids with an amino acid sequence that is identical with the amino acid sequence of any segment of a naturally occurring protein which has an affinity of <10 $\mu$M to any naturally occurring lipocalin-fold molecule, especially in the presence of a lipocalin-fold ligand.

4. A ligand regulated protein-protein interaction system according to any one of embodiments 1 to 3, wherein the affinity of the lipocalin-fold binding interaction partner to the lipocalin-fold molecule when bound to the lipocalin-fold ligand or in the second conformation, respectively, is below 10 $\mu$M, preferably below 2 $\mu$M, especially below 400 nM.

5. A ligand regulated protein-protein interaction system according to any one of embodiments 1 to 4, wherein the lipocalin-fold binding interaction partner is not a homolog of a different species than human of a naturally occurring human lipocalin-fold binding interaction partner.

6. A ligand regulated protein-protein interaction system according to any one of embodiments 1 to 5, wherein the lipocalin-fold molecule is an artificial lipocalin-fold molecule which has no natural counterpart.

7. A ligand regulated protein-protein interaction system according to any one of embodiments 1 to 6, wherein the lipocalin-fold molecule is not a homolog of a different species than human of a naturally occurring human lipocalin-fold molecule.

8. A ligand regulated protein-protein interaction system according to any one of embodiments 1 to 7, wherein the lipocalin-fold ligand is a pharmaceutically active molecule, especially a pharmaceutically active molecule with a therapeutic activity in human patients.

9. A ligand regulated protein-protein interaction system according to any one of embodiments 1 to 8, wherein the lipocalin-fold ligand is a molecule which can be effectively administered orally.

10. A ligand regulated protein-protein interaction system according to any one of embodiments 1 to 9, wherein the lipocalin-fold ligand is a molecule which can be effectively administered intravenously.

11. A ligand regulated protein-protein interaction system according to any one of embodiments 1 to 11, wherein the lipocalin-fold ligand is a molecule which can be effectively administered intravenously and/or orally to a human patient.

12. A ligand regulated protein-protein interaction system according to embodiment 1 to 11, wherein the lipocalin-fold binding interaction partner does not contain a domain of a naturally occurring protein that mediates binding to a naturally occurring lipocalin-fold molecule with an affinity of <10 $\mu$M, especially in the presence of a lipocalin-fold ligand.

13. A ligand regulated protein-protein interaction system according to embodiment 1 to 12, wherein the lipocalin-fold molecule is a molecule identical with a naturally occurring iLBP (intracellular lipid binding protein), a naturally occurring lipocalin or an anticalin, and derivatives of any of these molecules with 1-30 amino acid exchanges and fragments thereof.

14. A ligand regulated protein-protein interaction system according to any one of embodiments 1 to 13, wherein the lipocalin-fold molecule is a derivative of a naturally occurring lipocalin or iLBP with at least one, two, three, four, five, six, seven, eight, nine, ten, 25 or 30 amino acid exchanges.

15. A ligand regulated protein-protein interaction system according to any one of embodiments 1 to 14, wherein the lipocalin-fold molecule is a derivative of a naturally occurring lipocalin or iLBP with up to 15, up to 30, or up to 50 amino acid deletions and/or up to 15, up to 30, or up to 50 amino acid insertions outside of the structurally conserved β-barrel structure, preferably corresponding structurally to the regions of amino acid residues selected from

- amino acid residues 1-20, 31-40, 48-51, 59-70, 79-84, 89-101, 110-113, 121-131 and 139-183 in human RBP4, which define the regions adjoining the structurally conserved β-strands in human RBP4 according to the amino acid residue numbering scheme in the PDB entry 1RBP;
- amino acid residues 1-13, 24-36, 44-47, 55-61, 70-75, 80-83, 92-95, 103-110 and 118-158 in human TLC (according to the amino acid residue numbering scheme in Schiefner et al., Acc Chem Res. 2015;48(4):976-985), which define the regions adjoining the structurally conserved β-strands in human TLC;
- amino acid residues 1-43, 54-68, 76-80, 88-95, 104-109, 114-118, 127-130, 138-141 and 149-188 in human ApoM (according to the amino acid residue numbering scheme in Schiefner et al., Acc Chem Res. 2015;48(4):976-985), which define the regions adjoining the structurally conserved β-strands in human ApoM;
- amino acid residues 1-4, 13-40, 46-49, 55-60, 66-70, 74-80, 88-92, 97-107, 113-118, 125-128 and 136-137 in human CRABPII (according to the amino acid residue numbering scheme in PDB entry 2FS6), which define the regions adjoining the structurally conserved β-strands in human CRABPII;
- amino acid residues 1-4, 13-38, 44-47, 53-58, 64-68, 72-78, 86-90, 95-98, 104-108, 115-118 and 126-127 in human FABP1 (according to the amino acid residue numbering scheme in PDB entry 2F73), which define the regions adjoining the structurally conserved β-strands in human FABP1.

16. A ligand regulated protein-protein interaction system according to any one of embodiments 1 to 15, wherein the lipocalin-fold molecule is a derivative of a naturally occurring lipocalin or iLBP with at least 70%, preferably at least 80%, especially at least 90% sequence identity in the β-barrel structure, whereby this β-barrel structure is defined as the regions preferably corresponding structurally to the regions of amino acid residues selected from

- amino acid residues 21-30, 41-47, 52-58, 71-78, 85-88, 102-109, 114-120 and 132-138 in human RBP4 (according to the amino acid residue numbering scheme in the PDB entry 1RBP), which define the structurally conserved β-strands in human RBP4;
- amino acid residues 14-23, 37-43, 48-54, 62-69, 76-79, 84-91, 96-102 and 111-117 in human tear lipocalin (TLC; as defined by Schiefner et al., Acc Chem Res. 2015;48(4):976-985), which define the structurally conserved β-strands in human TLC;
- amino acid residues 44-53, 69-75, 81-87, 96-103, 110-113, 119-126, 131-137 and 142-148 in human apolipoprotein M (ApoM; as defined by Schiefner et al., Acc Chem Res. 2015;48(4):976-985), which define the structurally conserved β-strands in human ApoM;
- amino acid residues 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6), which define the structurally conserved β-strands in human CRABPII;
- amino acid residues 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73), which define the structurally conserved β-strands in human FABP1;

17. A ligand regulated protein-protein interaction system according to any one of embodiments 1 to 16, wherein the lipocalin-fold molecule is a fragment of a naturally occurring lipocalin or a derivative thereof with a length of at least 80, preferably at least 100, especially at least 120, amino acids covering at least the structurally conserved β-barrel structure of the lipocalin-fold, or wherein the lipocalin-fold molecule is a fragment of a naturally occurring iLBP or a derivative thereof with a length of at least 80, preferably at least 85, especially at least 90, amino acids covering at least the structurally conserved β-barrel structure of the lipocalin-fold, wherein the structurally conserved β-barrel structure comprises or consists of amino acid positions preferably corresponding structurally to the regions of amino acid residues selected from

- amino acid residues 21-30, 41-47, 52-58, 71-78, 85-88, 102-109, 114-120 and 132-138 in human RBP4 (according to the amino acid residue numbering scheme in the PDB entry 1RBP), which define the structurally conserved β-strands in human RBP4;
- amino acid residues 14-23, 37-43, 48-54, 62-69, 76-79, 84-91, 96-102 and 111-117 in human tear lipocalin (TLC; as defined by Schiefner et al., Acc Chem Res. 2015;48(4):976-985), which define the structurally conserved β-strands in human TLC;
- amino acid residues 44-53, 69-75, 81-87, 96-103, 110-113, 119-126, 131-137 and 142-148 in human apolipoprotein M (ApoM; as defined by Schiefner et al., Acc Chem Res. 2015;48(4):976-985), which define the structurally conserved β-strands in human ApoM;
- amino acid residues 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6), which define the structurally conserved β-strands in human CRABPII;
- amino acid residues 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73), which define the structurally conserved β-strands in human FABP1;

18. A ligand regulated protein-protein interaction system according to any one of embodiments 1 to 17, wherein the lipocalin-fold molecule bound to the lipocalin-fold ligand binds to the lipocalin-fold binding interaction partner with an affinity which is at least 20-fold higher, preferably at least 50-fold higher, than the affinity of the lipocalin-fold molecule not bound to the lipocalin-fold ligand.

19. A ligand regulated protein-protein interaction system according to any one of embodiments 1 to 18, wherein the lipocalin-fold molecule bound to the lipocalin-fold ligand binds to the lipocalin-fold binding interaction partner with an affinity which is at least 100-fold higher, preferably at least 200-fold higher, especially at least 500-fold higher, than the affinity of the lipocalin-fold molecule not bound to the lipocalin-fold ligand.

20. A ligand regulated protein-protein interaction system according to any one of embodiments 1 to 19, wherein the lipocalin-fold molecule bound to the lipocalin-fold ligand binds to the lipocalin-fold binding interaction partner with an affinity which is at least 1000-fold higher than the affinity of the lipocalin-fold molecule not bound to the lipocalin-fold ligand.

21. A ligand regulated protein-protein interaction system according to any one of embodiments 1 to 20, wherein the lipocalin-fold ligand has a molecular weight of 1500 to 75 Da, preferably of 750 Da to 150 Da.

22. A ligand regulated protein-protein interaction system according to any one of embodiments 1 to 21, wherein the lipocalin-fold binding interaction partner is or wherein the lipocalin-fold molecule and/or the lipocalin-fold binding interaction partner comprises an antigen, a cell surface receptor, an antibody, an antibody fragment, or a non-antibody based scaffold, preferably an affibody, a lipocalin-fold molecule, preferably an iLPB or a LCN, especially an anticalin; an avimer, a DARPin, a fynomer, a Kunitz domain, a knottin, a monobody, a Sso7d-based binder, reduced charge Sso7d (rcSso7d)-based binder or Sac7d-based binder.

23. A ligand regulated protein-protein interaction system according to any one of embodiments 1 to 22, wherein the lipocalin-fold binding interaction partner and/or the lipocalin-fold molecule comprises an antigen, a cell surface receptor, an antibody, an antibody fragment, or a non-antibody based scaffold, preferably an affibody, a lipocalin-fold molecule, preferably an iLPB or a LCN, especially an anticalin; an avimer, a DARPin, a fynomer, a Kunitz domain, a knottin, a monobody, a Sso7d-based binder, reduced charge Sso7d (rcSso7d)-based binder or Sac7d-based binder.

24. A ligand regulated protein-protein interaction system according to any one of embodiments 1 to 23, wherein the lipocalin-fold ligand has an affinity to the lipocalin-fold molecule of below 1 mM, preferably of below 100 μM, especially of below 10 μM.

25. A ligand regulated protein-protein interaction system according to any one of embodiments 1 to 24, wherein the lipocalin-fold ligand is a ligand selected from Table 1, especially fenretinide (15-[(4-hydroxyphenyl)amino]retinal), N-Ethylretinamide (PubChem CID: 5288173), all-trans retinoic acid (PubChem CID: 444795), axerophthene (PubChem CID: 5287722), A1120 (PubChem CID 25138295) and derivatives thereof, 1,4-butanediol, sphingosine-1-phosphate, tetradecanoic acid, indicaxanthin, vulgaxanthin, Montelukast, Cyclandelate, Oxolamine, Mazaticol, Butoctamid, Tonabersat, Novazin, Diphenidol, Neobornyval, Alloclamide, Diacetolol, Clindamycin, Proxazole, Tropesin, Triclabendazole, Triclabendazole sulfoxide, Triclabendazole sulfone and Trametinib.

26. A ligand regulated protein-protein interaction system according to any one of embodiments 1 to 25, wherein the lipocalin-fold molecule is part of an ectodomain of a chimeric antigen receptor and wherein the lipocalin-fold binding interaction partner is a cell surface antigen.

27. A ligand regulated protein-protein interaction system according to any one of embodiments 1 to 26, wherein the lipocalin-fold molecule is a lipocalin.

28. A ligand regulated protein-protein interaction system according to any one of embodiments 1 to 27, wherein the lipocalin-fold molecule has a sequence identity with human RBP4 of at least 95%.

29. A ligand regulated protein-protein interaction system according to any one of embodiments 1 to 27, wherein the lipocalin-fold molecule has a sequence identity with human tear lipocalin (TLC) of at least 95%.

30. A ligand regulated protein-protein interaction system according to any one of embodiments 1 to 27, wherein the lipocalin-fold molecule has a sequence identity with human apolipoprotein M (ApoM) of at least 95%.

31. A ligand regulated protein-protein interaction system according to any one of embodiments 1 to 30, wherein both, the lipocalin-fold molecule and the lipocalin-fold binding interaction partner are not part of a naturally occurring ligand regulated protein-protein interaction system.

32. A ligand regulated protein-protein interaction system according to any one of embodiments 1 to 31, wherein the lipocalin-fold ligand is selected from the group fenretinide (15-[(4-hydroxyphenyl)amino]retinal), N-Ethylretinamide (PubChem CID: 5288173), all-trans retinoic acid (PubChem CID: 444795), axerophthene (PubChem CID: 5287722), A1120 (PubChem CID 25138295) and derivatives thereof, 1,4-butanediol, sphingosine-1-phosphate, tetradecanoic acid, indicaxanthin, vulgaxanthin, Montelukast, Cyclandelate, Oxolamine, Mazaticol, Butoctamid, Tonabersat, Novazin, Diphenidol, Neobornyval, Alloclamide, Diacetolol, Clindamycin, Proxazole, Tropesin, Triclabendazole, Triclabendazole sulfoxide, Triclabendazole sulfone and Trametinib.

**Claims**

1. A ligand regulated protein-protein interaction system based on a lipocalin-fold molecule comprising:

   (a) a lipocalin-fold molecule
   (b) a lipocalin-fold ligand with a low molecular weight of 1500 Da or below, and
   (c) a lipocalin-fold binding interaction partner,

   wherein the lipocalin-fold molecule can bind to the lipocalin-fold ligand; and
   wherein the lipocalin-fold molecule bound to the lipocalin-fold ligand binds to the lipocalin-fold binding interaction partner with an affinity which is at least 10-fold higher than the affinity of the lipocalin-fold molecule not bound to the lipocalin-fold ligand,
   and wherein the lipocalin-fold binding interaction partner is not a naturally occurring protein which has an affinity of <10 μM to any naturally occurring lipocalin-fold molecule in the presence of any lipocalin-fold ligand.

2. A ligand regulated protein-protein interaction system based on a lipocalin-fold molecule comprising:

   (a) a lipocalin-fold molecule
   (b) a lipocalin-fold ligand with a low molecular weight of 1500 Da or below, and
   (c) a lipocalin-fold binding interaction partner,

   wherein the lipocalin-fold molecule has at least a first conformation when the lipocalin-fold ligand is not bound to the lipocalin-fold molecule and at least a second conformation when the lipocalin-fold ligand is bound to the lipocalin-fold molecule; and
   wherein the lipocalin-fold molecule bound to the lipocalin-fold ligand in the second conformation binds to the lipocalin-fold binding interaction partner with an affinity which is at least 10-fold higher than the affinity of the lipocalin-fold molecule not bound to the lipocalin-fold ligand in the first conformation,
   and wherein the lipocalin-fold binding interaction partner is not a naturally occurring protein which has an affinity of <10 μM to any naturally occurring lipocalin-fold molecule in the presence of any lipocalin-fold ligand.

3. A ligand regulated protein-protein interaction system according to claim 1 or 2, wherein the lipocalin-fold molecule is a molecule identical with a naturally occurring iLBP (intracellular lipid binding protein), a naturally occurring lipocalin or an anticalin, and derivatives of any of these molecules with 1-30 amino acid exchanges and fragments thereof.

4. A ligand regulated protein-protein interaction system according to any one of claims 1 to 3, wherein the lipocalin-fold molecule is a derivative of a naturally occurring lipocalin or iLBP with at least one, two, three, four, five, six, seven, eight, nine, ten, 25 or 30 amino acid exchanges.

5. A ligand regulated protein-protein interaction system according to any one of claims 1 to 4, wherein the lipocalin-fold molecule is a derivative of a naturally occurring lipocalin or iLBP with up to 15, up to 30, or up to 50 amino acid deletions and/or up to 15, up to 30, or up to 50 amino acid insertions outside of the structurally conserved β-barrel structure, preferably corresponding structurally to the regions of amino acid residues selected from

- amino acid residues 1-20, 31-40, 48-51, 59-70, 79-84, 89-101, 110-113, 121-131 and 139-183 in human RBP4, which define the regions adjoining the structurally conserved β-strands in human RBP4 according to the amino acid residue numbering scheme in the PDB entry 1RBP;

- amino acid residues 1-13, 24-36, 44-47, 55-61, 70-75, 80-83, 92-95, 103-110 and 118-158 in human TLC (according to the amino acid residue numbering scheme in Schiefner et al., Acc Chem Res. 2015;48(4):976-985), which define the regions adjoining the structurally conserved β-strands in human TLC;

- amino acid residues 1-43, 54-68, 76-80, 88-95, 104-109, 114-118, 127-130, 138-141 and 149-188 in human ApoM (according to the amino acid residue numbering scheme in Schiefner et al., Acc Chem Res. 2015;48(4):976-985), which define the regions adjoining the structurally conserved β-strands in human ApoM;

- amino acid residues 1-4, 13-40, 46-49, 55-60, 66-70, 74-80, 88-92, 97-107, 113-118, 125-128 and 136-137 in human CRABPII (according to the amino acid residue numbering scheme in PDB entry 2FS6), which define the regions adjoining the structurally conserved β-strands in human CRABPII;

- amino acid residues 1-4, 13-38, 44-47, 53-58, 64-68, 72-78, 86-90, 95-98, 104-108, 115-118 and 126-127 in human FABP1 (according to the amino acid residue numbering scheme in PDB entry 2F73), which define the regions adjoining the structurally conserved β-strands in human FABP1.

6. A ligand regulated protein-protein interaction system according to any one of claims 1 to 5, wherein the lipocalin-fold molecule is a derivative of a naturally occurring lipocalin or iLBP with at least 70%, preferably at least 80%, especially at least 90% sequence identity in the β-barrel structure, whereby this β-barrel structure is defined as the regions preferably corresponding structurally to the regions of amino acid residues selected from

- amino acid residues 21-30, 41-47, 52-58, 71-78, 85-88, 102-109, 114-120 and 132-138 in human RBP4 (according to the amino acid residue numbering scheme in the PDB entry 1RBP), which define the structurally conserved β-strands in human RBP4;

- amino acid residues 14-23, 37-43, 48-54, 62-69, 76-79, 84-91, 96-102 and 111-117 in human tear lipocalin (TLC; as defined by Schiefner et al., Acc Chem Res. 2015;48(4):976-985), which define the structurally conserved β-strands in human TLC;

- amino acid residues 44-53, 69-75, 81-87, 96-103, 110-113, 119-126, 131-137 and 142-148 in human apolipoprotein M (ApoM; as defined by Schiefner et al., Acc Chem Res. 2015;48(4):976-985), which define the structurally conserved β-strands in human ApoM;

- amino acid residues 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6), which define the structurally conserved β-strands in human CRABPII;

- amino acid residues 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73), which define the structurally conserved β-strands in human FABP1;

7. A ligand regulated protein-protein interaction system according to any one of claims 1 to 6, wherein the lipocalin-fold molecule is a fragment of a naturally occurring lipocalin or a derivative thereof with a length of at least 80, preferably at least 100, especially at least 120, amino acids covering at least the structurally conserved β-barrel structure of the lipocalin-fold, or wherein the lipocalin-fold molecule is a fragment of a naturally occurring iLBP or a derivative thereof with a length of at least 80, preferably at least 85, especially at least 90, amino acids covering at least the structurally conserved β-barrel structure of the lipocalin-fold,
wherein the structurally conserved β-barrel structure comprises or consists of amino acid positions preferably corresponding structurally to the regions of amino acid residues selected from

- amino acid residues 21-30, 41-47, 52-58, 71-78, 85-88, 102-109, 114-120 and 132-138 in human RBP4 (according to the amino acid residue numbering scheme in the PDB entry 1RBP), which define the structurally conserved β-strands in human RBP4;

- amino acid residues 14-23, 37-43, 48-54, 62-69, 76-79, 84-91, 96-102 and 111-117 in human tear lipocalin (TLC; as defined by Schiefner et al., Acc Chem Res. 2015;48(4):976-985), which define the structurally conserved β-strands in human TLC;

- amino acid residues 44-53, 69-75, 81-87, 96-103, 110-113, 119-126, 131-137 and 142-148 in human apolipoprotein M (ApoM; as defined by Schiefner et al., Acc Chem Res. 2015;48(4):976-985), which define the structurally conserved β-strands in human ApoM;

- amino acid residues 5-12, 41-45, 50-54, 61-65, 71-73, 81-87, 93-96, 108-112, 119-124 and 129-135 in human cellular retinoic acid binding protein II (CRABPII; according to the amino acid residue numbering scheme in PDB entry 2FS6), which define the structurally conserved β-strands in human CRABPII;

- amino acid residues 5-12, 39-43, 48-52, 59-63, 69-71, 79-85, 91-94, 99-103, 109-114 and 119-125 in human fatty acid binding protein 1 (FABP1; according to the amino acid residue numbering scheme in PDB entry 2F73), which define the structurally conserved β-strands in human FABP1;

8. A ligand regulated protein-protein interaction system according to any one of claims 1 to 7, wherein the lipocalin-fold molecule bound to the lipocalin-fold ligand binds to the lipocalin-fold binding interaction partner with an affinity which is at least 20-fold higher, preferably at least 50-fold higher, than the affinity of the lipocalin-fold molecule not bound to the lipocalin-fold ligand.

9. A ligand regulated protein-protein interaction system according to any one of claims 1 to 8, wherein the lipocalin-fold molecule bound to the lipocalin-fold ligand binds to the lipocalin-fold binding interaction partner with an affinity which is at least 100-fold higher, preferably at least 200-fold higher, especially at least 500-fold higher, than the affinity of the lipocalin-fold molecule not bound to the lipocalin-fold ligand.

10. A ligand regulated protein-protein interaction system according to any one of claims 1 to 9, wherein the lipocalin-fold molecule bound to the lipocalin-fold ligand binds to the lipocalin-fold binding interaction partner with an affinity which is at least 1000-fold higher than the affinity of the lipocalin-fold molecule not bound to the lipocalin-fold ligand.

11. A ligand regulated protein-protein interaction system according to any one of claims 1 to 10, wherein the lipocalin-fold ligand has a molecular weight of 1500 to 75 Da, preferably of 750 Da to 150 Da.

12. A ligand regulated protein-protein interaction system according to any one of claims 1 to 11, wherein the lipocalin-fold binding interaction partner is or wherein the lipocalin-fold molecule and/or the lipocalin-fold binding interaction partner comprises an antigen, a cell surface receptor, an antibody, an antibody fragment, or a non-antibody based scaffold, preferably an affibody, a lipocalin-fold molecule, preferably an iLPB or a LCN, especially an anticalin; an avimer, a DARPin, a fynomer, a Kunitz domain, a knottin, a monobody, a Sso7d-based binder, reduced charge Sso7d (rcSso7d)-based binder or Sac7d-based binder.

13. A ligand regulated protein-protein interaction system according to any one of claims 1 to 12, wherein the lipocalin-fold binding interaction partner and/or the lipocalin-fold molecule comprises an antigen, a cell surface receptor, an antibody, an antibody fragment, or a non-antibody based scaffold, preferably an affibody, a lipocalin-fold molecule, preferably an iLPB or a LCN, especially an anticalin; an avimer, a DARPin, a fynomer, a Kunitz domain, a knottin, a monobody, a Sso7d-based binder, reduced charge Sso7d (rcSso7d)-based binder or Sac7d-based binder.

14. A ligand regulated protein-protein interaction system according to any one of claims 1 to 13, wherein the lipocalin-fold ligand has an affinity to the lipocalin-fold molecule of below 1 mM, preferably of below 100 $\mu$M, especially of below 10 $\mu$M.

15. A ligand regulated protein-protein interaction system according to any one of claims 1 to 14, wherein the lipocalin-fold ligand is a ligand selected from Table 1, and/or selected from the group fenretinide (15-[(4-hydroxyphenyl)amino] retinal), N-Ethylretinamide (PubChem CID: 5288173), all-trans retinoic acid (PubChem CID: 444795), axerophthene (PubChem CID: 5287722), A1120 (PubChem CID 25138295) and derivatives thereof, 1,4-butanediol, sphingosine-1-phosphate, tetradecanoic acid, indicaxanthin, vulgaxanthin, Montelukast, Cyclandelate, Oxolamine, Mazaticol, Butoctamid, Tonabersat, Novazin, Diphenidol, Neobornyval, Alloclamide, Diacetolol, Clindamycin, Proxazole, Tropesin, Triclabendazole, Triclabendazole sulfoxide, Triclabendazole sulfone and Trametinib.

Fig. 1

Fig. 2

EP 3 502 130 A1

**rcSso7d-based binder (anti-RBP4)**

**FN3-based binder (anti-RBP4)**

| Binder | Mean $K_d$ [nM] in the presence of 5 µM A1120 |
|--------|-----------------------------------------------|
| RS1 | 1,50 |
| RS2 | 2,90 |
| RS3 | 11,50 |
| RS4 | 47,60 |
| RS5 | 69,50 |

| Binder | Mean $K_d$ [nM] in the presence of 5 µM A1120 |
|--------|-----------------------------------------------|
| RF1 | 168,8 |
| RF2 | 41,8 |
| RF3 | 67,0 |

Fig. 3

Fig. 4

# Fig. 5A

**rcSso7d:** 61 amino acids

ATVKFTYQGEEKQVDISKIKWVIRWGQHIAFKYDEGGGAAGYGWVSEKDAPKELLQMLEKQ

## RS1:

GCAACCGTGAAATTCACATACCAAGGCGAAGAAGAACAGGTGGATATTAGCAAAATCAAGAAAGTGGCTCGT
TACGGCCAGAACATTTACTTTTCTTATGATGAAGGTGGTGGTGCCTGGGATTATGGTGGCGTGAGCGAAAAAG
ATGCACCGAAAGAACTGCTGCAGATGCTGGAAAAGCAA

ATVKFTYQGEEEQVDISKIKKVARYGQNIYFSYDEGGGAWDYGGVSEKDAPKELLQMLEKQ

## RS2:

GCAACCGTGAAACTCACATACCAAGGCGAAGAAAAACAGGTGGATATTAGCAAAATCAAGCGTGTGGCTCGT
TACGGCCAGGGTATTTACTTTGACTATGGTGAAGGTGGTGGTGCCTGGGGTTACGGTAGCGTGAGCGAAAAA
GATGCACCGAAAGAACTGCTGCAGATGCTGGAAAAGCAA

ATVKLTYQGEEKQVDISKIKRVARYGQGIYFDYGEGGGAWGYGSVSEKDAPKELLQMLEKQ

## RS3:

GCAACCGTGAAACTCACATACCAAGGCGAAGAAAAACAGGTGGATATTAGCAAAATCAAGCGTGTGGCTCGT
TACGGCCAGAACATTTACTTTTCTTATGATGAAGGTGGTGGTGCCTATGATTATGGTGCAGTGAGCGAAAAAG
ATGCACCGAAAGAACTGCTGCAGATGCTGGAAAAGCAA

ATVKLTYQGEEKQVDISKIKRVARYGQNIYFSYDEGGGAYDYGAVSEKDAPKELLQMLEKQ

## RS4:

GCAACCGCGAAATTCACATACCAAGGCGAAGAAAAACAGGTGGATATTAGCAAAATCAAGCGCGTGGCTCGT
TACGGCCAGGGTATTTACTTTTCTTATGATGAAGGTGGTGGTGCCTATGGTTATGGTAGCGTGAGCGAAAAAG
ATGCACCGAAAGAACTGCTGCAGATGCTGGAAAAGCAA

ATAKFTYQGEEKQVDISKIKRVARYGQGIYFSYDEGGGAYGYGSVSEKDAPKELLQMLEKQ

## RS5:

GCAACCGTGAAATTCACATACCAAGGCGAAGAAAAACAGGTGGATATTAGCAAAATCAAGCGTGTGGCTCGT
TACGGCCAGGGTATTTACTTTGACTATGGTGAAGGTGGTGGTGCCTGGGGTTACGGTAGCGTGAGCGAAGAA
GATGCACCGAAAGAACTGCTGCAGATGCTGGAAAAGCAA

ATVKFTYQGEEKQVDISKIKRVARYGQGIYFDYGEGGGAWGYGSVSEEDAPKELLQMLEKQ

# Fig. 5B

**FN3 wild type:** 102 amino acids

VSDVPRDLEVVAATPTSLLISWDAPAVTVRYYRITYGETGGNSPVQEFTVPGSKSTATISGLKPGVDYTITVYAV
TGRGDSPASSKPISINYRTEIDKPSQ

**RF1**

GTTTCTGATGTTCCGAGGGACCTGGAAGTTGTTGCTGCGACCCCCACCAGCCTACTGATCAGCTGGTATTATCC
CAACGCTAGTCATGCGGGTTATTACAGGGTCACTTACGGAGAAACAGGAGGAAATAGCCCTGTCCAGGAGTT
CACTGTGCCTTTCTCTATTCGGTATACTATTGCTACCATCAGCGGCCTTAAACCTGGAGTTGATCATACCATCAC
TGTGTATGCTGTCACTGACTACGCCTATTACTACCGCTCGTCTGAGCCAATTTCCATTAATTACCGAACAGAAAT
TGACAAACCATCCCAGG

VSDVPRDLEVVAATPTSLLISWYYPNASHAGYYRVTYGETGGNSPVQEFTVPFSIRYTIATISGLKPGVDHTITV
YAVTDYAYYYRSSEPISINYRTEIDKPSQ

**RF2**

GTTTCTGATGTTCCGAGGGACCTGGAAGTTGTTGCTGCGACCCCCACCAGCCTACTGATCAGCTGGTATTATCC
CAACGCTAGTCATGCGGGTTATTACAGGATCACTTACGGAGAAACAGGAGGAAATAGCCCTGTCCAGGAGTTC
ACTGTGCCTTTCTCTATTCGGTATACTATTGCTACCATCAGCGGCCTTAAACCTGGAGTTGATTATACCATCACT
GTGTATGCTGTCACTGACTACGCCTATTACTACCGCTTGTCTGAGCCAATTTCCATTAATTACCGAACAGAAATT
GACAAACCATCCCAGG

VSDVPRDLEVVAATPTSLLISWYYPNASHAGYYRITYGETGGNSPVQEFTVPFSIRYTIATISGLKPGVDYTITV
YAVTDYAYYYRLSEPISINYRTEIDKPSQ

**RF3**

GTTTCTGATGTTCCGAGGGACCTGGAAGTTGTTGCTGCGACCCCCACCAGCCTACTGATCAGCTGGTATTATCC
CAACGCTAGTCATGCGGGTTATTACAGGATCACTTACGGAGAAACAGGAGGAAATAGCCCTGTCCAGGAGTTC
ACTGTGCCTTTCTCTATTCGGTATACTATTGCTACCATCAGCGGCCTTAAACCTGGAGTTGATTATACCATCACT
GTGTATGCTGTCACTGCCAGTTGTCAGTATTGCTCTTATCCAATTTCCATTAATTACCGAACAGAAATTGACAAA
CCATCCCAGG

VSDVPRDLEVVAATPTSLLISWYYPNASHAGYYRITYGETGGNSPVQEFTVPFSIRYTIATISGLKPGVDYTITV
YAVTASCQYCSYPISINYRTEIDKPSQ

Fig. 6A

**Design of RF long CARs** (CAR constructs containing Fibronectin domain type 3 („RF") as binding domain)

RF SUMO CARs

Fig. 6B

# Fig. 7A

Underlined text = signal peptide

Underlined (bold) text = rcSso7d or FN-based RBP4 binder

Dark grey text = c-myc Tag

Underlined (dotted) text = 2x G4S linker

Underlined text = Strep Tag

*Underlined and italic text* = His Tag

**Bold text** = CD8 stalk

*Italic text* = Fc spacer

Light grey = CD28 co-stimulatory domain

Underlined (dotted) text = 41BB domain

Underlined (double) text = CD3 zeta signalling domain

*Underlined (double) and italic text* = FKBP domain

**RS1 short CAR:**

MPLLLLLPLLWAGALAMATVKFTYQGEEEQVDISKIKKVARYGQNIYFSY
DEGGGAWDYGGVSEKDAPKELLQMLEKQGGGGSGGGGSNWSHPQFEK**TTTPAPRPPTPAP
TIASQPLSLRPEACRPAAGGAVHTRGLDFACD**IYIWAPLAGTCGVLLLSLVITLYCKRGR
KKLLYIFKQPFMRPVQTTQEEDGCSCRFPEEEEGGCELRVKFSRSADAPAYKQGQNQLYN
ELNLGRREEYDVLDKRRGRDPEMGGKPRRKNPQEGLYNELQKDKMAEAYSEIGMKGERRR
GKGHDGLYQGLSTATKDTYDALHMQALPPR

**RS2 short CAR:**

MPLLLLLPLLWAGALAMATVKLTYQGEEKQVDISKIKRVARYGQGIYFDY
GEGGGAWGYGSVSEKDAPKELLQMLEKQGGGGSGGGGSNWSHPQFEK**TTTPAPRPPTPAP
TIASQPLSLRPEACRPAAGGAVHTRGLDFACD**IYIWAPLAGTCGVLLLSLVITLYCKRGR
KKLLYIFKQPFMRPVQTTQEEDGCSCRFPEEEEGGCELRVKFSRSADAPAYKQGQNQLYN
ELNLGRREEYDVLDKRRGRDPEMGGKPRRKNPQEGLYNELQKDKMAEAYSEIGMKGERRR
GKGHDGLYQGLSTATKDTYDALHMQALPPR

**RS3 short CAR:**

MPLLLLLPLLWAGALAMATVKLTYQGEEKQVDISKIKRVARYGQNIYFSY
DEGGGAYDYGAVSEKDAPKELLQMLEKQGGGGSGGGGSNWSHPQFEK**TTTPAPRPPTPAP
TIASQPLSLRPEACRPAAGGAVHTRGLDFACD**IYIWAPLAGTCGVLLLSLVITLYCKRGR
KKLLYIFKQPFMRPVQTTQEEDGCSCRFPEEEEGGCELRVKFSRSADAPAYKQGQNQLYN
ELNLGRREEYDVLDKRRGRDPEMGGKPRRKNPQEGLYNELQKDKMAEAYSEIGMKGERRR
GKGHDGLYQGLSTATKDTYDALHMQALPPR

**RS3 c-myc short CAR:**

MPLLLLLPLLWAGALAMATVKLTYQGEEKQVDISKIKRVARYGQNIYFSY
DEGGGAYDYGAVSEKDAPKELLQMLEKQEQKLISEEDLGGGGSGGGGSNWSHPQFEK**TTT
PAPRPPTPAPTIASQPLSLRPEACRPAAGGAVHTRGLDFACD**IYIWAPLAGTCGVLLLSL
VITLYCKRGRKKLLYIFKQPFMRPVQTTQEEDGCSCRFPEEEEGGCELRVKFSRSADAPA
YKQGQNQLYNELNLGRREEYDVLDKRRGRDPEMGGKPRRKNPQEGLYNELQKDKMAEAYS
EIGMKGERRRGKGHDGLYQGLSTATKDTYDALHMQALPPR

# Fig. 7B

**RS4 short CAR:**

MPLLLLLLPLLWAGALAMATAKFTYQGEEKQVDISKIKRVARYGQGIYFSY
DEGGGAYGYGSVSEKDAPKELLQMLEKQGGGGSGGGGSNWSHPQFEK**TTTPAPRPPTPAP
TIASQPLSLRPEACRPAAGGAVHTRGLDFACD**IYIWAPLAGTCGVLLLSLVITLYCKRGR
KKLLYIFKQPFMRPVQTTQEEDGCSCRFPEEEEGGCELRVKFSRSADAPAYKQGQNQLYN
ELNLGRREEYDVLDKRRGRDPEMGGKPRRKNPQEGLYNELQKDKMAEAYSEIGMKGERRR
GKGHDGLYQGLSTATKDTYDALHMQALPPR

**RS5 short CAR:**

MPLLLLLLPLLWAGALAMATVKFTYQGEEKQVDISKIKRVARYGQGIYFDY
GEGGGAWGYGSVSEEDAPKELLQMLEKQGGGGSGGGGSNWSHPQFEK**TTTPAPRPPTPAP
TIASQPLSLRPEACRPAAGGAVHTRGLDFACD**IYIWAPLAGTCGVLLLSLVITLYCKRGR
KKLLYIFKQPFMRPVQTTQEEDGCSCRFPEEEEGGCELRVKFSRSADAPAYKQGQNQLYN
ELNLGRREEYDVLDKRRGRDPEMGGKPRRKNPQEGLYNELQKDKMAEAYSEIGMKGERRR
GKGHDGLYQGLSTATKDTYDALHMQALPPR

**RS5 c-myc short CAR:**

MPLLLLLLPLLWAGALAMATVKFTYQGEEKQVDISKIKRVARYGQGIYFDY
GEGGGAWGYGSVSEEDAPKELLQMLEKQEQKLISEEDLGGGGSGGGGSNWSHPQFEK**TTT
PAPRPPTPAPTIASQPLSLRPEACRPAAGGAVHTRGLDFACD**IYIWAPLAGTCGVLLLSL
VITLYCKRGRKKLLYIFKQPFMRPVQTTQEEDGCSCRFPEEEEGGCELRVKFSRSADAPA
YKQGQNQLYNELNLGRREEYDVLDKRRGRDPEMGGKPRRKNPQEGLYNELQKDKMAEAYS
EIGMKGERRRGKGHDGLYQGLSTATKDTYDALHMQALPPR

**RS3 c-myc long CAR:**

MPLLLLLLPLLWAGALAMATVKLTYQGEEKQVDISKIKRVARYGQNIYFSY
DEGGGAYDYGAVSEKDAPKELLQMLEKQEQKLISEEDLGGGGSGGGGSNWSHPQFEKEPK
*SPDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGKKDPK* GGVLACYSLLVTVAFIIFWVRSKRSRLLHSDYMNMTPRRPGPTRKHYQPYAPPRDFAAYR
SLRVKFSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR

**RS5 c-myc long CAR:**

MPLLLLLLPLLWAGALAMATVKFTYQGEEKQVDISKIKRVARYGQGIYFDY
GEGGGAWGYGSVSEEDAPKELLQMLEKQEQKLISEEDLGGGGSGGGGSNWSHPQFEKEPK
*SPDKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGKKDPK* GGVLACYSLLVTVAFIIFWVRSKRSRLLHSDYMNMTPRRPGPTRKHYQPYAPPRDFAAYR
SLRVKFSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR

# Fig. 7C

**RS3 long CAR:**

MPLLLLLPLLWAGALAMATVKLTYQGEEKQVDISKIKRVARYGQNIYFSYDEGGGAYDYG
AVSEKDAPKELLQMLEKQGGGGSGGGGSNWSHPQFEKEPKSPDKTHTCPPCPAPELLGGP
SVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGKKDPK~~FWVLVVVGGVLACYSLLVTVAFIIFWV~~
~~RSKRSRLLHSDYMNMTPRRPGPTRKHYQPYAPPRDFAAYRSL~~RVKFSRSADAPAYQQGQN
QLYNELNLGRREEYDVLDKRRGRDPEMGGKPRRKNPQEGLYNELQKDKMAEAYSEIGMKG
ERRRGKGHDGLYQGLSTATKDTYDALHMQALPPR


**RS5 long CAR:**

MPLLLLLPLLWAGALAMATVKFTYQGEEKQVDISKIKRVARYGQGIYFDYGEGGGAWGYG
SVSEEDAPKELLQMLEKQGGGGSGGGGSNWSHPQFEKEPKSPDKTHTCPPCPAPELLGGP
SVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDEL
TKNQVSLTCLVKGFYPSDIAVEWESNGQPENNYKTTPPVLDSDGSFFLYSKLTVDKSRWQ
QGNVFSCSVMHEALHNHYTQKSLSLSPGKKDPK~~FWVLVVVGGVLACYSLLVTVAFIIFWV~~
~~RSKRSRLLHSDYMNMTPRRPGPTRKHYQPYAPPRDFAAYRSL~~RVKFSRSADAPAYQQGQN
QLYNELNLGRREEYDVLDKRRGRDPEMGGKPRRKNPQEGLYNELQKDKMAEAYSEIGMKG
ERRRGKGHDGLYQGLSTATKDTYDALHMQALPPR


**RF2 long CAR:**

MPLLLLLPLLWAGALAMVSDVPRDLEVVAATPTSLLISWYYPNASHAGYYRITYGETGGN
SPVQEFTVPFSIRYTIATISGLKPGVDYTITVYAVTDYAYYYRLSEPISINYRTGGGGSG
GGGSDPAEPKSPDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKAL
PAPIEKTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSLSLSPGKK
DPK~~FWVLVVVGGVLACYSLLVTVAFIIFWVRSKRSRLLHSDYMNMTPRRPGPTRKHYQPY~~
~~APPRDFAAYRSL~~RVKFSRSADAPAYQQGQNQLYNELNLGRREEYDVLDKRRGRDPEMGGK
PRRKNPQEGLYNELQKDKMAEAYSEIGMKGERRRGKGHDGLYQGLSTATKDTYDALHMQA
LPPR


**RF2 SUMO CAR:**

METDTLLLWVLLLWVPGSTGDG*HHHHHH*GSLQDSEVNQEAKPEVKPEVKPETHINLKVSDGS
SEIFFKIKKTTPLRRLMEAFAKRQGKEMDSLTFLYDGIEIQADQTPEDLDMEDNDIIEAHRE
QIGGGGGSGGGGSVSDVPRDLEVVAATPTSLLISWYYPNASHAGYYRITYGETGGNSPVQEF
TVPFSIRYTIATISGLKPGVDYTITVYAVTDYAYYYRLSEPISINYRTGGGGSGGGGSNWSH
PQFEK**TTTPAPRPPTPAPTIASQPLSLRPEASRPAAGGAVHTRGLDFASD**IYIWAPLAGTCG
VLLLSLVITLYCKRGRKKLLYIFKQPFMRPVQTTQEEDGCSCRFPEEEEGGCELSRGSGSGS
GS*MGVQVETISPGDGRTFPKRGQTCVVHYTGMLEDGKKVDSSRDRNKPFKFMLGKQEVIRGW
EEGVAQMSVGQRAKLTISPDYAYGATGHPGIIPPHATLVFDVELLKLEG*SGSGSGSSLRVKF
SRSADAPAYKQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPR


**RBP4 Fc CAR: (bold letters indicate RBP4)**

MPLLLLLPLLWAGALAM**ERDCRVSSFRVKENFDKARFSGTWYAMAKKDPE**
**GLFLQDNIVAEFSVDETGQMSATAKGRVRLLNNWDVCADMVGTFTDTEDPAKFKMKYWGV**
**ASFLQKGNDDHWIVDTDYDTYAVQYSCRLLNLDGTCADSYSFVFSRDPNGLPPEAQKIVR**
**QRQEELCLARQYRLIVHNGYCDGRSERNLL**GGGGSGGGGSNWSHPQFEKEPKSPDKTHTC
PPCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFL
YSKLTVDKSRWQQGNVFSCSVMHEALHNHYTQKSLSLSPGKKDPK~~WVLVVVGGVLACYS~~
~~LLVTVAFIIFWVRSKRSRLLHSDYMNMTPRRPGPTRKHYQPYAPPRDFAAYRSL~~RVKFSR
SADAPAYQQGQNQLYNELNLGRREEYDVLDKRRGRDPEMGGKPRRKNPQEGLYNELQKDK
MAEAYSEIGMKGERRRGKGHDGLYQGLSTATKDTYDALHMQALPPR

EP 3 502 130 A1

**A**

Fig. 8

EP 3 502 130 A1

**B**

Fig. 8 cont

Fig. 9

**Fig. 10**

Underlined text = RBP4 signal peptide (SP)
Underlined (bold) text = CD19 scFv
Dark grey text = G4S linker (GGGGS) linker
Underlined (dotted) text = His-Tag
*Italic text* = Fc domain (IgG)
Underlined (double) text = RBP4
Underlined text = RS3 (rcSso7d-based RBP4-binder)
*Underlined italic text* = rcSso7d-based EGFR-binder

## Fusion protein "A"

MKWVWALLLLAALGSGRADIQMTQTTSSLSASLGDRVTISCRASQDISKY
LNWYQQKPDGTVKLLIYHTSRLHSGVPSRFSGSGSGTDYSLTISNLEQEDIATYFCQQGN
TLPYTFGGGTKLEITGSTSGSGKPGSGEGSTKGEVKLQESGPGLVAPSQSLSVTCTVSGV
SLPDYGVSWIRQPPRKGLEWLGVIWGSETTYYNSALKSRLTIIKDNSKSQVFLKMNSLQT
DDTAIYYCAKHYYYGGSYAMDYWGQGTSVTVSSGGGGSHHHHHHERDCRVSSFRVKENFD
KARFSGTWYAMAKKDPEGLFLQDNIVAEFSVDETGQMSATAKGRVRLLNNWDVCADMVGT
FTDTEDPAKFKMKYWGVASFLQKGNDDHWIVDTDYDTYAVQYSCRLLNLDGTCADSYSFV
FSRDPNGLPPEAQKIVRQRQEELCLARQYRLIVHNGYCDGRSERNLL

## Fusion protein "B"

MKWVWALLLLAALGSGRADIQMTQTTSSLSASLGDRVTISCRASQDISKY
LNWYQQKPDGTVKLLIYHTSRLHSGVPSRFSGSGSGTDYSLTISNLEQEDIATYFCQQGN
TLPYTFGGGTKLEITGSTSGSGKPGSGEGSTKGEVKLQESGPGLVAPSQSLSVTCTVSGV
SLPDYGVSWIRQPPRKGLEWLGVIWGSETTYYNSALKSRLTIIKDNSKSQVFLKMNSLQT
DDTAIYYCAKHYYYGGSYAMDYWGQGTSVTVSSGGGGS*EPKSPDKTHTCPPCPAPELLGG*
*PSVFLFPPKPKDTLMISRTPEVTCVVVDVSHEDPEVKFNWYVDGVEVHNAKTKPREEQYN*
*STYRVVSVLTVLHQDWLNGKEYKCKVSNKALPAPIEKTISKAKGQPREPQVYTLPPSRDE*
*LTKNQVSLTCLVKGFYPSDIAVEWESNGQPENNYKTTPPVLDSDGSFFLYSKLTVDKSRW*
*QQGNVFSCSVMHEALHNHYTQKSLSLSPGK*GGGGSHHHHHHERDCRVSSFRVKENFDKAR
FSGTWYAMAKKDPEGLFLQDNIVAEFSVDETGQMSATAKGRVRLLNNWDVCADMVGTFTD
TEDPAKFKMKYWGVASFLQKGNDDHWIVDTDYDTYAVQYSCRLLNLDGTCADSYSFVFSR
DPNGLPPEAQKIVRQRQEELCLARQYRLIVHNGYCDGRSERNLL

## Fusion protein "C"

MKWVWALLLLAALGSGRADIQMTQTTSSLSASLGDRVTISCRASQDISKY
LNWYQQKPDGTVKLLIYHTSRLHSGVPSRFSGSGSGTDYSLTISNLEQEDIATYFCQQGN
TLPYTFGGGTKLEITGSTSGSGKPGSGEGSTKGEVKLQESGPGLVAPSQSLSVTCTVSGV
SLPDYGVSWIRQPPRKGLEWLGVIWGSETTYYNSALKSRLTIIKDNSKSQVFLKMNSLQT
DDTAIYYCAKHYYYGGSYAMDYWGQGTSVTVSSGGGGSHHHHHHATVKLTYQGEEKQVDI
SKIKRVARYGQNIYFSYDEGGGAYDYGAVSEKDAPKELLQMLEKQEQKLISEEDL

## Fusion protein "D"

MKWVWALLLLAALGSGRA*ATVKFTYQGEEKQVDISKIMYVIRGGQRIAFG*
*YDEGDGAWGDGIVSEKDAPKELLQMLEKQ*GGGGSHHHHHHERDCRVSSFRVKENFDKARF
SGTWYAMAKKDPEGLFLQDNIVAEFSVDETGQMSATAKGRVRLLNNWDVCADMVGTFTDT
EDPAKFKMKYWGVASFLQKGNDDHWIVDTDYDTYAVQYSCRLLNLDGTCADSYSFVFSRD
PNGLPPEAQKIVRQRQEELCLARQYRLIVHNGYCDGRSERNLL

Fig. 11

Fig. 12

**A)**

**B)**

Fig. 13

Europäisches
Patentamt

European
Patent Office

Office européen
des brevets

**EUROPEAN SEARCH REPORT**

Application Number

EP 17 20 8924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STEPHANIE VOSS ET AL: "Chemically induced dimerization: reversible and spatiotemporal control of protein function in cells", CURRENT OPINION IN CHEMICAL BIOLOGY, vol. 28, 29 September 2015 (2015-09-29), pages 194-201, XP055451688, GB ISSN: 1367-5931, DOI: 10.1016/j.cbpa.2015.09.003 * table 1 * | 1-15 | INV. C07K14/725 C07K14/705 C07K14/775 C07K14/78 C07K14/195 C07K14/47 G01N33/68 |
| A | ROBERT DEROSE ET AL: "Manipulating signaling at will: chemically-inducible dimerization (CID) techniques resolve problems in cell biology", PFLUEGERS ARCHIV: EUROPEAN JOURNAL OF PHYSIOLOGY, vol. 465, no. 3, 9 January 2013 (2013-01-09), pages 409-417, XP055221913, DE ISSN: 0031-6768, DOI: 10.1007/s00424-012-1208-6 * abstract * * page 409, right-hand column, paragraph 2 * * figures 1-3 * | 1-15 | |

-----

-----

-/--

TECHNICAL FIELDS
SEARCHED (IPC)

C07K
G01N

The present search report has been drawn up for all claims

| Place of search | Date of completion of the search | Examiner |
|---|---|---|
| Munich | 16 February 2018 | Tudor, Mark |

EPO FORM 1503 03.82 (P04C01)

page 1 of 2

Europäisches
Patentamt

European
Patent Office

Office européen
des brevets

## EUROPEAN SEARCH REPORT

Application Number

EP 17 20 8924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | A. MOTANI ET AL: "Identification and Characterization of a Non-retinoid Ligand for Retinol-binding Protein 4 Which Lowers Serum Retinol-binding Protein 4 Levels in Vivo", JOURNAL OF BIOLOGICAL CHEMISTRY, vol. 284, no. 12, 15 January 2009 (2009-01-15), pages 7673-7680, XP055115833, ISSN: 0021-9258, DOI: 10.1074/jbc.M809654200 * abstract * * page 7676, left-hand column, lines 4-5 * * page 7676, right-hand column, paragraph 1 * ----- | 1-15 | |

TECHNICAL FIELDS
SEARCHED       (IPC)

The present search report has been drawn up for all claims

| Place of search | Date of completion of the search | Examiner |
|---|---|---|
| Munich | 16 February 2018 | Tudor, Mark |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014127261 A1
- WO 2017032777 A1
- DE 19742706 A1
- WO 99016873 A1
- EP 1017814 B1
- WO 2012065978 A1
- WO 2016113203 A1
- WO 2015017214 A1
- EP 3087101 A1
- US 20170081411 A1

### Non-patent literature cited in the description

- **MURZIN et al.** *J Mol Biol.,* 1995, vol. 247 (4), 536-540
- **LAKSHMI et al.** *PLoS One,* 2015, vol. 10 (8), e0135507
- **ZHANG et al.** *PLoS One,* 2012, vol. 7 (5), e36772
- **SMATHERS et al.** *Hum Genomics,* 2011, vol. 5 (3), 170-191
- **GRZYB et al.** *J Plant Physiol.,* 2006, vol. 163 (9), 895-915
- **FLOWER et al.** *Biochim Biophys Acta,* 2000, vol. 1482 (1-2), 9-24
- **SCHIEFNER et al.** *Acc Chem Res.,* 2015, vol. 48 (4), 976-985
- **SKERRA.** *Biochim Biophys Acta,* 2000, vol. 1482 (1-2), 337-350
- **KORNDORFER et al.** *Proteins,* 2003, vol. 53 (1), 121-129
- **KORNDORFER et al.** *J Mol Biol.,* 2003, vol. 330 (2), 385-396
- **KIM et al.** *J Am Chem Soc.,* 2009, vol. 131 (10), 3565-3576
- **SCHLEHUBER et al.** *Biophys Chem.,* 2002, vol. 96 (2-3), 213-228
- **RICHTER et al.** *FEBS Lett.,* 2014, vol. 588 (2), 213-218
- **SCHONFELD et al.** *Proc Natl Acad Sci USA,* 2009, vol. 106 (20), 8198-8203
- **GEBAUER et al.** *J Mol Biol,* 2013, vol. 425 (4), 780-802
- **BARINKA et al.** *Protein Eng Des Sel.,* 2016, vol. 29 (3), 105-115
- **JONES et al.** *Front Pharmacol.,* 2014, vol. 5, 254
- **SUN et al.** *Cell Res.,* 2015, vol. 25 (12), 1281-1282
- **SERGEEVA et al.** *Advanced Drug Delivery Reviews,* 2006, vol. 58, 1622-1654
- **RUTKOWSKA et al.** *Angew Chem Int Ed Engl.,* 2012, vol. 51 (33), 8166-8176
- **PAPIZ et al.** *Nature,* 1986, vol. 324 (6095), 383-385
- **SPINELLI et al.** *Eur J Biochem.,* 2002, vol. 269 (10), 2449-2456
- **SEVVANA et al.** *J Mol Biol.,* 2010, vol. 404 (3), 363-371
- **BERNI et al.** *FEBS Lett.,* 1992, vol. 308 (1), 43-45
- **ZANOTTI et al.** *J Biol Chem.,* 1993, vol. 268 (33), 24873-24879
- **ZANOTTI et al.** *J Biol Chem.,* 1994, vol. 269 (47), 29613-29620
- **PATTANAYEK et al.** *Protein Sci.,* 1999, vol. 8 (10), 2027-2032
- **MOTANI et al.** *J Biol Chem.,* 2009, vol. 284 (12), 7673-7680
- **GASYMOV et al.** *Biochim Biophys Acta,* 1998, vol. 1386 (1), 145-156
- **BREUSTEDT et al.** *Acta Crystallogr D Biol Crystallogr,* 2009, vol. 65 (10), 1118-1125
- **GASYMOV et al.** *Biochemistry,* 2012, vol. 51 (14), 2991-3002
- **ZHANG et al.** *Sci Rep.,* 2016, vol. 6, 30655
- **CHRISTOFFERSEN et al.** *Proc Natl Acad Sci USA.,* 2011, vol. 108 (23), 9613-9618
- **REDONDO et al.** *FASEB J.,* 2008, vol. 22 (4), 1043-1054
- **COWARD et al.** *Anal Biochem.,* 2009, vol. 384 (2), 312-320
- **SHARIF et al.** *Anal Biochem.,* 2009, vol. 392 (2), 162-168
- **CHRISTOFFERSEN et al.** *Proc Natl Acad Sci USA,* 2011, vol. 108 (23), 9613-9618
- **BAO et al.** *RSC Adv.,* 2015, vol. 5 (126), 104363-104374
- **EGGENSTEIN et al.** *J Struct Biol.,* 2014, vol. 185 (2), 203-214

- **GASYMOV et al.** *Biochim Biophys Acta,* 2011, vol. 1814 (5), 671-683
- **SKERRA.** *Biochim Biophys Acta,* 2000, vol. 1482 (1-2), 337-35049
- **SCHMIDT.** Untersuchungen zur Protein-faltung durch Protein-Design am Retinol-Bindungsprotein. Herbert Utz Verlag, 1998
- **WHITEHEAD et al.** *Methods Enzymol.,* 2013, vol. 523, 1-19
- **STRAUCH et al.** *Proc Natl Acad Sci USA,* 14 January 2014, vol. 111 (2), 675-80
- **WORN et al.** *J Biol Chem.,* 2000, vol. 275 (4), 2795-2803
- **TRAXLMAYR et al.** *Biochim Biophys Acta,* 2012, vol. 1824 (4), 542-549
- **HOTAMISLIGIL et al.** *Nat Rev Endocrinol.,* 2015, vol. 11 (10), 592-605
- **CURTO et al.** *Protein Sci.,* 2009, vol. 18 (4), 735-746
- **OGBAY et al.** *Protein Sci.,* 2004, vol. 13 (5), 1227-1237
- **YU et al.** *Sci Rep.,* 2016, vol. 6, 34171
- **SHARMA et al.** *J Biol Chem.,* 2011, vol. 286 (36), 31924-31928
- **CAI et al.** *Biophys J,* 2012, vol. 102 (11), 2585-2594
- **FRANZONI et al.** *J Lipid Res.,* 2010, vol. 51 (6), 1332-1343
- **VAEZESLAMI et al.** *J Mol Biol.,* 2006, vol. 363 (3), 687-701
- **GILLILAN et al.** *J Mol Biol.,* 2007, vol. 372 (5), 1246-1260
- **MENOZZI et al.** *J Struct Biol.,* 2017, vol. 197 (3), 330-339
- **LONG et al.** *Biophys J.,* 2010, vol. 98 (12), 3054-3061
- **ARMSTRONG et al.** *J Biol Chem.,* 2014, vol. 289 (21), 14941-14954
- **AMBER-VITOS et al.** *PLoS One,* 2015, vol. 10 (8), e0132138
- **BERRY et al.** *Mol Cell Biol.,* 2012, vol. 32 (15), 3164-3175
- **SESSLER et al.** *Mol Cell,* 2005, vol. 18 (3), 343-353
- **HOFER et al.** *J Biol Chem.,* 2015, vol. 290 (30), 18438-18453
- **FURUHASHI et al.** *Nat Rev Drug Discov.,* 2008, vol. 7 (6), 489-503
- **VELKOV T.** *Chem Biol.,* 2007, vol. 14 (4), 453-465
- **VELKOV T.** *PPAR Res.,* 2013, vol. 2013, 938401
- **BERINGHELLI T.** *PLoS One,* 2015, vol. 10 (7), e0132096
- **CHUANG S.** *J Med Chem.,* 2008, vol. 51 (13), 3755-3764

- **SESSLER et al.** *Mol Cell.,* 2005, vol. 18 (3), 343-353
- **DOBRI et al.** *Invest Ophthalmol Vis Sci.,* 2013, vol. 54 (1), 85-95
- **VELKOV et al.** *Chem Biol.,* 2007, vol. 14 (4), 453-465
- **BERINGHELLI et al.** *PLoS One,* 2015, vol. 10 (7), e0132096
- **CHUANG.** *J Med Chem.,* 2008, vol. 51 (13), 3755-3764
- **CIOFFI et al.** *J Med Chem.,* 2014, vol. 57 (18), 7731-7757
- **CIOFFI et al.** *J Med Chem.,* 2015, vol. 58 (15), 5863-5888
- Receptor Binding Assays for HTS and Drug Discovery. **AULD et al.** Assay Guidance Manual. Eli Lilly & Company and the National Center for Advancing Translational Sciences, 2004
- **QING et al.** *Journal of Receptor, Ligand and Channel Research,* 2014, vol. 7, 81-92
- **BREUSTEDT et al.** *Acta Crystallogr D Biol Crystallogr.,* 2009, vol. 65 (10), 1118-1125
- **GASYMOV et al.** *Biochemistry.,* 2012, vol. 51 (14), 2991-3002
- **SIMEON et al.** *Protein Cell,* 2017
- **GILBRETH et al.** *Curr Opin Struct Biol,* 2012, vol. 22 (4), 413-420
- **KOIDE et al.** *ACS Chem Biol.,* 2009, vol. 4 (5), 325-334
- **TRAXLMAYR et al.** *J Biol Chem.,* 2016, vol. 291 (43), 22496-22508
- **PLUCKTHUN.** Alternative Scaffolds: Expanding the options of antibodies. Cambridge University Press, 2009, 244-271
- **CHAPMAN et al.** *Cell Chem Biol.,* 2016, vol. 23 (5), 543-553
- **BINZ et al.** *Nat Biotechnol.,* 2005, vol. 23 (10), 1257-1268
- **VAZQUEZ-LOMBARDI et al.** *Drug Discov Today,* 2015, vol. 20 (10), 1271-1283
- **VASQUEZ-LOMBARDI et al.** *Drug Discov. Today,* 2015, vol. 20, 1271-1283
- Alternative Scaffolds: Expanding the Options of Antibodies. **PLUCKTHUN ; MELVYN LITTLE.** Recombinant Antibodies for Immunotherapy. Cambridge University Press, 2009, 244-271
- **ABATE-DAGA et al.** *Mol Ther Oncolytics,* 2016, vol. 3, 16014
- **CHEN et al.** *Methods Enzymol.,* 2013, vol. 523, 303-326
- **WYSOCKA-KAPCINSKA et al.** *Protein Expr Purif.,* 2010, vol. 71 (1), 28-32
- **HACKEL et al.** *JMB,* 2010, vol. 401, 84-96
- **CHAO et al.** *Nat Protoc.,* 2006, vol. 1 (2), 755-768
- **ANGELINI et al.** *Methods Mol Biol.,* 2015, vol. 1319, 3-36